# EUROPEAN PATENT SPECIFICATION

(11) **EP 3 265 580 B1**
(45) Date of publication and mention of the grant of the patent: **30.03.2022**
(21) Application number: 16707044.0
(22) Date of filing: 24.02.2016
(51) Int. Cl.: C12Q 1/68

(54) **SIGNATURE OF HEALTH**
SIGNATUR DER GESUNDHEIT
SIGNATURE DE SANTÉ

(30) Priority: 04.03.2015 EP 15157672; 12.03.2015 EP 15158794; 20.04.2015 EP 15164272
(43) Date of publication of application: 10.01.2018
(73) Proprietor: Hummingbird Diagnostics GmbH, 69120 Heidelberg (DE)
(72) Inventor: BEIER, Markus, 69469 Weinheim (DE); BREFORT, Thomas, 85560 Ebersberg (DE); KOHLHAAS, Jochen, 69121 Heidelberg (DE); KELLER, Andreas, 66346 Püttlingen (DE)
(74) Representative: Geling, Andrea
(86) International application number: PCT/EP2016/053834
(87) International publication number: WO 2016/139092

(56) References cited:
- WO-A1-2010/139810
- WO-A1-2014/085906
- WO-A1-2014/145612
- WO-A2-2009/111643
- AILBHE M. MCDERMOTT ET AL: "Identification and Validation of miRNAs as Endogenous Controls for RQ-PCR in Blood Specimens for Breast Cancer Studies", PLOS ONE, vol. 8, no. 12, 31 December 2013 (2013-12-31), page e83718, XP055197914, DOI: 10.1371/journal.pone.0083718
- CHANG KAH HOONG ET AL: "MicroRNA expression profiling to identify and validate reference genes for relative quantification in colorectal cancer", BMC CANCER, BIOMED CENTRAL, LONDON, GB, vol. 10, no. 1, 29 April 2010 (2010-04-29) , page 173, XP021075012, ISSN: 1471-2407, DOI: 10.1186/1471-2407-10-173
- DAVOREN PAMELA A ET AL: "Identification of suitable endogenous control genes for microRNA gene expression analysis in human breast cancer", BMC MOLECULAR BIOLOGY, BIOMED CENTRAL LTD, GB, vol. 9, no. 1, 21 August 2008 (2008-08-21) , page 76, XP021042428, ISSN: 1471-2199, DOI: 10.1186/1471-2199-9-76

## Description

### TECHNICAL FIELD OF THE INVENTION

The present invention relates to methods for assessing the status of health of a subject based on the determination of expression profiles of miRNAs and to uses of said methods.

### BACKGROUND OF THE INVENTION

Today, biomarkers play a key role in diagnosis, risk stratification, and therapeutic management of various diseases. MicroRNAs (miRNAs) are a new class of biomarkers. They represent a group of small noncoding RNAs that regulate gene expression at the posttranslational level by degrading or blocking translation of messenger RNA (mRNA) targets. It has been found that miRNAs are expressed in a highly tissue-specific manner and further, miRNAs are also present in body fluid samples, including blood. Nevertheless, the mechanism why miRNAs are found in blood, especially in blood cells, or their function in these blood fractions is not understood yet. Especially desirable are non-invasive biomarkers, that allow for quick, easy and cost-effective diagnosis/prognosis, eliminating the need for surgical intervention. Particularly, the potential role of miRNAs as non-invasive biomarkers for general diagnosis of a change in a subjects status of health has not been systematically evaluated yet. Accordingly, there is still a need for effective methods and kits for the non-invasive diagnosis of change in a subjects status of health.

WO 2014/145612 A1 relates to tissue and blood-based miRNA biomarkers for the diagnosis, prognosis, and metastasis-predictive potential in colorectal cancer. WO 2010/139810 A1 discloses a method for diagnosing lung cancer on the basis of miRNA expression profiling. Ailbhe M et al. (PLoS ONE, Vol. 8, No. 12, December 31, 2013) relate to the identification and validation of miRNAs as endogenous controls for RQ-PCR in blood specimens for breast cancer studies.

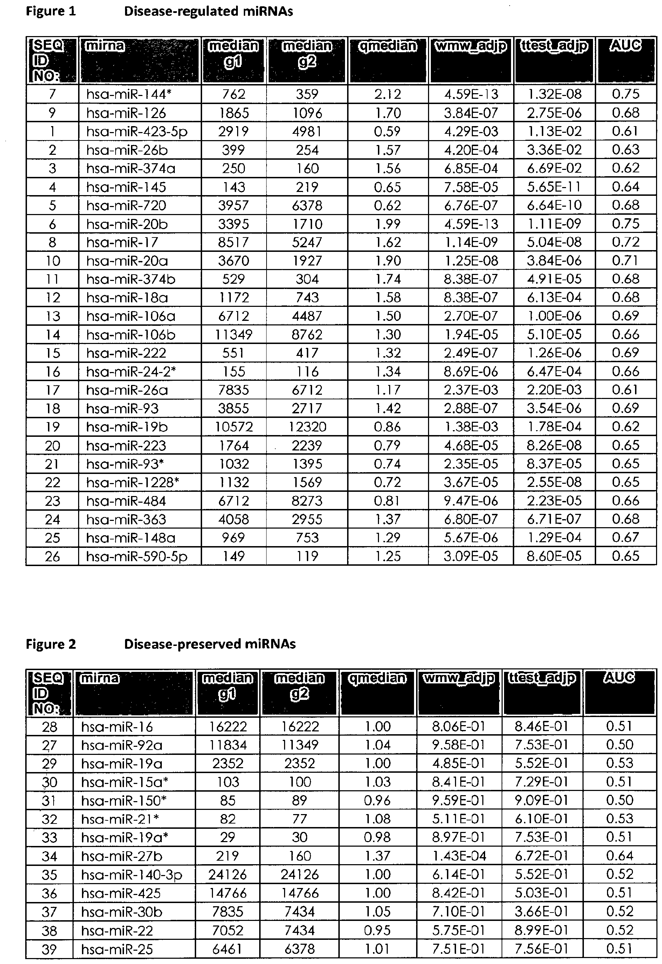

The inventors of the present invention assessed for the first time the expression of miRNAs on a whole-genome level in broad range of subjects being healthy (n=158) of being diagnosed with a plurality of diseases (n=883) including cancer (i.e. lung, colon, kidney, glioblastoma, prostate, melanoma), neurodegenerative (i.e. multiple sclerosis, parkinson), cardiovascular (acute myocardial infarction, heart failure), autoimmune (i.e. sarcoidosis, psoriasis) or inflammatory diseases (i.e. COPD, BPH). They surprisingly found that certain miRNAs (disease-regulated miRNAs, Figure 1) are significantly dysregulated between healthy controls and diseased subjects, while certain other miRNAs (disease-preserved miRNAs, Figure 2) are not significantly dysregulated between healthy controls and diseased subjects in blood samples, preferably in whole blood samples or blood cellular fractions comprising erythrocytes, leukocytes and/or thrombocytes. Thus, said disease regulated miRNAs in combination with said disease-preserved miRNAs are appropriated non-invasive biomarkers for diagnosis of a change in a subject's status of health. In particular, the disease-preserved miRNAs may act as internal normalizers for the disease-regulated miRNAs, which is of great advantage when aiming at bringing a reliable and robust diagnostic test to the market as such a test cannot rely on global normalization methods because a set of only a few miRNA biomarkers are used. The test according to the present invention hence may rely on comparing relative expression levels to a reference. Furthermore, the inventors of the present invention explored the use of miRNAs from blood cells respective blood cellular fractions comprising erythrocytes, leukocytes and/or thrombocytes, which are obtained from collecting whole blood samples. Thus, the miRNAs of all blood cells, representing the constituents of the peripheral immune system are analyzed. Therefore, the diagnostic content that is assessed by this approach refers to assessment of the miRNA-biomarker information of the peripheral immune system, which is different from an approach based on miRNAs derived from the extra-cellular blood fraction (serum, plasma), which directly relates to a diseased organ or tissue. Furthermore, an approach relying on miRNA derived from the extra-cellular blood fraction (serum, plasma) is highly prone to contamination from miRNAs leaking from blood cells into the extra-cellular matrix upon collection and/or pre-analytical workup, which is not the case when employing miRNA biomarkers from blood cells or blood cellular fractions.

Thus, said disease-regulated in combination with said disease-preserved miRNAs derived from blood cells or blood cellular fractions comprising erythrocytes, leukocytes and/or thrombocytes are analytically robust and reliable appropriated non-invasive biomarkers with internal normalization capabilities for diagnosis of a change in a subject's status of health using orthogonal diagnostic information originating from the peripheral immune system.

### SUMMARY OF THE INVENTION

In a first aspect, the invention provides a method for assessing the health status in a subject, comprising the steps of:
(a) isolating total intracellular RNA from erythrocytes, leukocytes, and thrombocytes which have been obtained from a whole blood sample which has been taken from a subject,
(b) determining in said total intracellular RNA an expression profile of one or more first miRNAs, wherein the one or more first miRNAs have a nucleotide sequence selected from the group consisting of SEQ ID NO: 1 to SEQ ID NO: 16 and SEQ ID NO: 20 to SEQ ID NO: 26,
(c) determining in said total intracellular RNA an expression profile of one or more second miRNAs, wherein the one or more second miRNAs have a nucleotide sequence selected from the group consisting of SEQ ID NO: 27, SEQ ID NO: 29, SEQ ID NO: 35, SEQ ID NO: 38, SEQ ID NO: 39, SEQ ID NO: 43 to SEQ ID NO: 47, SEQ ID NO: 49, SEQ ID NO: 50, SEQ ID NO: 52, SEQ ID NO: 55 to SEQ ID NO: 57, and SEQ ID NO: 59,
(d) normalizing the expression profile of said one or more first miRNAs to the expression profile of said one or more second miRNAs, thereby obtaining one or more relative expression levels of: SEQ ID NO: 1/SEQ ID NO: 45, SEQ ID NO: 1/SEQ ID NO: 59, SEQ ID NO: 1/SEQ ID NO: 56, SEQ ID NO: 2/SEQ ID NO: 27, SEQ ID NO: 2/SEQ ID NO: 35, SEQ ID NO: 3/SEQ ID NO: 27, SEQ ID NO: 4/SEQ ID NO: 55, SEQ ID NO: 4/SEQ ID NO: 47, SEQ ID NO: 4/SEQ ID NO: 29^{∗}, SEQ ID NO: 4/SEQ ID NO: 56, SEQ ID NO: 4/SEQ ID NO: 59, SEQ ID NO: 4/SEQ ID NO: 43, SEQ ID NO: 5/SEQ ID NO: 59, SEQ ID NO: 5/SEQ ID NO: 29, SEQ ID NO: 5/SEQ ID NO: 55, SEQ ID NO: 5/SEQ ID NO: 45, SEQ ID NO: 5/SEQ ID NO: 44, SEQ ID NO: 5/SEQ ID NO: 57, SEQ ID NO: 5/SEQ ID NO: 46, SEQ ID NO: 5/SEQ ID NO: 50, SEQ ID NO: 5/SEQ ID NO: 49, SEQ ID NO: 5/SEQ ID NO: 56, SEQ ID NO: 5/SEQ ID NO: 47, SEQ ID NO: 6/SEQ ID NO: 35, SEQ ID NO: 6/SEQ ID NO: 27, SEQ ID NO: 6/SEQ ID NO: 38, SEQ ID NO: 6/SEQ ID NO: 29, SEQ ID NO: 6/SEQ ID NO: 43, SEQ ID NO: 6/SEQ ID NO: 49, SEQ ID NO: 6/SEQ ID NO: 50, SEQ ID NO: 6/SEQ ID NO: 52, SEQ ID NO: 6/SEQ ID NO: 39, SEQ ID NO: 7/SEQ ID NO: 27, SEQ ID NO: 7/SEQ ID NO: 35, SEQ ID NO: 7/SEQ ID NO: 29, SEQ ID NO: 7/SEQ ID NO: 43, SEQ ID NO: 7/SEQ ID NO: 50, SEQ ID NO: 7/SEQ ID NO: 39, SEQ ID NO: 7/SEQ ID NO: 38, SEQ ID NO: 7/SEQ ID NO: 47, SEQ ID NO: 8/SEQ ID NO: 35, SEQ ID NO: 8/SEQ ID NO: 27, SEQ ID NO: 8/SEQ ID NO: 38, SEQ ID NO: 8/SEQ ID NO: 39, SEQ ID NO: 8/SEQ ID NO: 43, SEQ ID NO: 9/SEQ ID NO: 35, SEQ ID NO: 9/SEQ ID NO: 27, SEQ ID NO: 9/SEQ ID NO: 38, SEQ ID NO: 9/SEQ ID NO: 43, SEQ ID NO: 9/SEQ ID NO: 29, SEQ ID NO: 9/SEQ ID NO: 39, SEQ ID NO: 10/SEQ ID NO: 35, SEQ ID NO: 10/SEQ ID NO: 27, SEQ ID NO: 10/SEQ ID NO: 38, SEQ ID NO: 10/SEQ ID NO: 49, SEQ ID NO: 10/SEQ ID NO: 39, SEQ ID NO: 10/SEQ ID NO: 43, SEQ ID NO: 11/SEQ ID NO: 27, SEQ ID NO: 11/SEQ ID NO: 35, SEQ ID NO: 11/SEQ ID NO: 39, SEQ ID NO: 11/SEQ ID NO: 38, SEQ ID NO: 12/SEQ ID NO: 35, SEQ ID NO: 13/SEQ ID NO: 35, SEQ ID NO: 13/SEQ ID NO: 27, SEQ ID NO: 13/SEQ ID NO: 39, SEQ ID NO: 14/SEQ ID NO: 35, SEQ ID NO: 15/SEQ ID NO: 35, SEQ ID NO: 15/SEQ ID NO: 38, SEQ ID NO: 15/SEQ ID NO: 27, SEQ ID NO: 15/SEQ ID NO: 39, SEQ ID NO: 16/SEQ ID NO: 35, SEQ ID NO: 20/SEQ ID NO: 29, SEQ ID NO: 20/SEQ ID NO: 59, SEQ ID NO: 20/SEQ ID NO: 57, SEQ ID NO: 20/SEQ ID NO: 38, SEQ ID NO: 20/SEQ ID NO: 39, SEQ ID NO: 20/SEQ ID NO: 47, SEQ ID NO: 21/SEQ ID NO: 50, SEQ ID NO: 21/SEQ ID NO: 59, SEQ ID NO: 21/SEQ ID NO: 29^{∗}, SEQ ID NO: 21/SEQ ID NO: 57, SEQ ID NO: 21/SEQ ID NO: 55, SEQ ID NO: 21/SEQ ID NO: 49, SEQ ID NO: 21/SEQ ID NO: 56, SEQ ID NO: 22/SEQ ID NO: 59, SEQ ID NO: 22/SEQ ID NO: 47, SEQ ID NO: 22/SEQ ID NO: 56, SEQ ID NO: 22/SEQ ID NO: 57, SEQ ID NO: 22/SEQ ID NO: 29^{∗}, SEQ ID NO: 23/SEQ ID NO: 59, SEQ ID NO: 23/SEQ ID NO: 56, SEQ ID NO: 23/SEQ ID NO: 57, SEQ ID NO: 24/SEQ ID NO: 27, SEQ ID NO: 24/SEQ ID NO: 35, SEQ ID NO: 24/SEQ ID NO: 38, SEQ ID NO: 24/SEQ ID NO: 39, SEQ ID NO: 24/SEQ ID NO: 43, SEQ ID NO: 25/SEQ ID NO: 35, SEQ ID NO: 25/SEQ ID NO: 39, SEQ ID NO: 25/SEQ ID NO: 43, SEQ ID NO: 25/SEQ ID NO: 38, SEQ ID NO: 25/SEQ ID NO: 27, SEQ ID NO: 26/SEQ ID NO: 35, or SEQ ID NO: 26/SEQ ID NO: 27,
(e) comparing the one or more relative expression levels of step (d) to one or more relative reference expression levels, and
(f) assessing a change of the health status in said subject when the comparison to said one or more relative reference expression levels is altered.

In a second aspect, the invention provides a method for assessing the health status in a subject, comprising the steps of:
(a) isolating total intracellular RNA from erythrocytes, leukocytes, and thrombocytes which have been obtained from a whole blood sample which has been taken from a subject,
(b) determining in said total intracellular RNA an expression profile of at least one first miRNA,
(c) determining in said total intracellular RNA an expression profile of at least one second miRNA,
(d) comparing the expression profile of the at least one first miRNA of step (b) to a reference expression profile of said at least one first miRNA and the expression profile of the at least one second miRNA of step (c) to a reference expression profile of said at least one second miRNA, and
(e) assessing a change of the health status in said subject when the comparison of the expression profile of the at least one first miRNA to the reference expression profile of said at least one first miRNA is altered and when the comparison to the expression profile of the at least one second miRNA to the reference expression profile of said at least one second miRNA is not altered,
wherein
the at least one first miRNA has a nucleotide sequence according to SEQ ID NO: 1 and the at least one second miRNA has a nucleotide sequence according to SEQ ID NO: 27,
the at least one first miRNA has a nucleotide sequence according to SEQ ID NO: 1 and the at least one second miRNA has a nucleotide sequence according to SEQ ID NO: 29,
the at least one first miRNA has a nucleotide sequence according to SEQ ID NO: 1 and the at least one second miRNA has a nucleotide sequence according to SEQ ID NO: 35,
the at least one first miRNA has a nucleotide sequence according to SEQ ID NO: 2 and the at least one second miRNA has a nucleotide sequence according to SEQ ID NO: 27,
the at least one first miRNA has a nucleotide sequence according to SEQ ID NO: 2 and the at least one second miRNA has a nucleotide sequence according to SEQ ID NO: 29,
the at least one first miRNA has a nucleotide sequence according to SEQ ID NO: 3 and the at least one second miRNA has a nucleotide sequence according to SEQ ID NO: 27,
the at least one first miRNA has a nucleotide sequence according to SEQ ID NO: 3 and the at least one second miRNA has a nucleotide sequence according to SEQ ID NO: 29,
the at least one first miRNA has a nucleotide sequence according to SEQ ID NO: 4 and the at least one second miRNA has a nucleotide sequence according to SEQ ID NO: 27,
the at least one first miRNA has a nucleotide sequence according to SEQ ID NO: 4 and the at least one second miRNA has a nucleotide sequence according to SEQ ID NO: 29,
the at least one first miRNA has a nucleotide sequence according to SEQ ID NO: 5 and the at least one second miRNA has a nucleotide sequence according to SEQ ID NO: 27,
the at least one first miRNA has a nucleotide sequence according to SEQ ID NO: 5 and the at least one second miRNA has a nucleotide sequence according to SEQ ID NO: 29,
the at least one first miRNA has a nucleotide sequence according to SEQ ID NO: 5 and the at least one second miRNA has a nucleotide sequence according to SEQ ID NO: 55,
the at least one first miRNA has a nucleotide sequence according to SEQ ID NO: 5 and the at least one second miRNA has a nucleotide sequence according to SEQ ID NO: 59,
the at least one first miRNA has a nucleotide sequence according to SEQ ID NO: 6 and the at least one second miRNA has a nucleotide sequence according to SEQ ID NO: 27,
the at least one first miRNA has a nucleotide sequence according to SEQ ID NO: 6 and the at least one second miRNA has a nucleotide sequence according to SEQ ID NO: 29,
the at least one first miRNA has a nucleotide sequence according to SEQ ID NO: 6 and the at least one second miRNA has a nucleotide sequence according to SEQ ID NO: 35,
the at least one first miRNA has a nucleotide sequence according to SEQ ID NO: 6 and the at least one second miRNA has a nucleotide sequence according to SEQ ID NO: 38,
the at least one first miRNA has a nucleotide sequence according to SEQ ID NO: 7 and the at least one second miRNA has a nucleotide sequence according to SEQ ID NO: 27,
the at least one first miRNA has a nucleotide sequence according to SEQ ID NO: 7 and the at least one second miRNA has a nucleotide sequence according to SEQ ID NO: 29,
the at least one first miRNA has a nucleotide sequence according to SEQ ID NO: 7 and the at least one second miRNA has a nucleotide sequence according to SEQ ID NO: 35,
the at least one first miRNA has a nucleotide sequence according to SEQ ID NO: 8 and the at least one second miRNA has a nucleotide sequence according to SEQ ID NO: 27,
the at least one first miRNA has a nucleotide sequence according to SEQ ID NO: 8 and the at least one second miRNA has a nucleotide sequence according to SEQ ID NO: 29,
the at least one first miRNA has a nucleotide sequence according to SEQ ID NO: 8 and the at least one second miRNA has a nucleotide sequence according to SEQ ID NO: 35,
the at least one first miRNA has a nucleotide sequence according to SEQ ID NO: 9 and the at least one second miRNA has a nucleotide sequence according to SEQ ID NO: 27,
the at least one first miRNA has a nucleotide sequence according to SEQ ID NO: 10 and the at least one second miRNA has a nucleotide sequence according to SEQ ID NO: 35,
the at least one first miRNA has a nucleotide sequence according to SEQ ID NO: 13 and the at least one second miRNA has a nucleotide sequence according to SEQ ID NO: 35,
the at least one first miRNA has a nucleotide sequence according to SEQ ID NO: 15 and the at least one second miRNA has a nucleotide sequence according to SEQ ID NO: 35,
the at least one first miRNA has a nucleotide sequence according to SEQ ID NO: 24 and the at least one second miRNA has a nucleotide sequence according to SEQ ID NO: 35, or
the at least one first miRNA has a nucleotide sequence according to SEQ ID NO: 24 and the at least one second miRNA has a nucleotide sequence according to SEQ ID NO: 38.

In a third aspect, the invention provides the use of the method according to the first and second aspect of the invention for:
(i.) comparing the health status between one or more subjects,
(ii.) monitoring the health status in a subject,
(iii.) monitoring the state of the immune system in a subject, and/or
(iv.) monitoring the response to therapeutic treatment, preferably drug treatment, in a subject.

In a fourth aspect, the invention provides a method for monitoring the health status in a subject, comprising the steps of:
(a) assessing the health status in a subject at a first point in time by carrying out the method according to the first or second aspect,
(b) assessing the health status in the subject at one or more later points in time by carrying out the method according to the first or second aspect, and
(c) comparing the health status diagnosed in step (a) with the health status diagnosed in step (b), thereby monitoring the health status in the subject.

This summary of the invention does not necessarily describe all features of the present invention. Other embodiments will become apparent from a review of the ensuing detailed description.

### DETAILED DESCRIPTION OF THE INVENTION

### Definitions

Before the present invention is described in detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.

Preferably, the terms used herein are defined as described in "A multilingual glossary of biotechnological terms: (IUPAC Recommendations)", H.G.W. Leuenberger, B. Nagel, and H. Kölbl, Eds., Helvetica Chimica Acta, CH-4010 Basel, Switzerland, (1995).

To practice the present invention, unless otherwise indicated, conventional methods of chemistry, biochemistry, and recombinant DNA techniques are employed which are explained in the literature in the field (cf., e.g., Molecular Cloning: A Laboratory Manual, 2nd Edition, J. Sambrook et al. eds., Cold Spring Harbor Laboratory Press, Cold Spring Harbor 1989).

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integers or steps.

As used in this specification and in the appended claims, the singular forms "a", "an", and "the" include plural referents, unless the content clearly dictates otherwise. For example, the term "a test compound" also includes "test compounds".

The terms "microRNA" or "miRNA" refer to single-stranded RNA molecules of at least 10 nucleotides and of not more than 35 nucleotides covalently linked together. Preferably, the polynucleotides of the present invention are molecules of 10 to 33 nucleotides or 15 to 30 nucleotides in length, more preferably of 17 to 27 nucleotides or 18 to 26 nucleotides in length, i.e. 10, 11, 12, 13, 14, 15, 16, 17, 18, 19, 20, 21, 22, 23, 24, 25, 26, 27, 28, 29, 30, 31, 32, 33, 34, or 35 nucleotides in length. The terms "microRNA^{∗}" or "miRNA^{∗}" refer to miRNA molecules derived from the passenger strand upon processing. In the context of the present invention, the terms "miRNA" and "miRNA^{∗}" are interchangeable used. The miRBase (www.mirbase.org) is a well established repository and searchable database of published miRNA sequences and annotation.

The term "whole blood sample", as used in the context of the present invention, refers to a blood sample originating from a subject containing all blood fractions, including both the cellular (red blood cells, white blood cells, platelets) and the extra-cellar blood fractions (serum, plasma). The whole blood sample may be derived by removing blood from a subject by conventional blood collecting techniques, but may also be provided by using previously isolated and/or stored blood samples. Preferably, the whole blood sample from a subject (e.g. human or animal) has a volume of between 0.1 and 20 ml, more preferably of between 0.5 and 15 ml, more preferably between 1 and 10 ml and most preferably between 2 and 7.5 ml, i.e. 0.1, 0.2, 0.3, 0.4, 0.5, 0.6, 0.7, 0.8, 0.9, 1, 2, 2.5, 3, 4, 5, 6, 7, 8, 9, 10, 11, 12, 13, 14, 15, 16, 17, 18, 19, or 20 ml. Preferably the whole blood sample is collected by means of a blood collection tube, preferably it is collected in a PAXgene Blood RNA tube, in a Tempus Blood RNA tube, in an EDTA-tube (e.g. K2-EDTA Monovette tube), in a Na-citrate tube, Heparin-tube or in a ACD-tube (Acid citrate dextrose). Preferably, when the whole blood sample is collected, the RNA-fraction, especially the miRNA fraction, may be protected/guarded against degradation. For this purpose special collection tubes (e.g. PAXgene Blood RNA tubes from Preanalytix, Tempus Blood RNA tubes from Applied Biosystems) or additives (e.g. RNAlater from Ambion, RNAsin from Promega), that stabilize the RNA fraction and/or the miRNA fraction, may be employed.

The term "blood cell sample", as used in the context of the present invention, refers to a preparation of the whole blood sample, that comprises or substantially comprises blood cells (red blood cells, white blood cells, platelets), more preferably the blood cell sample contains red blood cells, white blood cells and platelets. Preferably, the blood cell sample does not contain miRNAs that originate from the extra-cellular fraction (e.g. plasma, serum) of whole blood or it does contain miRNAs that originate from the extra-cellular fraction (e.g. plasma, serum) only in minor amounts in order that these do not or do not substantially contribute to the expression profile of the set of one or more miRNA in a blood cell sample according to the present invention. Blood cell samples comprising red blood cells, white blood cells and/or platelets or blood cell samples containing red blood cells, white blood cells and platelets are obtained from processing of whole blood samples collected in PAXgene Blood RNA Tubes, Tempus Blood RNA Tubes, EDTA-tubes (e.g. K2-EDTA Monovette tubes), Na-citrate tubes or Heparin-tubes, maintaining or substantially maintaining the initial cellular distribution (blood cell composition) of the whole blood sample. From the blood cell sample the total RNA (comprising the short RNA fraction including the miRNA fraction) is isolated and which is used for determining the expression profile of a set of at least one, preferably of at least two miRNAs of a subject in said sample.

The term "blood cellular fractions" as used herein relates to cellular fractions, namely blood cell fractions, derived from a blood sample. Blood cell fraction include red blood cells (erythrocytes), white blood cells (leukocytes), platelets (thrombocytes) or subfractions (i.e. Tcells, Bcells, NK-cells, neutrophils, granulocytes, reticulocytes, etc.) of the aforementioned blood cell types thereof or combinations from the aforementioned entities. Preferably, blood cellular fractions do not contain miRNAs that originate from the extra-cellular fraction (e.g. plasma, serum) of whole blood or do contain miRNAs that originate from the extra-cellular fraction (e.g. plasma, serum) only in minor amounts in order that these do not or do not substantially contribute to the expression profile of the set of one or more miRNA in blood cellular fractions according to the present invention. In the context of the present invention, the terms "blood cell sample" and "sample of blood cellular fractions" are interchangeable used.

The term "total RNA" as used herein relates to the isolated RNA comprising the miRNA-fraction present in the respective blood cell sample, which is derived from a whole blood sample. Preferably, the total RNA according to the present invention contains the miRNA-fraction or contains a miRNA-enriched fraction of said total RNA. The total RNA (comprising the miRNA-fraction or miRNA-enriched fraction) is obtained by lysis (e.g. Trizol) of the blood cells in the blood cell sample, followed by RNA purification e.g. by phenol/chloroform extraction and/or separation based techniques (e.g. glass fiber filter column, silica-membrane column). Examples of kits for RNA isolation and purification include the miRNeasy Kits (Qiagen), PAXgene Blood miRNA Kit (Qiagen), mirVana PARIS Kit (Life Technologies), PARIS Kit (Life Technologies), Tempus Spin RNA Isolation Kit (Life Technologies).

The term "disease-regulated miRNAs", as used herein, refers to at least one fixed defined miRNA comprised in a set which is known to be differential (regulated) between healthy subjects and diseased subjects. Preferred disease-regulated miRNAs are selected from the group of miRNAs listed in Figure 1, namely miRNAs with SEQ ID NO: 1 to 26.

The term "disease-preserved miRNAs", as used herein, refers to at least one fixed defined miRNA comprised in a set which is known to be not differential (not regulated) between healthy subjects and diseased subjects. Preferred disease-preserved miRNAs are selected from the group of miRNAs listed in Figure 2, namely miRNAs with SEQ ID NO: 27 to 59.

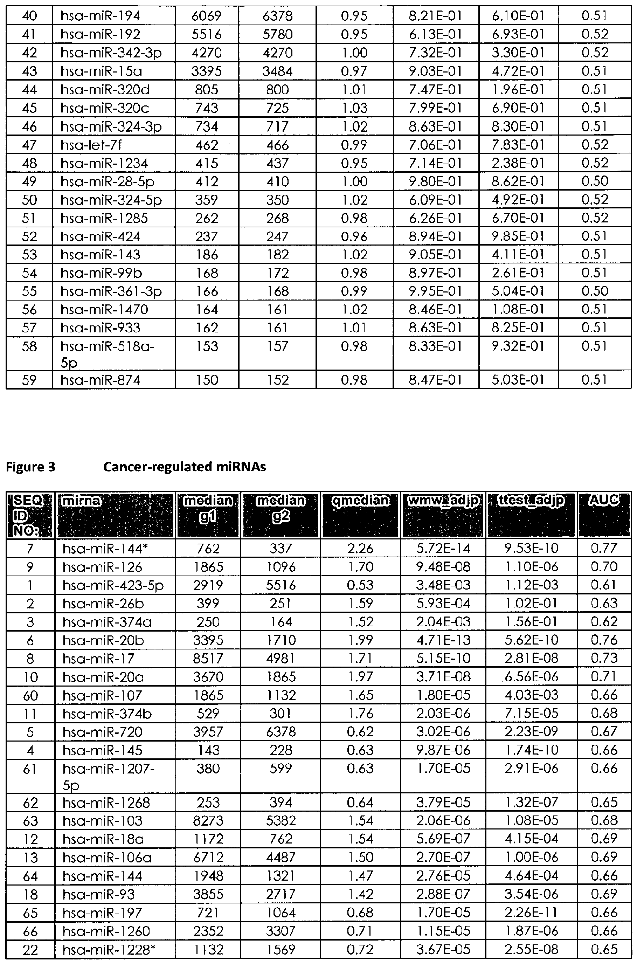

The term "cancer-regulated miRNAs", as used herein, refers to at least one fixed defined miRNA comprised in a set which is known to be differential (regulated) between healthy subjects and subjects suffering from cancer. Preferred cancer-regulated miRNAs are selected from the group of miRNAs listed in Figure 3.

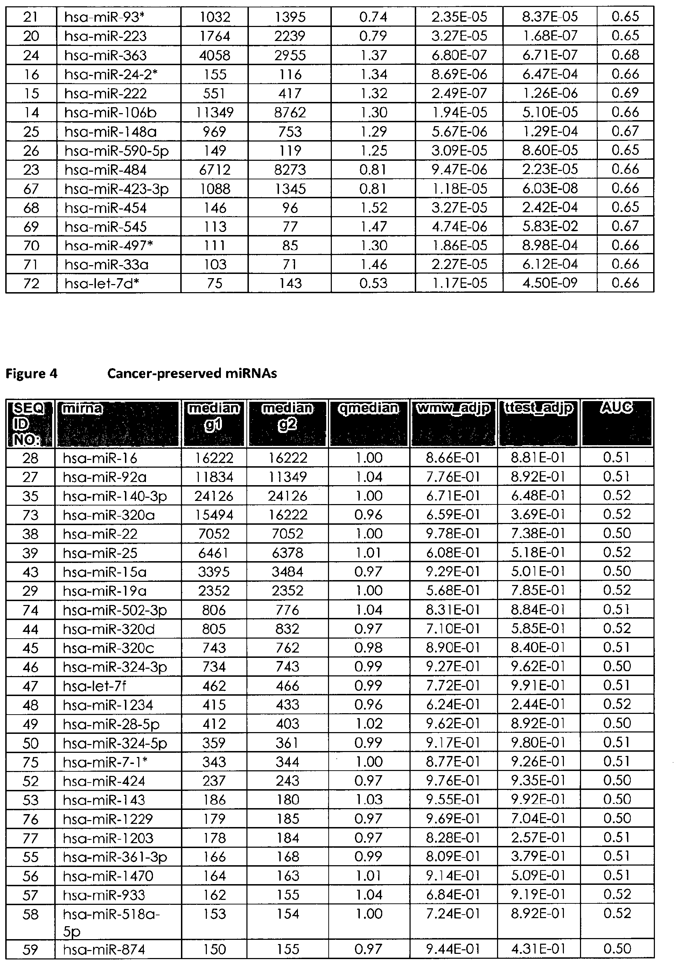

The term "cancer-preserved miRNAs", as used herein, refers to at least one fixed defined miRNA comprised in a set which is known to be not differential (not regulated) between healthy subjects and subjects suffering from cancer. Preferred cancer-preserved miRNAs are selected from the group of miRNAs listed in Figure 4.

The term "expression profile" as used in the context of the present invention, refers to the determination of the miRNA expression profile or refers to a measure that correlates with the miRNA expression in a sample (e.g. in a blood cell sample or a blood cellular fraction derived from a whole blood sample). By determining the miRNA expression profile, each miRNA is represented by a numerical value. The higher the value of an individual miRNA, the higher is the expression level of said miRNA, or the lower the value of an individual miRNA, the lower is the expression level of said miRNA. The expression profile may be generated by any convenient means, e.g. nucleic acid hybridization (e.g. to a microarray), nucleic acid amplification (PCR, RT-PCR, qRT-PCR, high-throughput RT-PCR), ELISA for quantitation, next generation sequencing (e.g. ABI SOLID, Illumina Genome Analyzer, Roche/454 GS FLX), flow cytometry (e.g. LUMINEX, Milipore Guava) and the like, that allow the analysis of miRNA expression profile in a subject and comparison between samples. The sample material measured by the aforementioned means are derived from a blood cell sample and may be a total RNA, labeled total RNA, amplified total RNA, cDNA, labeled cDNA, amplified cDNA, miRNA, labeled miRNA, amplified miRNA or any derivatives that may be generated from the aforementioned RNA/DNA species. The "expression profile", as used herein, relates to a collection of expression profiles of one or more miRNAs, preferably of least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38 ,39,40, 41, 42, 43, 44, 45, 46, 47, 48, 49, 50, 51, 51, 53, 54, 55, 56, 57, 58, 59 or more miRNAs, preferably selected from miRNAs with SEQ ID NO: 1 to 77, more preferably from miRNAs with SEQ ID NO: 1 to 59.

The term "determining an expression profile in (from) a blood cell sample" as used herein, relates to the determination of the expression profile from the miRNAs present in said blood cell sample, therefore it is a measure that correlates with the miRNAs present in said blood cell sample. Herein, all steps or transformations required to bring the blood cell sample into a form which allows to record the expression profile by any convenient means (e.g. nucleic acid hybridisation, nucleic acid amplification, polymerase extension, mass spectroscopy, flow cytometry, sequencing, next-generation-sequencing) and which are known to the person skilled in the art, are included, e.g. cell-lysis, RNA-isolation, RNA-labeling, polymerase extension of RNA, ligation of RNA reverse-transcription into cDNA, amplification of the cDNA, labelling of cDNA, etc.

The term "diagnosis" as used herein refers to the process of determining or monitoring a possible disease or disorder and therefore is a process attempting to define or monitor the health status of a subject. The determination of the expression profile of one or more miRNAs selected from the list of disease-regulated (Figure 1; SEQ ID NO: 1 to 26) and one or more disease-preserved miRNAs (Figure 2, SEQ ID NO: 27 to 59), correlates with the health status of a subject. Nucleic acid hybridization may be performed using a microarray/biochip or *in situ* hybridization. Nucleic acid amplification may be performed using real time polymerase chain reaction (RT-PCR) such as real time quantitative polymerase chain reaction (RT qPCR). The aforesaid real time polymerase chain reaction (RT-PCR) may include the following steps: (i) extracting the total RNA from a blood cell sample derived from a whole blood sample of a subject, (ii) obtaining cDNA samples by RNA reverse transcription (RT) reaction using universal or miRNA-specific RT primers (e.g. stem-loop RT primers); (iii) optionally amplifying the obtained cDNA (e.g. by PCR such as a specific target amplification (STA), (iv) detecting the miRNA(s) level in the sample by means of (real time) quantification of the cDNA of step (ii) or (iii) e.g. by real time polymerase chain reaction wherein a fluorescent dye (e.g. SYBR Green) or a fluorescent probe (e.g. Taqman probe) are added. In Step (i) the isolation and/or extraction of RNA may be omitted in cases where the RT-PCR is conducted directly from the miRNA-containing sample. Kits for determining a miRNA expression profile by real time polymerase chain reaction (RT-PCR) are e.g. from Life Technologies, Applied Biosystems, Ambion, Roche, Qiagen, Invitrogen, SABiosciences, Exiqon. In Step(ii) the universal or miRNA-specific RT primers may be single-stranded (dna) oligonucleotides that are ligated to the 3'-end of the miRNA, resulting in a non-naturally occurring miRNA-dna chimera molecule, which is transcribed into to dna by the reverse transcriptase enzyme.

Nucleic acid sequencing may be performed by conventional Sanger sequencing or by socalled "next generation sequencing (NGS)", which includes -but is not limited to - sequencing-by-synthesis, sequencing-by-ligation, single molecule sequencing, nanopore-sequencing or the like techniques. The NGS may include the following steps : (i) extracting the total RNA from a blood cell sample derived from a whole blood sample of a subject, (ii) obtaining cDNA samples by RNA reverse transcription (RT) reaction using universal or miRNA-specific RT primers (e.g. stem-loop RT primers); (iii) optionally amplifying the obtained cDNA-transcripts (e.g. by PCR), (iv) subjecting the cDNA-transcripts of the miRNAs to the aforementioned sequencing techniques, such as sequencing-by-synthesis, sequencing-by-ligation. In step (ii) single-stranded oligonucleotides are ligated to the 3'-end comprising a universal or miRNA-specific reverse transcriptase (RT)-primer. In case that the cDNA should be amplified in step (iii), the oligo ligated to the 3'-end also comprises a PCT-primer sequence and a single-stranded oligonucleotide is also ligated to the 5'-end, which also comprises a PCR-primer sequence. In step (iii) the cDNA is then amplified making use of the primer sequences introduced in step (ii) at the 3' and 5'-end.

The term "health status", as used in the context of the present invention, relates to a measure of the status of the overall health in a subject, including, but not limited to assessment of the presence or absence of an illness or a disease in said subject. According to the present invention, the health status in a subject may be assessed by determining a set of one or more disease-regulated miRNAs in combination with a set of one or more disease-preserved miRNAs from a blood sample, preferably from a blood cell sample or from a blood cellular fraction of said subject. The health status may the presence or absence of a disease in a subject and/or the presence or absence of cancer in a subject. With the methods of the present invention, a change of the health status is diagnosed. Said change of the health status may be an improvement or a worsening of the health status, e.g. the improvement or worsening of a disease and/or the improvement or worsening of cancer.

The term "disease", as used herein, refers to an abnormal condition that affects the body of a subject. A disease is often construed as a medical condition associated with specific symptoms and signs. A disease may be caused by factors originally from an external source, such as infectious disease, or it may be caused by internal dysfunctions, such as autoimmune diseases. In humans, "disease" is often used more broadly to refer to any condition that causes pain, dysfunction, distress, social problems, or death to the subject afflicted, or similar problems for those in contact with the subject. In this broader sense, it sometimes includes injuries, disabilities, disorders, syndromes, infections, isolated symptoms, deviant behaviors, and atypical variations of structure and function, while in other contexts and for other purposes these may be considered distinguishable categories. Diseases usually affect subjects not only physically, but also emotionally, as contracting and living with many diseases can alter one's perspective on life, and one's personality. The term "disease", as used herein, also covers cancer.

The term "infectious disease", as used herein, refers to any disease which can be transmitted from subject to subject and is caused by a microbial agent (e.g. common cold). Infectious diseases are known in the art and include, for example, a viral disease, a bacterial disease, or a parasitic disease, which diseases are caused by a virus, a bacterium, and a parasite, respectively. In this regard, the infectious disease can be, for example, hepatitis, sexually transmitted diseases (e.g. chlamydia or gonorrhea), tuberculosis, HIV/acquired immune deficiency syndrome (AIDS), diphtheria, hepatitis B, hepatitis C, cholera, severe acute respiratory syndrome (SARS), the bird flu, and influenza.

The term "autoimmune disease", as used herein, refers to any disease in which the body produces an immunogenic (i.e. immune system) response to some constituent of its own tissue. In other words, the immune system loses its ability to recognize some tissue or system within the body as self and targets and attacks it as if it were foreign. Autoimmune diseases can be classified into those in which predominantly one organ is affected (e.g. hemolytic anemia and anti-immune thyroiditis), and those in which the autoimmune disease process is diffused through many tissues (e.g. systemic lupus erythematosus). For example, multiple sclerosis is thought to be caused by T cells attacking the sheaths that surround the nerve fibers of the brain and spinal cord. This results in loss of coordination, weakness, and blurred vision. Autoimmune diseases are known in the art and include, for instance, Hashimoto's thyroiditis, Grave's disease, lupus, multiple sclerosis, rheumatic arthritis, hemolytic anemia, anti-immune thyroiditis, systemic lupus erythematosus, celiac disease, Crohn's disease, colitis, diabetes, scleroderma, psoriasis, and the like.

The terms "cancer disease" or "cancer", as used herein, refer to or describe the physiological condition in a subject that is typically characterized by unregulated cell growth. Examples of cancers include, but are not limited to, carcinoma, lymphoma, blastoma, sarcoma, and leukemia. More particularly, examples of such cancers include bone cancer, blood cancer lung cancer, liver cancer, pancreatic cancer, skin cancer, cancer of the head or neck, cutaneous or intraocular melanoma, uterine cancer, ovarian cancer, rectal cancer, cancer of the anal region, stomach cancer, colon cancer, breast cancer, prostate cancer, uterine cancer, carcinoma of the sexual and reproductive organs, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the bladder, cancer of the kidney, renal cell carcinoma, carcinoma of the renal pelvis, neoplasms of the central nervous system (CNS), neuroectodermal cancer, spinal axis tumors, glioma, meningioma, and pituitary adenoma. The term "cancer" according to the invention also comprises cancer metastases.

The term "subject", as used in the context of the present invention, means an individual for which the health status is to be assessed, hence a subject may be one that is afflicted by a disease and/or cancer or not, or may be one that is suspected to be afflicted by a disease and/or cancer or not. With the methods of the present invention, a change of the health status is diagnosed. Said change of the health status may be an improvement or a worsening of the health status, e.g. the improvement or worsening of a disease and/or the improvement or worsening of cancer.

The term "therapeutic treatment" relates to any treatment which improves the health status and/or prolongs (increases) the lifespan of a subject. Said treatment may eliminate the disease in a subject, arrest or slow the development of a disease in a subject, inhibit or slow the development of a disease in a subject, decrease the frequency or severity of symptoms in a subject, and/or decrease the recurrence in a subject who currently has or who previously has had a disease.

### Embodiments of the invention

The present invention will now be further described. In the following passages different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous, unless clearly indicated to the contrary.

In a first aspect, the present invention relates to a method for assessing the health status in a subject comprising the steps of:
(a) isolating total intracellular RNA from erythrocytes, leukocytes, and thrombocytes which have been obtained from a whole blood sample which has been taken from a subject,
(b) determining in said total intracellular RNA an expression profile of one or more first miRNAs, wherein the one or more first miRNAs have a nucleotide sequence selected from the group consisting of SEQ ID NO: 1 to SEQ ID NO: 16 and SEQ ID NO: 20 to SEQ ID NO: 26,
(c) determining in said total intracellular RNA an expression profile of one or more second miRNAs, wherein the one or more second miRNAs have a nucleotide sequence selected from the group consisting of SEQ ID NO: 27, SEQ ID NO: 29, SEQ ID NO: 35, SEQ ID NO: 38, SEQ ID NO: 39, SEQ ID NO: 43 to SEQ ID NO: 47, SEQ ID NO: 49, SEQ ID NO: 50, SEQ ID NO: 52, SEQ ID NO: 55 to SEQ ID NO: 57, and SEQ ID NO: 59,
(d) normalizing the expression profile of said one or more first miRNAs to the expression profile of said one or more second miRNAs, thereby obtaining one or more relative expression levels of: SEQ ID NO: 1/SEQ ID NO: 45, SEQ ID NO: 1/SEQ ID NO: 59, SEQ ID NO: 1/SEQ ID NO: 56, SEQ ID NO: 2/SEQ ID NO: 27, SEQ ID NO: 2/SEQ ID NO: 35, SEQ ID NO: 3/SEQ ID NO: 27, SEQ ID NO: 4/SEQ ID NO: 55, SEQ ID NO: 4/SEQ ID NO: 47, SEQ ID NO: 4/SEQ ID NO: 29^{∗}, SEQ ID NO: 4/SEQ ID NO: 56, SEQ ID NO: 4/SEQ ID NO: 59, SEQ ID NO: 4/SEQ ID NO: 43, SEQ ID NO: 5/SEQ ID NO: 59, SEQ ID NO: 5/SEQ ID NO: 29, SEQ ID NO: 5/SEQ ID NO: 55, SEQ ID NO: 5/SEQ ID NO: 45, SEQ ID NO: 5/SEQ ID NO: 44, SEQ ID NO: 5/SEQ ID NO: 57, SEQ ID NO: 5/SEQ ID NO: 46, SEQ ID NO: 5/SEQ ID NO: 50, SEQ ID NO: 5/SEQ ID NO: 49, SEQ ID NO: 5/SEQ ID NO: 56, SEQ ID NO: 5/SEQ ID NO: 47, SEQ ID NO: 6/SEQ ID NO: 35, SEQ ID NO: 6/SEQ ID NO: 27, SEQ ID NO: 6/SEQ ID NO: 38, SEQ ID NO: 6/SEQ ID NO: 29, SEQ ID NO: 6/SEQ ID NO: 43, SEQ ID NO: 6/SEQ ID NO: 49, SEQ ID NO: 6/SEQ ID NO: 50, SEQ ID NO: 6/SEQ ID NO: 52, SEQ ID NO: 6/SEQ ID NO: 39, SEQ ID NO: 7/SEQ ID NO: 27, SEQ ID NO: 7/SEQ ID NO: 35, SEQ ID NO: 7/SEQ ID NO: 29, SEQ ID NO: 7/SEQ ID NO: 43, SEQ ID NO: 7/SEQ ID NO: 50, SEQ ID NO: 7/SEQ ID NO: 39, SEQ ID NO: 7/SEQ ID NO: 38, SEQ ID NO: 7/SEQ ID NO: 47, SEQ ID NO: 8/SEQ ID NO: 35, SEQ ID NO: 8/SEQ ID NO: 27, SEQ ID NO: 8/SEQ ID NO: 38, SEQ ID NO: 8/SEQ ID NO: 39, SEQ ID NO: 8/SEQ ID NO: 43, SEQ ID NO: 9/SEQ ID NO: 35, SEQ ID NO: 9/SEQ ID NO: 27, SEQ ID NO: 9/SEQ ID NO: 38, SEQ ID NO: 9/SEQ ID NO: 43, SEQ ID NO: 9/SEQ ID NO: 29, SEQ ID NO: 9/SEQ ID NO: 39, SEQ ID NO: 10/SEQ ID NO: 35, SEQ ID NO: 10/SEQ ID NO: 27, SEQ ID NO: 10/SEQ ID NO: 38, SEQ ID NO: 10/SEQ ID NO: 49, SEQ ID NO: 10/SEQ ID NO: 39, SEQ ID NO: 10/SEQ ID NO: 43, SEQ ID NO: 11/SEQ ID NO: 27, SEQ ID NO: 11/SEQ ID NO: 35, SEQ ID NO: 11/SEQ ID NO: 39, SEQ ID NO: 11/SEQ ID NO: 38, SEQ ID NO: 12/SEQ ID NO: 35, SEQ ID NO: 13/SEQ ID NO: 35, SEQ ID NO: 13/SEQ ID NO: 27, SEQ ID NO: 13/SEQ ID NO: 39, SEQ ID NO: 14/SEQ ID NO: 35, SEQ ID NO: 15/SEQ ID NO: 35, SEQ ID NO: 15/SEQ ID NO: 38, SEQ ID NO: 15/SEQ ID NO: 27, SEQ ID NO: 15/SEQ ID NO: 39, SEQ ID NO: 16/SEQ ID NO: 35, SEQ ID NO: 20/SEQ ID NO: 29, SEQ ID NO: 20/SEQ ID NO: 59, SEQ ID NO: 20/SEQ ID NO: 57, SEQ ID NO: 20/SEQ ID NO: 38, SEQ ID NO: 20/SEQ ID NO: 39, SEQ ID NO: 20/SEQ ID NO: 47, SEQ ID NO: 21/SEQ ID NO: 50, SEQ ID NO: 21/SEQ ID NO: 59, SEQ ID NO: 21/SEQ ID NO: 29^{∗}, SEQ ID NO: 21/SEQ ID NO: 57, SEQ ID NO: 21/SEQ ID NO: 55, SEQ ID NO: 21/SEQ ID NO: 49, SEQ ID NO: 21/SEQ ID NO: 56, SEQ ID NO: 22/SEQ ID NO: 59, SEQ ID NO: 22/SEQ ID NO: 47, SEQ ID NO: 22/SEQ ID NO: 56, SEQ ID NO: 22/SEQ ID NO: 57, SEQ ID NO: 22/SEQ ID NO: 29^{∗}, SEQ ID NO: 23/SEQ ID NO: 59, SEQ ID NO: 23/SEQ ID NO: 56, SEQ ID NO: 23/SEQ ID NO: 57, SEQ ID NO: 24/SEQ ID NO: 27, SEQ ID NO: 24/SEQ ID NO: 35, SEQ ID NO: 24/SEQ ID NO: 38, SEQ ID NO: 24/SEQ ID NO: 39, SEQ ID NO: 24/SEQ ID NO: 43, SEQ ID NO: 25/SEQ ID NO: 35, SEQ ID NO: 25/SEQ ID NO: 39, SEQ ID NO: 25/SEQ ID NO: 43, SEQ ID NO: 25/SEQ ID NO: 38, SEQ ID NO: 25/SEQ ID NO: 27, SEQ ID NO: 26/SEQ ID NO: 35, or SEQ ID NO: 26/SEQ ID NO: 27,
(e) comparing the one or more relative expression levels of step (d) to one or more relative reference expression levels, and
(f) assessing a change of the health status in said subject when the comparison to said one or more relative reference expression levels is altered.

According to the present invention the whole blood sample is collected from the subject by conventional blood draw techniques. Blood collection tubes suitable for collection of whole blood include EDTA- (e.g. K2-EDTA Monovette tube), Na-citrate-, ACD-, Heparin-, PAXgene Blood RNA-, Tempus Blood RNA-tubes. According to the present invention the collected whole blood sample, which intermediately may be stored before use, is processed to result in a blood cell sample of whole blood. This is achieved by separation of the blood cell fraction (the cellular fraction of whole blood) from the serum/plasma fraction (the extra-cellular fraction of whole blood), e.g. by centrifugation techniques. It is preferred, that the blood cell sample derived from the whole blood sample comprises red blood cells, white blood cells and/or platelets, it is more preferred that the blood cell sample derived from the whole blood sample comprises red blood cells, white blood cells and platelets.

The total RNA, including the miRNA fraction, is isolated from said blood cells present within said blood cell samples. Kits for isolation of total RNA including the miRNA fraction or kits for isolation of the miRNA-fraction are well known to those skilled in the art, e.g. miRNeasykit (Qiagen, Hilden, Germany), Paris-kit (Life Technologies, Weiterstadt, Germany). The determination of the expression profile may be by any convenient means for determining miRNAs or miRNA profiles. A variety of techniques are well known to those skilled in the art, e.g. nucleic acid hybridisation, nucleic acid amplification, sequencing, mass spectroscopy, flow cytometry based techniques or combinations thereof.

The normalizing generates relative expression levels. Preferably said relative expression levels are ratios that are obtained from one or more first (disease- and/or cancer-regulated) miRNAs and from one or more second (disease- and/or cancer-preserved) miRNAs from one or more subjects to be assessed. More preferably, said relative expression levels are obtained by dividing the expression level of one or more first (disease- and/or cancer-regulated) miRNAs by the expression level of one or more second (disease- and/or cancer-preserved) miRNAs. Hence, the second (disease- and/or cancer-preserved) miRNAs act as normalizers for said first (disease- and/or cancer-regulated) miRNAs. Examples of said relative expression levels are the ratio of the miRNA expression levels of hsa-miR-423-5p (SEQ ID NO:1) divided by hsa-miR-92a (SEQ ID NO:27), or hsa-miR-423-5p (SEQ ID NO:1) divided by hsa-miR-16 (SEQ ID NO:28), or hsa-miR-423-5p (SEQ ID NO:1) divided by hsa-miR-19a (SEQ ID NO:29), or hsa-miR-423-5p (SEQ ID NO:1) divided by hsa-miR-140-3p (SEQ ID NO:35), or hsa-miR-423-5p (SEQ ID NO:1) divided by hsa-miR-425 (SEQ ID NO:36), or hsa-miR-423-5p (SEQ ID NO:1) divided by hsa-miR-30b (SEQ ID NO:37), or hsa-miR-423-5p (SEQ ID NO:1) divided by hsa-miR-22 (SEQ ID NO:38), or hsa-miR-423-5p (SEQ ID NO:1) divided by hsa-miR-25 (SEQ ID NO:39), or hsa-miR-26b (SEQ ID NO:2) divided by hsa-miR-92a (SEQ ID NO:27), or hsa-miR-26b (SEQ ID NO:2) divided by hsa-miR-16 (SEQ ID NO:28), or hsa-miR-26b (SEQ ID NO:2) divided by hsa-miR-19a (SEQ ID NO:29), or hsa-miR-26b (SEQ ID NO:2) divided by hsa-miR-140-3p (SEQ ID NO:35), or hsa-miR-26b (SEQ ID NO:2) divided by hsa-miR-425 (SEQ ID NO:36), or hsa-miR-26b (SEQ ID NO:2) divided by hsa-miR-30b (SEQ ID NO:37), or hsa-miR-26b (SEQ ID NO:2) divided by hsa-miR-22 (SEQ ID NO:38), or hsa-miR-26b (SEQ ID NO:2) divided by hsa-miR-25 (SEQ ID NO:39), or hsa-miR-374a (SEQ ID NO:3) divided by hsa-miR-92a (SEQ ID NO:27), or hsa-miR-374a (SEQ ID NO:3) divided by hsa-miR-16 (SEQ ID NO:28), or hsa-miR-374a (SEQ ID NO:3) divided by hsa-miR-19a (SEQ ID NO:29), or hsa-miR-374a (SEQ ID NO:3) divided by hsa-miR-140-3p (SEQ ID NO:35), or hsa-miR-374a (SEQ ID NO:3) divided by hsa-miR-425 (SEQ ID NO:36), or hsa-miR-374a (SEQ ID NO:3) divided by hsa-miR-30b (SEQ ID NO:37), or hsa-miR-374a (SEQ ID NO:3) divided by hsa-miR-22 (SEQ ID NO:38), or hsa-miR-374a (SEQ ID NO:3) divided by hsa-miR-25 (SEQ ID NO:39), or hsa-miR-720 (SEQ ID NO:5) divided by hsa-miR-92a (SEQ ID NO:27), or hsa-miR-720 (SEQ ID NO:5) divided by hsa-miR-16 (SEQ ID NO:28), or hsa-miR-720 (SEQ ID NO:5) divided by hsa-miR-19a (SEQ ID NO:29), or hsa-miR-720 (SEQ ID NO:5) divided by hsa-miR-140-3p (SEQ ID NO:35), or hsa-miR-720 (SEQ ID NO:5) divided by hsa-miR-425 (SEQ ID NO:36), or hsa-miR-720 (SEQ ID NO:5) divided by hsa-miR-30b (SEQ ID NO:37), or hsa-miR-720 (SEQ ID NO:5) divided by hsa-miR-22 (SEQ ID NO:38), or hsa-miR-720 (SEQ ID NO:5) divided by hsa-miR-25 (SEQ ID NO:39), or hsa-miR-20b (SEQ ID NO:6) divided by hsa-miR-92a (SEQ ID NO:27), or hsa-miR-20b (SEQ ID NO:6) divided by hsa-miR-16 (SEQ ID NO:28), or hsa-miR-20b (SEQ ID NO:6) divided by hsa-miR-19a (SEQ ID NO:29), or hsa-miR-20b (SEQ ID NO:6) divided by hsa-miR-140-3p (SEQ ID NO:35), or hsa-miR-20b (SEQ ID NO:6) divided by hsa-miR-425 (SEQ ID NO:36), or hsa-miR-20b (SEQ ID NO:6) divided by hsa-miR-30b (SEQ ID NO:37), or hsa-miR-20b (SEQ ID NO:6) divided by hsa-miR-22 (SEQ ID NO:38), or hsa-miR-20b (SEQ ID NO:6) divided by hsa-miR-25 (SEQ ID NO:39), or hsa-miR-144^{∗} (SEQ ID NO:7) divided by hsa-miR-92a (SEQ ID NO:27), or hsa-miR-144^{∗} (SEQ ID NO:7) divided by hsa-miR-16 (SEQ ID NO:28), or hsa-miR-144^{∗} (SEQ ID NO:7) divided by hsa-miR-19a (SEQ ID NO:29), or hsa-miR-144^{∗} (SEQ ID NO:7) divided by hsa-miR-140-3p (SEQ ID NO:35), or hsa-miR-144^{∗} (SEQ ID NO:7) divided by hsa-miR-425 (SEQ ID NO:36), or hsa-miR-144^{∗} (SEQ ID NO:7) divided by hsa-miR-30b (SEQ ID NO:37), or hsa-miR-144^{∗} (SEQ ID NO:7) divided by hsa-miR-22 (SEQ ID NO:38), or hsa-miR-144^{∗} (SEQ ID NO:7) divided by hsa-miR-25 (SEQ ID NO:39), or hsa-miR-17 (SEQ ID NO:8) divided by hsa-miR-92a (SEQ ID NO:27), or hsa-miR-17 (SEQ ID NO:8) divided by hsa-miR-16 (SEQ ID NO:28), or hsa-miR-17 (SEQ ID NO:8) divided by hsa-miR-19a (SEQ ID NO:29), or hsa-miR-17 (SEQ ID NO:8) divided by hsa-miR-140-3p (SEQ ID NO:35), or hsa-miR-17 (SEQ ID NO:8) divided by hsa-miR-425 (SEQ ID NO:36), or hsa-miR-17 (SEQ ID NO:8) divided by hsa-miR-30b (SEQ ID NO:37), or hsa-miR-17 (SEQ ID NO:8) divided by hsa-miR-22 (SEQ ID NO:38), or hsa-miR-17 (SEQ ID NO:8) divided by hsa-miR-25 (SEQ ID NO:39), or hsa-miR-126 (SEQ ID NO:9) divided by hsa-miR-92a (SEQ ID NO:27), or hsa-miR-126 (SEQ ID NO:9) divided by hsa-miR-16 (SEQ ID NO:28), or hsa-miR-126 (SEQ ID NO:9) divided by hsa-miR-19a (SEQ ID NO:29), or hsa-miR-126 (SEQ ID NO:9) divided by hsa-miR-140-3p (SEQ ID NO:35), or hsa-miR-126 (SEQ ID NO:9) divided by hsa-miR-425 (SEQ ID NO:36), or hsa-miR-126 (SEQ ID NO:9) divided by hsa-miR-30b (SEQ ID NO:37), or hsa-miR-126 (SEQ ID NO:9) divided by hsa-miR-22 (SEQ ID NO:38), or hsa-miR-126 (SEQ ID NO:9) divided by hsa-miR-25 (SEQ ID NO:39), or hsa-miR-20a (SEQ ID NO:10) divided by hsa-miR-92a (SEQ ID NO:27), or hsa-miR-20a (SEQ ID NO:10) divided by hsa-miR-16 (SEQ ID NO:28), or hsa-miR-20a (SEQ ID NO:10) divided by hsa-miR-19a (SEQ ID NO:29), or hsa-miR-20a (SEQ ID NO:10) divided by hsa-miR-140-3p (SEQ ID NO:35), or hsa-miR-20a (SEQ ID NO:10) divided by hsa-miR-425 (SEQ ID NO:36), or hsa-miR-20a (SEQ ID NO:10) divided by hsa-miR-30b (SEQ ID NO:37), or hsa-miR-20a (SEQ ID NO:10) divided by hsa-miR-22 (SEQ ID NO:38), or hsa-miR-20a (SEQ ID NO:10) divided by hsa-miR-25 (SEQ ID NO:39), or hsa-miR-374b (SEQ ID NO:11) divided by hsa-miR-92a (SEQ ID NO:27), or hsa-miR-374b (SEQ ID NO: 11) divided by hsa-miR-16 (SEQ ID NO:28), or hsa-miR-374b (SEQ ID NO:11) divided by hsa-miR-19a (SEQ ID NO:29), or hsa-miR-374b (SEQ ID NO:11) divided by hsa-miR-140-3p (SEQ ID NO:35), or hsa-miR-374b (SEQ ID NO:11) divided by hsa-miR-425 (SEQ ID NO:36), or hsa-miR-374b (SEQ ID NO:11) divided by hsa-miR-30b (SEQ ID NO:37), or hsa-miR-374b (SEQ ID NO:11) divided by hsa-miR-22 (SEQ ID NO:38), or hsa-miR-374b (SEQ ID NO:11) divided by hsa-miR-25 (SEQ ID NO:39) or hsa-miR-144^{∗} (SEQ ID NO:7) divided by hsa-miR-16 (SEQ ID NO: 28), or hsa-miR-144^{∗} (SEQ ID NO:7) divided by hsa-miR-92a (SEQ ID NO: 27), or hsa-miR-126 (SEQ ID NO:9) divided by hsa-miR-16 (SEQ ID NO: 28), or hsa-miR-20b (SEQ ID NO:6) divided by hsa-miR-16 (SEQ ID NO: 28) , or hsa-miR-20b (SEQ ID NO:6) divided by hsa-miR-140-3p (SEQ ID NO: 35), or hsa-miR-20b (SEQ ID NO:6) divided by hsa-miR-92a (SEQ ID NO: 27), or hsa-miR-363 (SEQ ID NO:24) divided by hsa-miR-16 (SEQ ID NO: 28), or hsa-miR-20b (SEQ ID NO:6) divided by hsa-miR-22 (SEQ ID NO: 38), or hsa-miR-363 (SEQ ID NO:24) divided by hsa-miR-192 (SEQ ID NO: 41), or hsa-miR-20b (SEQ ID NO:6) divided by hsa-miR-192 (SEQ ID NO: 41), or hsa-miR-720 (SEQ ID NO:5) divided by hsa-miR-874 (SEQ ID NO: 59) , or hsa-miR-17 (SEQ ID NO:8) divided by hsa-miR-16 (SEQ ID NO: 28), or hsa-miR-20a (SEQ ID NO:10) divided by hsa-miR-140-3p (SEQ ID NO: 35), or hsa-miR-20a (SEQ ID NO:10) divided by hsa-miR-16 (SEQ ID NO: 28), or hsa-miR-144^{∗} (SEQ ID NO:7) divided by hsa-miR-30b (SEQ ID NO: 37), or hsa-miR-106a (SEQ ID NO:13) divided by hsa-miR-16 (SEQ ID NO: 28), or hsa-miR-17 (SEQ ID NO:8) divided by hsa-miR-140-3p (SEQ ID NO: 35), or hsa-miR-144^{∗} (SEQ ID NO:7) divided by hsa-miR-140-3p (SEQ ID NO: 35), or hsa-miR-106a (SEQ ID NO:13) divided by hsa-miR-140-3p (SEQ ID NO: 35), or hsa-miR-93^{∗} (SEQ ID NO:21) divided by hsa-miR-27b (SEQ ID NO: 34) , or hsa-miR-144^{∗} (SEQ ID NO:7) divided by hsa-miR-19a (SEQ ID NO: 29), or hsa-miR-222 (SEQ ID NO:15) divided by hsa-miR-140-3p (SEQ ID NO: 35), or hsa-miR-20b (SEQ ID NO:6) divided by hsa-miR-19a (SEQ ID NO: 29), or hsa-miR-363 (SEQ ID NO:24) divided by hsa-miR-22 (SEQ ID NO: 38), or hsa-miR-363 (SEQ ID NO:24) divided by hsa-miR-140-3p (SEQ ID NO: 35), or hsa-miR-93^{∗} (SEQ ID NO:21) divided by hsa-miR-99b (SEQ ID NO: 54), or hsa-miR-720 (SEQ ID NO:5) divided by hsa-miR-19a (SEQ ID NO: 29), or hsa-miR-720 (SEQ ID NO:5) divided by hsa-miR-361-3p (SEQ ID NO: 55) from one or more subjects to be diagnosed.

The reference to which the normalized expression level is compared is a relative reference expression level. It is preferred that the relative reference expression level is obtained in a comparable fashion from one or more reference subjects as the expression levels were obtained from the subjects to be assessed.

Preferably, said one or more relative reference expression levels are obtained comprising the steps of:
(i) isolating total intracellular RNA from erythrocytes, leukocytes, and thrombocytes which have been obtained from a whole blood sample which has been taken from one or more reference subjects,
(ii) determining in said total intracellular RNA a reference expression profile of one or more first miRNAs, wherein the one or more first miRNAs have a nucleotide sequence selected from the group consisting of SEQ ID NO: 1 to SEQ ID NO: 16 and SEQ ID NO: 20 to SEQ ID NO: 26,
(iii) determining in said total intracellular RNA a reference expression profile of one or more second miRNAs, wherein the one or more second miRNAs have a nucleotide sequence selected from the group consisting of SEQ ID NO: 27, SEQ ID NO: 29, SEQ ID NO: 35, SEQ ID NO: 38, SEQ ID NO: 39, SEQ ID NO: 43 to SEQ ID NO: 47, SEQ ID NO: 49, SEQ ID NO: 50, SEQ ID NO: 52, SEQ ID NO: 55 to SEQ ID NO: 57, and SEQ ID NO: 59, and
(iv) normalizing the reference expression profile of said one or more first miRNAs to the reference expression profile of said one or more second miRNAs of said one or more reference subjects thereby obtaining one or more relative reference expression levels of: SEQ ID NO: 1/SEQ ID NO: 45, SEQ ID NO: 1/SEQ ID NO: 59, SEQ ID NO: 1/SEQ ID NO: 56, SEQ ID NO: 2/SEQ ID NO: 27, SEQ ID NO: 2/SEQ ID NO: 35, SEQ ID NO: 3/SEQ ID NO: 27, SEQ ID NO: 4/SEQ ID NO: 55, SEQ ID NO: 4/SEQ ID NO: 47, SEQ ID NO: 4/SEQ ID NO: 29^{∗}, SEQ ID NO: 4/SEQ ID NO: 56, SEQ ID NO: 4/SEQ ID NO: 59, SEQ ID NO: 4/SEQ ID NO: 43, SEQ ID NO: 5/SEQ ID NO: 59, SEQ ID NO: 5/SEQ ID NO: 29, SEQ ID NO: 5/SEQ ID NO: 55, SEQ ID NO: 5/SEQ ID NO: 45, SEQ ID NO: 5/SEQ ID NO: 44, SEQ ID NO: 5/SEQ ID NO: 57, SEQ ID NO: 5/SEQ ID NO: 46, SEQ ID NO: 5/SEQ ID NO: 50, SEQ ID NO: 5/SEQ ID NO: 49, SEQ ID NO: 5/SEQ ID NO: 56, SEQ ID NO: 5/SEQ ID NO: 47, SEQ ID NO: 6/SEQ ID NO: 35, SEQ ID NO: 6/SEQ ID NO: 27, SEQ ID NO: 6/SEQ ID NO: 38, SEQ ID NO: 6/SEQ ID NO: 29, SEQ ID NO: 6/SEQ ID NO: 43, SEQ ID NO: 6/SEQ ID NO: 49, SEQ ID NO: 6/SEQ ID NO: 50, SEQ ID NO: 6/SEQ ID NO: 52, SEQ ID NO: 6/SEQ ID NO: 39, SEQ ID NO: 7/SEQ ID NO: 27, SEQ ID NO: 7/SEQ ID NO: 35, SEQ ID NO: 7/SEQ ID NO: 29, SEQ ID NO: 7/SEQ ID NO: 43, SEQ ID NO: 7/SEQ ID NO: 50, SEQ ID NO: 7/SEQ ID NO: 39, SEQ ID NO: 7/SEQ ID NO: 38, SEQ ID NO: 7/SEQ ID NO: 47, SEQ ID NO: 8/SEQ ID NO: 35, SEQ ID NO: 8/SEQ ID NO: 27, SEQ ID NO: 8/SEQ ID NO: 38, SEQ ID NO: 8/SEQ ID NO: 39, SEQ ID NO: 8/SEQ ID NO: 43, SEQ ID NO: 9/SEQ ID NO: 35, SEQ ID NO: 9/SEQ ID NO: 27, SEQ ID NO: 9/SEQ ID NO: 38, SEQ ID NO: 9/SEQ ID NO: 43, SEQ ID NO: 9/SEQ ID NO: 29, SEQ ID NO: 9/SEQ ID NO: 39, SEQ ID NO: 10/SEQ ID NO: 35, SEQ ID NO: 10/SEQ ID NO: 27, SEQ ID NO: 10/SEQ ID NO: 38, SEQ ID NO: 10/SEQ ID NO: 49, SEQ ID NO: 10/SEQ ID NO: 39, SEQ ID NO: 10/SEQ ID NO: 43, SEQ ID NO: 11/SEQ ID NO: 27, SEQ ID NO: 11/SEQ ID NO: 35, SEQ ID NO: 11/SEQ ID NO: 39, SEQ ID NO: 11/SEQ ID NO: 38, SEQ ID NO: 12/SEQ ID NO: 35, SEQ ID NO: 13/SEQ ID NO: 35, SEQ ID NO: 13/SEQ ID NO: 27, SEQ ID NO: 13/SEQ ID NO: 39, SEQ ID NO: 14/SEQ ID NO: 35, SEQ ID NO: 15/SEQ ID NO: 35, SEQ ID NO: 15/SEQ ID NO: 38, SEQ ID NO: 15/SEQ ID NO: 27, SEQ ID NO: 15/SEQ ID NO: 39, SEQ ID NO: 16/SEQ ID NO: 35, SEQ ID NO: 20/SEQ ID NO: 29, SEQ ID NO: 20/SEQ ID NO: 59, SEQ ID NO: 20/SEQ ID NO: 57, SEQ ID NO: 20/SEQ ID NO: 38, SEQ ID NO: 20/SEQ ID NO: 39, SEQ ID NO: 20/SEQ ID NO: 47, SEQ ID NO: 21/SEQ ID NO: 50, SEQ ID NO: 21/SEQ ID NO: 59, SEQ ID NO: 21/SEQ ID NO: 29^{∗}, SEQ ID NO: 21/SEQ ID NO: 57, SEQ ID NO: 21/SEQ ID NO: 55, SEQ ID NO: 21/SEQ ID NO: 49, SEQ ID NO: 21/SEQ ID NO: 56, SEQ ID NO: 22/SEQ ID NO: 59, SEQ ID NO: 22/SEQ ID NO: 47, SEQ ID NO: 22/SEQ ID NO: 56, SEQ ID NO: 22/SEQ ID NO: 57, SEQ ID NO: 22/SEQ ID NO: 29^{∗}, SEQ ID NO: 23/SEQ ID NO: 59, SEQ ID NO: 23/SEQ ID NO: 56, SEQ ID NO: 23/SEQ ID NO: 57, SEQ ID NO: 24/SEQ ID NO: 27, SEQ ID NO: 24/SEQ ID NO: 35, SEQ ID NO: 24/SEQ ID NO: 38, SEQ ID NO: 24/SEQ ID NO: 39, SEQ ID NO: 24/SEQ ID NO: 43, SEQ ID NO: 25/SEQ ID NO: 35, SEQ ID NO: 25/SEQ ID NO: 39, SEQ ID NO: 25/SEQ ID NO: 43, SEQ ID NO: 25/SEQ ID NO: 38, SEQ ID NO: 25/SEQ ID NO: 27, SEQ ID NO: 26/SEQ ID NO: 35, or SEQ ID NO: 26/SEQ ID NO: 27.

Examples of said relative reference expression levels are the ratio of the miRNA expression levels of hsa-miR-423-5p (SEQ ID NO:1) divided by hsa-miR-92a (SEQ ID NO:27), or hsa-miR-423-5p (SEQ ID NO:1) divided by hsa-miR-16 (SEQ ID NO:28), or hsa-miR-423-5p (SEQ ID NO:1) divided by hsa-miR-19a (SEQ ID NO:29), or hsa-miR-423-5p (SEQ ID NO:1) divided by hsa-miR-140-3p (SEQ ID NO:35), or hsa-miR-423-5p (SEQ ID NO:1) divided by hsa-miR-425 (SEQ ID NO:36), or hsa-miR-423-5p (SEQ ID NO:1) divided by hsa-miR-30b (SEQ ID NO:37), or hsa-miR-423-5p (SEQ ID NO:1) divided by hsa-miR-22 (SEQ ID NO:38), or hsa-miR-423-5p (SEQ ID NO:1) divided by hsa-miR-25 (SEQ ID NO:39), or hsa-miR-26b (SEQ ID NO:2) divided by hsa-miR-92a (SEQ ID NO:27), or hsa-miR-26b (SEQ ID NO:2) divided by hsa-miR-16 (SEQ ID NO:28), or hsa-miR-26b (SEQ ID NO:2) divided by hsa-miR-19a (SEQ ID NO:29), or hsa-miR-26b (SEQ ID NO:2) divided by hsa-miR-140-3p (SEQ ID NO:35), or hsa-miR-26b (SEQ ID NO:2) divided by hsa-miR-425 (SEQ ID NO:36), or hsa-miR-26b (SEQ ID NO:2) divided by hsa-miR-30b (SEQ ID NO:37), or hsa-miR-26b (SEQ ID NO:2) divided by hsa-miR-22 (SEQ ID NO:38), or hsa-miR-26b (SEQ ID NO:2) divided by hsa-miR-25 (SEQ ID NO:39), or hsa-miR-374a (SEQ ID NO:3) divided by hsa-miR-92a (SEQ ID NO:27), or hsa-miR-374a (SEQ ID NO:3) divided by hsa-miR-16 (SEQ ID NO:28), or hsa-miR-374a (SEQ ID NO:3) divided by hsa-miR-19a (SEQ ID NO:29), or hsa-miR-374a (SEQ ID NO:3) divided by hsa-miR-140-3p (SEQ ID NO:35), or hsa-miR-374a (SEQ ID NO:3) divided by hsa-miR-425 (SEQ ID NO:36), or hsa-miR-374a (SEQ ID NO:3) divided by hsa-miR-30b (SEQ ID NO:37), or hsa-miR-374a (SEQ ID NO:3) divided by hsa-miR-22 (SEQ ID NO:38), or hsa-miR-374a (SEQ ID NO:3) divided by hsa-miR-25 (SEQ ID NO:39), or hsa-miR-720 (SEQ ID NO:5) divided by hsa-miR-92a (SEQ ID NO:27), or hsa-miR-720 (SEQ ID NO:5) divided by hsa-miR-16 (SEQ ID NO:28), or hsa-miR-720 (SEQ ID NO:5) divided by hsa-miR-19a (SEQ ID NO:29), or hsa-miR-720 (SEQ ID NO:5) divided by hsa-miR-140-3p (SEQ ID NO:35), or hsa-miR-720 (SEQ ID NO:5) divided by hsa-miR-425 (SEQ ID NO:36), or hsa-miR-720 (SEQ ID NO:5) divided by hsa-miR-30b (SEQ ID NO:37), or hsa-miR-720 (SEQ ID NO:5) divided by hsa-miR-22 (SEQ ID NO:38), or hsa-miR-720 (SEQ ID NO:5) divided by hsa-miR-25 (SEQ ID NO:39), or hsa-miR-20b (SEQ ID NO:6) divided by hsa-miR-92a (SEQ ID NO:27), or hsa-miR-20b (SEQ ID NO:6) divided by hsa-miR-16 (SEQ ID NO:28), or hsa-miR-20b (SEQ ID NO:6) divided by hsa-miR-19a (SEQ ID NO:29), or hsa-miR-20b (SEQ ID NO:6) divided by hsa-miR-140-3p (SEQ ID NO:35), or hsa-miR-20b (SEQ ID NO:6) divided by hsa-miR-425 (SEQ ID NO:36), or hsa-miR-20b (SEQ ID NO:6) divided by hsa-miR-30b (SEQ ID NO:37), or hsa-miR-20b (SEQ ID NO:6) divided by hsa-miR-22 (SEQ ID NO:38), or hsa-miR-20b (SEQ ID NO:6) divided by hsa-miR-25 (SEQ ID NO:39), or hsa-miR-144^{∗} (SEQ ID NO:7) divided by hsa-miR-92a (SEQ ID NO:27), or hsa-miR-144^{∗} (SEQ ID NO:7) divided by hsa-miR-16 (SEQ ID NO:28), or hsa-miR-144^{∗} (SEQ ID NO:7) divided by hsa-miR-19a (SEQ ID NO:29), or hsa-miR-144^{∗} (SEQ ID NO:7) divided by hsa-miR-140-3p (SEQ ID NO:35), or hsa-miR-144^{∗} (SEQ ID NO:7) divided by hsa-miR-425 (SEQ ID NO:36), or hsa-miR-144^{∗} (SEQ ID NO:7) divided by hsa-miR-30b (SEQ ID NO:37), or hsa-miR-144^{∗} (SEQ ID NO:7) divided by hsa-miR-22 (SEQ ID NO:38), or hsa-miR-144^{∗} (SEQ ID NO:7) divided by hsa-miR-25 (SEQ ID NO:39), or hsa-miR-17 (SEQ ID NO:8) divided by hsa-miR-92a (SEQ ID NO:27), or hsa-miR-17 (SEQ ID NO:8) divided by hsa-miR-16 (SEQ ID NO:28), or hsa-miR-17 (SEQ ID NO:8) divided by hsa-miR-19a (SEQ ID NO:29), or hsa-miR-17 (SEQ ID NO:8) divided by hsa-miR-140-3p (SEQ ID NO:35), or hsa-miR-17 (SEQ ID NO:8) divided by hsa-miR-425 (SEQ ID NO:36), or hsa-miR-17 (SEQ ID NO:8) divided by hsa-miR-30b (SEQ ID NO:37), or hsa-miR-17 (SEQ ID NO:8) divided by hsa-miR-22 (SEQ ID NO:38), or hsa-miR-17 (SEQ ID NO:8) divided by hsa-miR-25 (SEQ ID NO:39), or hsa-miR-126 (SEQ ID NO:9) divided by hsa-miR-92a (SEQ ID NO:27), or hsa-miR-126 (SEQ ID NO:9) divided by hsa-miR-16 (SEQ ID NO:28), or hsa-miR-126 (SEQ ID NO:9) divided by hsa-miR-19a (SEQ ID NO:29), or hsa-miR-126 (SEQ ID NO:9) divided by hsa-miR-140-3p (SEQ ID NO:35), or hsa-miR-126 (SEQ ID NO:9) divided by hsa-miR-425 (SEQ ID NO:36), or hsa-miR-126 (SEQ ID NO:9) divided by hsa-miR-30b (SEQ ID NO:37), or hsa-miR-126 (SEQ ID NO:9) divided by hsa-miR-22 (SEQ ID NO:38), or hsa-miR-126 (SEQ ID NO:9) divided by hsa-miR-25 (SEQ ID NO:39), or hsa-miR-20a (SEQ ID NO:10) divided by hsa-miR-92a (SEQ ID NO:27), or hsa-miR-20a (SEQ ID NO:10) divided by hsa-miR-16 (SEQ ID NO:28), or hsa-miR-20a (SEQ ID NO:10) divided by hsa-miR-19a (SEQ ID NO:29), or hsa-miR-20a (SEQ ID NO:10) divided by hsa-miR-140-3p (SEQ ID NO:35), or hsa-miR-20a (SEQ ID NO:10) divided by hsa-miR-425 (SEQ ID NO:36), or hsa-miR-20a (SEQ ID NO:10) divided by hsa-miR-30b (SEQ ID NO:37), or hsa-miR-20a (SEQ ID NO:10) divided by hsa-miR-22 (SEQ ID NO:38), or hsa-miR-20a (SEQ ID NO:10) divided by hsa-miR-25 (SEQ ID NO:39), or hsa-miR-374b (SEQ ID NO:11) divided by hsa-miR-92a (SEQ ID NO:27), or hsa-miR-374b (SEQ ID NO:11) divided by hsa-miR-16 (SEQ ID NO:28), or hsa-miR-374b (SEQ ID NO:11) divided by hsa-miR-19a (SEQ ID NO:29), or hsa-miR-374b (SEQ ID NO:11) divided by hsa-miR-140-3p (SEQ ID NO:35), or hsa-miR-374b (SEQ ID NO:11) divided by hsa-miR-425 (SEQ ID NO:36), or hsa-miR-374b (SEQ ID NO:11) divided by hsa-miR-30b (SEQ ID NO:37), or hsa-miR-374b (SEQ ID NO:11) divided by hsa-miR-22 (SEQ ID NO:38), or hsa-miR-374b (SEQ ID NO:11) divided by hsa-miR-25 (SEQ ID NO:39) or hsa-miR-144^{∗} (SEQ ID NO:7) divided by hsa-miR-16 (SEQ ID NO: 28), or hsa-miR-144^{∗} (SEQ ID NO:7) divided by hsa-miR-92a (SEQ ID NO: 27), or hsa-miR-126 (SEQ ID NO:9) divided by hsa-miR-16 (SEQ ID NO: 28), or hsa-miR-20b (SEQ ID NO:6) divided by hsa-miR-16 (SEQ ID NO: 28), or hsa-miR-20b (SEQ ID NO:6) divided by hsa-miR-140-3p (SEQ ID NO: 35), or hsa-miR-20b (SEQ ID NO:6) divided by hsa-miR-92a (SEQ ID NO: 27), or hsa-miR-363 (SEQ ID NO:24) divided by hsa-miR-16 (SEQ ID NO: 28), or hsa-miR-20b (SEQ ID NO:6) divided by hsa-miR-22 (SEQ ID NO: 38), or hsa-miR-363 (SEQ ID NO:24) divided by hsa-miR-192 (SEQ ID NO: 41), or hsa-miR-20b (SEQ ID NO:6) divided by hsa-miR-192 (SEQ ID NO: 41), or hsa-miR-720 (SEQ ID NO:5) divided by hsa-miR-874 (SEQ ID NO: 59), or hsa-miR-17 (SEQ ID NO:8) divided by hsa-miR-16 (SEQ ID NO: 28), or hsa-miR-20a (SEQ ID NO:10) divided by hsa-miR-140-3p (SEQ ID NO: 35), or hsa-miR-20a (SEQ ID NO:10) divided by hsa-miR-16 (SEQ ID NO: 28), or hsa-miR-144^{∗} (SEQ ID NO:7) divided by hsa-miR-30b (SEQ ID NO: 37), or hsa-miR-106a (SEQ ID NO:13) divided by hsa-miR-16 (SEQ ID NO: 28), or hsa-miR-17 (SEQ ID NO:8) divided by hsa-miR-140-3p (SEQ ID NO: 35), or hsa-miR-144^{∗} (SEQ ID NO:7) divided by hsa-miR-140-3p (SEQ ID NO: 35), or hsa-miR-106a (SEQ ID NO:13) divided by hsa-miR-140-3p (SEQ ID NO: 35), or hsa-miR-93^{∗} (SEQ ID NO:21) divided by hsa-miR-27b (SEQ ID NO: 34), or hsa-miR-144^{∗} (SEQ ID NO:7) divided by hsa-miR-19a (SEQ ID NO: 29), or hsa-miR-222 (SEQ ID NO: 15) divided by hsa-miR-140-3p (SEQ ID NO: 35), or hsa-miR-20b (SEQ ID NO:6) divided by hsa-miR-19a (SEQ ID NO: 29), or hsa-miR-363 (SEQ ID NO:24) divided by hsa-miR-22 (SEQ ID NO: 38), or hsa-miR-363 (SEQ ID NO:24) divided by hsa-miR-140-3p (SEQ ID NO: 35), or hsa-miR-93^{∗} (SEQ ID NO:21) divided by hsa-miR-99b (SEQ ID NO: 54), or hsa-miR-720 (SEQ ID NO:5) divided by hsa-miR-19a (SEQ ID NO: 29), or hsa-miR-720 (SEQ ID NO:5) divided by hsa-miR-361-3p (SEQ ID NO: 55) from one or more reference subjects.

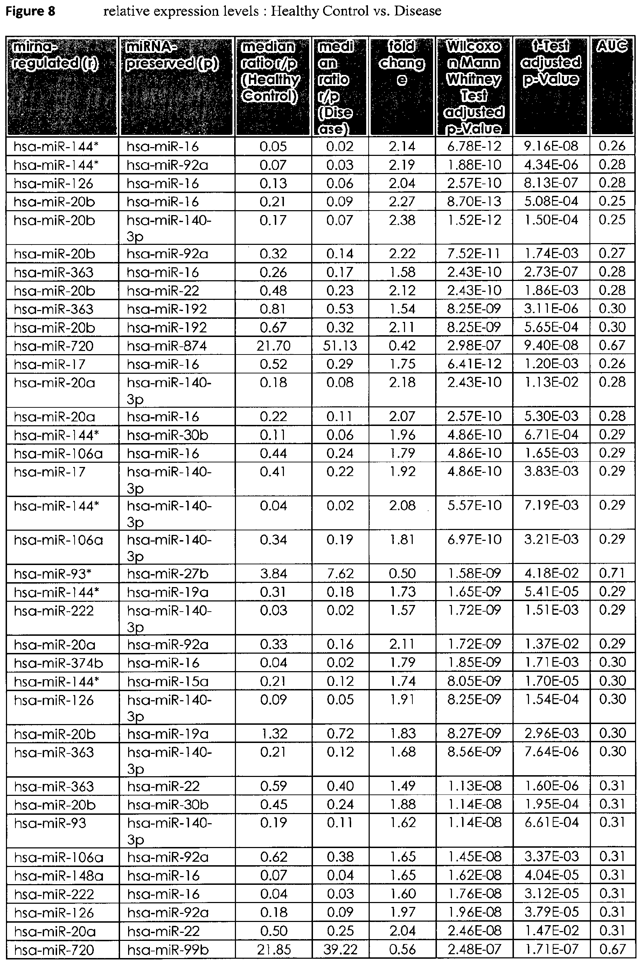

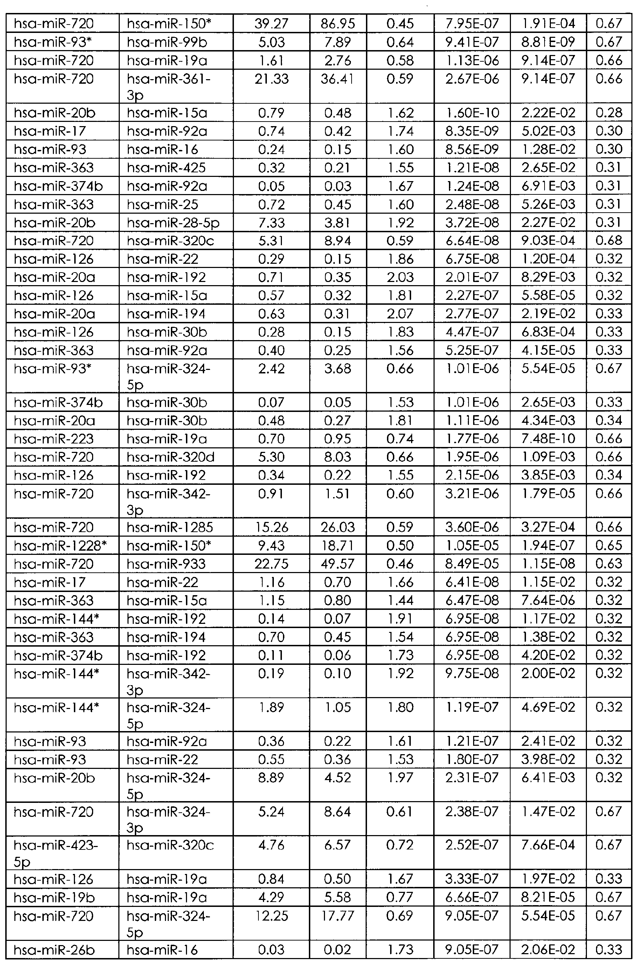

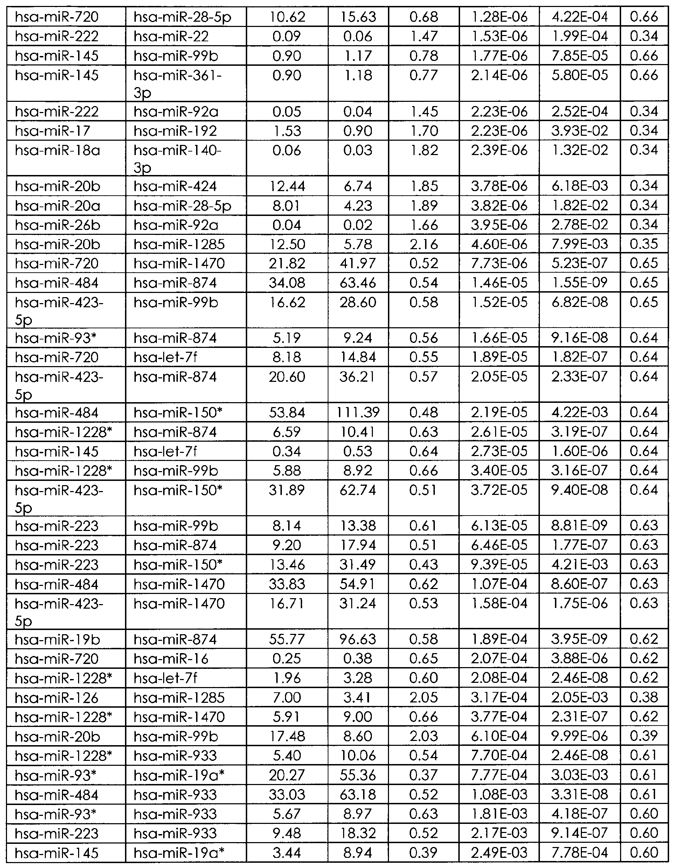

It is preferred that the relative expression levels are obtained by calculating a ratio from the one or more first miRNAs having a nucleotide sequence selected from the group consisting of SEQ ID NO: 1 to SEQ ID NO: 16 and SEQ ID NO: 20 to SEQ ID NO: 26 and the one or more second miRNAs having a nucleotide sequence selected from the group consisting of SEQ ID NO: 27, SEQ ID NO: 29, SEQ ID NO: 35, SEQ ID NO: 38, SEQ ID NO: 39, SEQ ID NO: 43 to SEQ ID NO: 47, SEQ ID NO: 49, SEQ ID NO: 50, SEQ ID NO: 52, SEQ ID NO: 55 to SEQ ID NO: 57, and SEQ ID NO: 59, wherein the relative expression levels are preferably selected from the group of ratios listed in Figure 8.

It is further preferred that in step (f) a change of the health status is assessed when the comparison of the relative expression levels from said subject to the relative reference expression levels exceeds a threshold.

Preferably, the threshold for assessing a change of the health status is set at 3 standard deviations, preferably is set at 5 standard deviations, more preferably is set at 7 standard deviations.

In a second aspect, the present invention relates to a method for assessing the health status in a subject, comprising the steps of:
(a) isolating total intracellular RNA from erythrocytes, leukocytes, and thrombocytes which have been obtained from a whole blood sample which has been taken from a subject,
(b) determining in said total intracellular RNA an expression profile of at least one first miRNA,
(c) determining in said total intracellular RNA an expression profile of at least one second miRNA,
(d) comparing the expression profile of the at least one first miRNA of step (b) to a reference expression profile of said at least one first miRNA and the expression profile of the at least one second miRNA of step (c) to a reference expression profile of said at least one second miRNA, and
(e) assessing a change of the health status in said subject when the comparison of the expression profile of the at least one first miRNA to the reference expression profile of said at least one first miRNA is altered and when the comparison of the expression profile of the at least one second miRNA to the reference expression profile of said at least one second miRNA is not altered,
wherein
the at least one first miRNA has a nucleotide sequence according to SEQ ID NO: 1 and the at least one second miRNA has a nucleotide sequence according to SEQ ID NO: 27,
the at least one first miRNA has a nucleotide sequence according to SEQ ID NO: 1 and the at least one second miRNA has a nucleotide sequence according to SEQ ID NO: 29,
the at least one first miRNA has a nucleotide sequence according to SEQ ID NO: 1 and the at least one second miRNA has a nucleotide sequence according to SEQ ID NO: 35,
the at least one first miRNA has a nucleotide sequence according to SEQ ID NO: 2 and the at least one second miRNA has a nucleotide sequence according to SEQ ID NO: 27,
the at least one first miRNA has a nucleotide sequence according to SEQ ID NO: 2 and the at least one second miRNA has a nucleotide sequence according to SEQ ID NO: 29,
the at least one first miRNA has a nucleotide sequence according to SEQ ID NO: 3 and the at least one second miRNA has a nucleotide sequence according to SEQ ID NO: 27,
the at least one first miRNA has a nucleotide sequence according to SEQ ID NO: 3 and the at least one second miRNA has a nucleotide sequence according to SEQ ID NO: 29,
the at least one first miRNA has a nucleotide sequence according to SEQ ID NO: 4 and the at least one second miRNA has a nucleotide sequence according to SEQ ID NO: 27,
the at least one first miRNA has a nucleotide sequence according to SEQ ID NO: 4 and the at least one second miRNA has a nucleotide sequence according to SEQ ID NO: 29,
the at least one first miRNA has a nucleotide sequence according to SEQ ID NO: 5 and the at least one second miRNA has a nucleotide sequence according to SEQ ID NO: 27,
the at least one first miRNA has a nucleotide sequence according to SEQ ID NO: 5 and the at least one second miRNA has a nucleotide sequence according to SEQ ID NO: 29,
the at least one first miRNA has a nucleotide sequence according to SEQ ID NO: 5 and the at least one second miRNA has a nucleotide sequence according to SEQ ID NO: 55,
the at least one first miRNA has a nucleotide sequence according to SEQ ID NO: 5 and the at least one second miRNA has a nucleotide sequence according to SEQ ID NO: 59,
the at least one first miRNA has a nucleotide sequence according to SEQ ID NO: 6 and the at least one second miRNA has a nucleotide sequence according to SEQ ID NO: 27,
the at least one first miRNA has a nucleotide sequence according to SEQ ID NO: 6 and the at least one second miRNA has a nucleotide sequence according to SEQ ID NO: 29,
the at least one first miRNA has a nucleotide sequence according to SEQ ID NO: 6 and the at least one second miRNA has a nucleotide sequence according to SEQ ID NO: 35,
the at least one first miRNA has a nucleotide sequence according to SEQ ID NO: 6 and the at least one second miRNA has a nucleotide sequence according to SEQ ID NO: 38,
the at least one first miRNA has a nucleotide sequence according to SEQ ID NO: 7 and the at least one second miRNA has a nucleotide sequence according to SEQ ID NO: 27,
the at least one first miRNA has a nucleotide sequence according to SEQ ID NO: 7 and the at least one second miRNA has a nucleotide sequence according to SEQ ID NO: 29,
the at least one first miRNA has a nucleotide sequence according to SEQ ID NO: 7 and the at least one second miRNA has a nucleotide sequence according to SEQ ID NO: 35,
the at least one first miRNA has a nucleotide sequence according to SEQ ID NO: 8 and the at least one second miRNA has a nucleotide sequence according to SEQ ID NO: 27,
the at least one first miRNA has a nucleotide sequence according to SEQ ID NO: 8 and the at least one second miRNA has a nucleotide sequence according to SEQ ID NO: 29,
the at least one first miRNA has a nucleotide sequence according to SEQ ID NO: 8 and the at least one second miRNA has a nucleotide sequence according to SEQ ID NO: 35,
the at least one first miRNA has a nucleotide sequence according to SEQ ID NO: 9 and the at least one second miRNA has a nucleotide sequence according to SEQ ID NO: 27,
the at least one first miRNA has a nucleotide sequence according to SEQ ID NO: 10 and the at least one second miRNA has a nucleotide sequence according to SEQ ID NO: 35,
the at least one first miRNA has a nucleotide sequence according to SEQ ID NO: 13 and the at least one second miRNA has a nucleotide sequence according to SEQ ID NO: 35,
the at least one first miRNA has a nucleotide sequence according to SEQ ID NO: 15 and the at least one second miRNA has a nucleotide sequence according to SEQ ID NO: 35,
the at least one first miRNA has a nucleotide sequence according to SEQ ID NO: 24 and the at least one second miRNA has a nucleotide sequence according to SEQ ID NO: 35, or
the at least one first miRNA has a nucleotide sequence according to SEQ ID NO: 24 and the at least one second miRNA has a nucleotide sequence according to SEQ ID NO: 38.

It is preferred, that the one or more relative reference expression levels are obtained from an earlier time point from the same subject.

Preferably, the change of the health status according to the first and the second aspect of the invention is an improvement or a worsening of the health status.

Exemplarily, in the methods described herein, the nucleic sequences of the at least two miRNAs, namely one disease-regulated miRNAs (of a first predetermined set of one or more miRNAs selected from the miRNAs listed in Figure 1 with SEQ ID NO: 1 to 26) and one disease-preserved miRNAs (of a second predetermined set of one or more miRNAs selected from the miRNAs listed in Figure 2 with SEQ ID NO: 27 to 59) when determining an expression profile in a blood cell sample derived from a whole blood sample have SEQ ID NO: 1 and SEQ ID NO: 27, the nucleic sequences of the at least two miRNAs have SEQ ID NO: 1 and SEQ ID NO: 28, the nucleic sequences of the at least two miRNAs have SEQ ID NO: 1 and SEQ ID NO: 29, the nucleic sequences of the at least two miRNAs have SEQ ID NO: 1 and SEQ ID NO: 30, the nucleic sequences of the at least two miRNAs have SEQ ID NO: 1 and SEQ ID NO: 31, the nucleic sequences of the at least two miRNAs have SEQ ID NO: 1 and SEQ ID NO: 32, the nucleic sequences of the at least two miRNAs have SEQ ID NO: 1 and SEQ ID NO: 33, the nucleic sequences of the at least two miRNAs have SEQ ID NO: 1 and SEQ ID NO: 34, the nucleic sequences of the at least two miRNAs have SEQ ID NO: 1 and SEQ ID NO: 35, the nucleic sequences of the at least two miRNAs have SEQ ID NO: 2 and SEQ ID NO: 27, the nucleic sequences of the at least two miRNAs have SEQ ID NO: 2 and SEQ ID NO: 28, the nucleic sequences of the at least two miRNAs have SEQ ID NO: 2 and SEQ ID NO: 29, the nucleic sequences of the at least two miRNAs have SEQ ID NO: 2 and SEQ ID NO: 30, the nucleic sequences of the at least two miRNAs have SEQ ID NO: 2 and SEQ ID NO: 31, the nucleic sequences of the at least two miRNAs have SEQ ID NO: 2 and SEQ ID NO: 32, the nucleic sequences of the at least two miRNAs have SEQ ID NO: 2 and SEQ ID NO: 33, the nucleic sequences of the at least two miRNAs have SEQ ID NO: 2 and SEQ ID NO: 34, the nucleic sequences of the at least two miRNAs have SEQ ID NO: 3 and SEQ ID NO: 27, the nucleic sequences of the at least two miRNAs have SEQ ID NO: 3 and SEQ ID NO: 28, the nucleic sequences of the at least two miRNAs have SEQ ID NO: 3 and SEQ ID NO: 29, the nucleic sequences of the at least two miRNAs have SEQ ID NO: 3 and SEQ ID NO: 30, the nucleic sequences of the at least two miRNAs have SEQ ID NO: 3 and SEQ ID NO: 31, the nucleic sequences of the at least two miRNAs have SEQ ID NO: 3 and SEQ ID NO: 32, the nucleic sequences of the at least two miRNAs have SEQ ID NO: 3 and SEQ ID NO: 33, the nucleic sequences of the at least two miRNAs have SEQ ID NO: 4 and SEQ ID NO: 27, the nucleic sequences of the at least two miRNAs have SEQ ID NO: 4 and SEQ ID NO: 28, the nucleic sequences of the at least two miRNAs have SEQ ID NO: 4 and SEQ ID NO: 29, the nucleic sequences of the at least two miRNAs have SEQ ID NO: 4 and SEQ ID NO: 30, the nucleic sequences of the at least two miRNAs have SEQ ID NO: 4 and SEQ ID NO: 31, the nucleic sequences of the at least two miRNAs have SEQ ID NO: 4 and SEQ ID NO: 32, the nucleic sequences of the at least two miRNAs have SEQ ID NO: 5 and SEQ ID NO: 27, the nucleic sequences of the at least two miRNAs have SEQ ID NO: 5 and SEQ ID NO: 28, the nucleic sequences of the at least two miRNAs have SEQ ID NO: 5 and SEQ ID NO: 29, the nucleic sequences of the at least two miRNAs have SEQ ID NO: 5 and SEQ ID NO: 30, the nucleic sequences of the at least two miRNAs have SEQ ID NO: 5 and SEQ ID NO: 31, the nucleic sequences of the at least two miRNAs have SEQ ID NO: 6 and SEQ ID NO: 27, the nucleic sequences of the at least two miRNAs have SEQ ID NO: 6 and SEQ ID NO: 28, the nucleic sequences of the at least two miRNAs have SEQ ID NO: 6 and SEQ ID NO: 29, the nucleic sequences of the at least two miRNAs have SEQ ID NO: 6 and SEQ ID NO: 30, the nucleic sequences of the at least two miRNAs have SEQ ID NO: 7 and SEQ ID NO: 27, the nucleic sequences of the at least two miRNAs have SEQ ID NO: 7 and SEQ ID NO: 28, the nucleic sequences of the at least two miRNAs have SEQ ID NO: 7 and SEQ ID NO: 29, the nucleic sequences of the at least two miRNAs have SEQ ID NO: 8 and SEQ ID NO: 27, the nucleic sequences of the at least two miRNAs have SEQ ID NO: 8 and SEQ ID NO: 29, or the nucleic sequences of the at least two miRNAs have SEQ ID NO: 9 and SEQ ID NO: 27, or the nucleic sequences of the at least two miRNAs have SEQ ID NO: 7 and SEQ ID NO: 28 , or the nucleic sequences of the at least two miRNAs have SEQ ID NO: 7 and SEQ ID NO: 27 , or the nucleic sequences of the at least two miRNAs have SEQ ID NO: 9 and SEQ ID NO: 28 , or the nucleic sequences of the at least two miRNAs have SEQ ID NO: 6 and SEQ ID NO: 28 , or the nucleic sequences of the at least two miRNAs have SEQ ID NO: 6 and SEQ ID NO: 35 , or the nucleic sequences of the at least two miRNAs have SEQ ID NO: 6 and SEQ ID NO: 27 , or the nucleic sequences of the at least two miRNAs have SEQ ID NO: 24 and SEQ ID NO: 28 , or the nucleic sequences of the at least two miRNAs have SEQ ID NO: 6 and SEQ ID NO: 38 , or the nucleic sequences of the at least two miRNAs have SEQ ID NO: 24 and SEQ ID NO: 41 , or the nucleic sequences of the at least two miRNAs have SEQ ID NO: 6 and SEQ ID NO: 41 , or the nucleic sequences of the at least two miRNAs have SEQ ID NO: 5 and SEQ ID NO: 59 , or the nucleic sequences of the at least two miRNAs have SEQ ID NO: 8 and SEQ ID NO: 28 , or the nucleic sequences of the at least two miRNAs have SEQ ID NO: 10 and SEQ ID NO: 35 , or the nucleic sequences of the at least two miRNAs have SEQ ID NO: 10 and SEQ ID NO: 28 , or the nucleic sequences of the at least two miRNAs have SEQ ID NO: 7 and SEQ ID NO: 37 , or the nucleic sequences of the at least two miRNAs have SEQ ID NO: 13 and SEQ ID NO: 28 , or the nucleic sequences of the at least two miRNAs have SEQ ID NO: 8 and SEQ ID NO: 35 , or the nucleic sequences of the at least two miRNAs have SEQ ID NO: 7 and SEQ ID NO: 35 , or the nucleic sequences of the at least two miRNAs have SEQ ID NO: 13 and SEQ ID NO: 35 , or the nucleic sequences of the at least two miRNAs have SEQ ID NO: 21 and SEQ ID NO: 34 , or the nucleic sequences of the at least two miRNAs have SEQ ID NO: 7 and SEQ ID NO: 29 , or the nucleic sequences of the at least two miRNAs have SEQ ID NO: 15 and SEQ ID NO: 35 , or the nucleic sequences of the at least two miRNAs have SEQ ID NO: 6 and SEQ ID NO: 29 , or the nucleic sequences of the at least two miRNAs have SEQ ID NO: 24 and SEQ ID NO: 38 , or the nucleic sequences of the at least two miRNAs have SEQ ID NO: 24 and SEQ ID NO: 35 , or the nucleic sequences of the at least two miRNAs have SEQ ID NO: 21 and SEQ ID NO: 54 , or the nucleic sequences of the at least two miRNAs have SEQ ID NO: 5 and SEQ ID NO: 29 , or the nucleic sequences of the at least two miRNAs have SEQ ID NO: 5 and SEQ ID NO: 55.

The expression profile may be determined comprising the steps of:
(a) Providing a whole blood sample of a subject to be diagnosed for the health status
(b) Deriving a blood cell sample from said whole blood sample
(c) Extracting the total RNA from said blood cell sample
(d) Determining the expression profile of first set of one or more disease-regulated miRNAs (selected from the miRNAs listed in Figure 1 with SEQ ID NO: 1 to 26) and a second set of one or more disease-preserved miRNAs (selected from the miRNAs listed in Figure 2 with SEQ ID NO: 27 to 59) from the total RNA extracted.

Preferably, the blood cell sample is derived from a whole blood sample by separating the blood cells from the remaining parts of the whole blood sample, which may be achieved by centrifugation, wherein the blood cells (including red blood cells, white blood cells and platelets) form a pellet that may be harvested (see Example 1). The person skilled in the art is aware of alternative methods to separate the blood cells from the remaining parts of the whole blood sample (e.g. size exclusion, size distribution, dielectrophoresis, positive or negative antibody selection etc.).

It is further preferred that the determining of the expression profile includes the reverse-transcription of the nucleotide sequence of the at least one miRNA, preferably at least two miRNAs comprised in the set into cDNA (complementary DNA). Herein, the RNA-sequence is reverse-transcribed into DNA (e.g. by use of reverse-transcriptase) before the expression profile of said miRNAs is determined. Preferably, the nucleotide sequence of the at least one miRNA, preferably at least two miRNAs comprised in the set is reverse-transcribed into cDNA when nucleic acid amplification (PCR, RT-PCR), sequencing (next generation sequencing, Sanger sequencing) or hybridisation based techniques are employed in the determination of the miRNA expression profile. Furthermore, it is preferred that the total RNA is transcribed into cDNA from which the expression profile is determined.

In a third aspect, the invention relates to the use of the method according to the first and the second aspect of the invention for:
(i.) comparing the health status between one or more subjects,
(ii.) monitoring the health status in a subject,
(iii.) monitoring the state of the immune system in a subject,
(iv.) monitoring the response to therapeutic treatment, preferably drug treatment, in a subject.

Also described herein, but not encompassed by the present invention, is a kit for diagnosing the health status of a subject comprising:
(i.) means for determining the expression profile of a set comprising at least one miRNAs selected from the group consisting of SEQ ID NO: 1 to 26 and at least one miRNAs selected from the group consisting of SEQ ID NO: 27 to 59 in a blood sample of a subject, and
(ii.) at least one reference

It is understood that kit includes and/or comprises the aspects detailed in the method according to the first and second aspect, the aspects detailed in the use according to the third aspect of the present invention.

Preferably, said means for determining the expression profile comprise:
(i.) a set of at least two polynucleotides for determining of a set comprising at least one miRNA selected from the group consisting of SEQ ID NO: 1 to 26 and at least one miRNA selected from the group consisting of SEQ ID NO: 27 to 59 in a blood sample of a subject, and
(ii.) a microarray, a RT-PCT system, a PCR-system, a flow cytometer, a bead-based multiplex system or a next generation sequencing system.

The kit comprises at least one reference according to the present invention as outlined in the first aspect of the present invention. In a preferred embodiment, the reference may be contained in the data carrier of the kit. In a further preferred embodiment the reference may be a reference sample and/or a reference standard that is included in the kit and which is employed when performing the kit, e.g. in the determining of the expression profile.

Optionally, the kit comprises a data carrier. Preferably the data carrier is an electronic or a non-electronic data carrier, more preferably it is an electronic data carrier, such as a storage medium. The kit optionally comprises a data carrier, which optionally comprises the reference and/or an instruction on how to apply the expression profile and the reference in the diagnosis of the health status. This instruction on how to apply the expression profile and the reference may include instructions for the doctor and/or the diagnostic laboratory that are involved in the diagnosis of the health status. It is preferred that the data carrier further comprises tools for analysis and evaluation of the determined expression profile(s). These tools may be any tools to assist the doctor and/or the diagnostic laboratory in the diagnosis of the health status. It is preferred that the instruction comprised is an algorithm or a software. Preferably, these tools are software-tools that assist in analysis of the determined expression profile(s) and/or assist in the subsequently diagnosis. The tools for analysis and evaluation may include a reference according to the present invention.

The kit optionally comprises a whole blood collection tube, which is preferably selected from group consisting of EDTA-, Na-citrate-, ACD-, Heparin-, PAXgene Blood RNA-, Tempus Blood RNA-tubes and optionally contains an additive for stabilizing the RNA-fraction.

The kit optionally comprises means for deriving the blood cell sample from a whole blood sample. These means are preferably for separating and/or isolating of the respective blood cell sample (e.g. a blood cell sample comprising white blood cells, red blood cells or platelets, a blood cell sample comprising white blood cells, red blood cells and platelets, a platelet-preparation) from the remaining parts of the whole blood sample. These means may include reagents or consumables for isolating/separating the respective blood cell fraction(s) and/or suitable instrumentation (e.g. centrifuge, special collection tubes).

In a fourth aspect, the invention relates to a method for monitoring the health status in a subject, comprising the steps of:
(a) assessing the health status in a subject at a first point in time by carrying out the method according to the first or second aspect,
(b) assessing the health status in the subject at one or more later points in time by carrying out the method according to the first or second aspect, and
(c) comparing the health status diagnosed in step (a) with the health status diagnosed in step (b), thereby monitoring the health status in the subject.

In one embodiment, the health status in the subject improves or worsens over time.

In one other embodiment, the health status in the subject which is monitored encompasses the state of the immune system of said subject.

In one other embodiment, the subject has received a therapeutic treatment between the first point in time and the one or more later points in time. Said treatment may be a drug treatment, i.e. it may comprise the administration of a drug. Said drug may be a drug to treat a disease and/or cancer in the subject. Said treatment may alternatively and/or additionally comprise surgery, chemotherapy, radiation therapy, and/or immunotherapy.

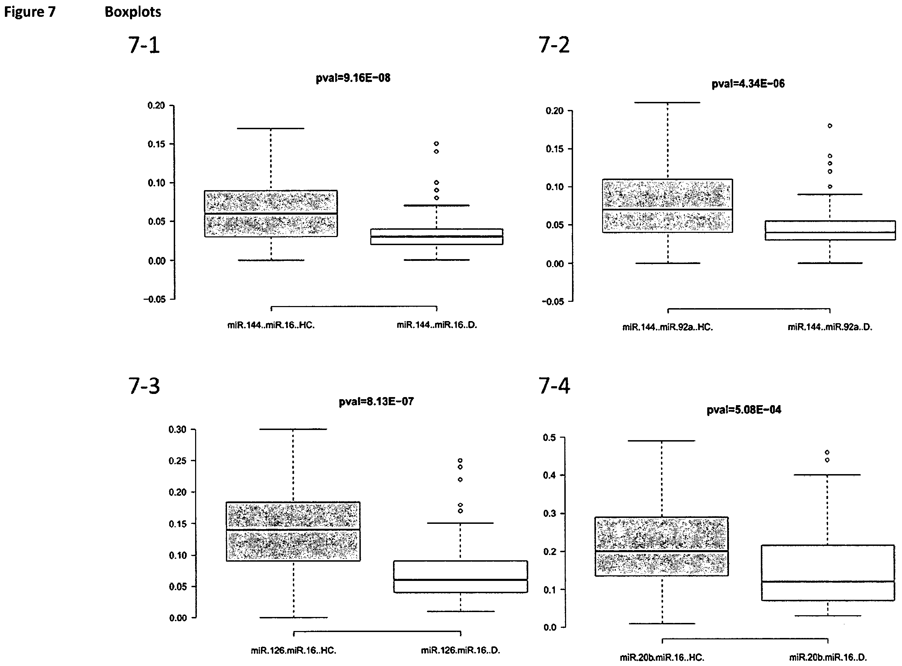

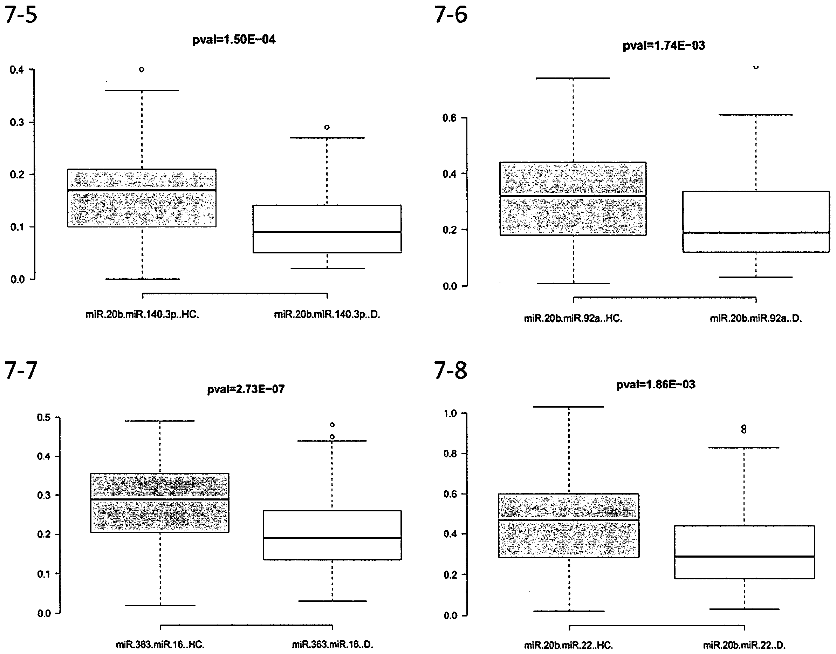

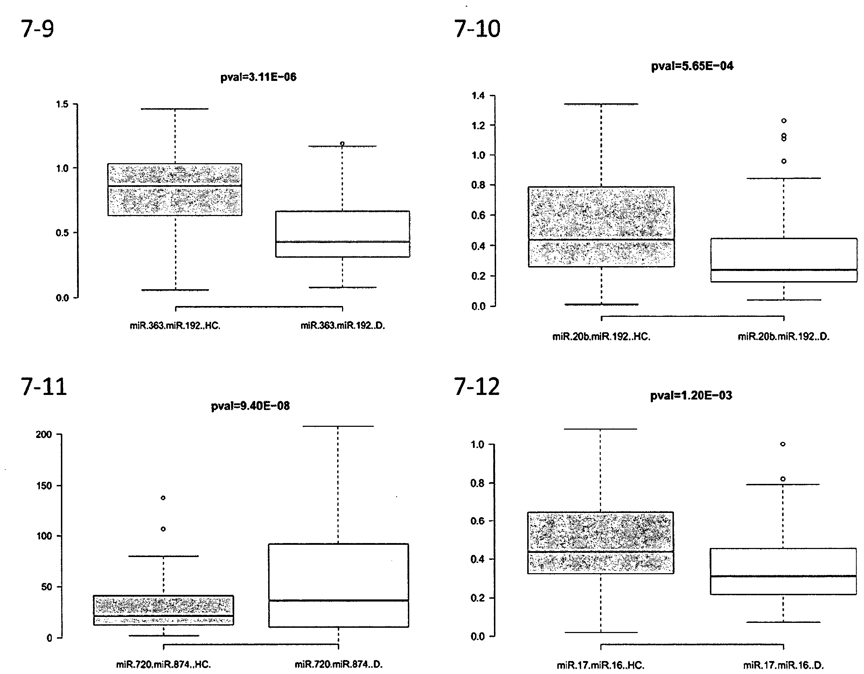

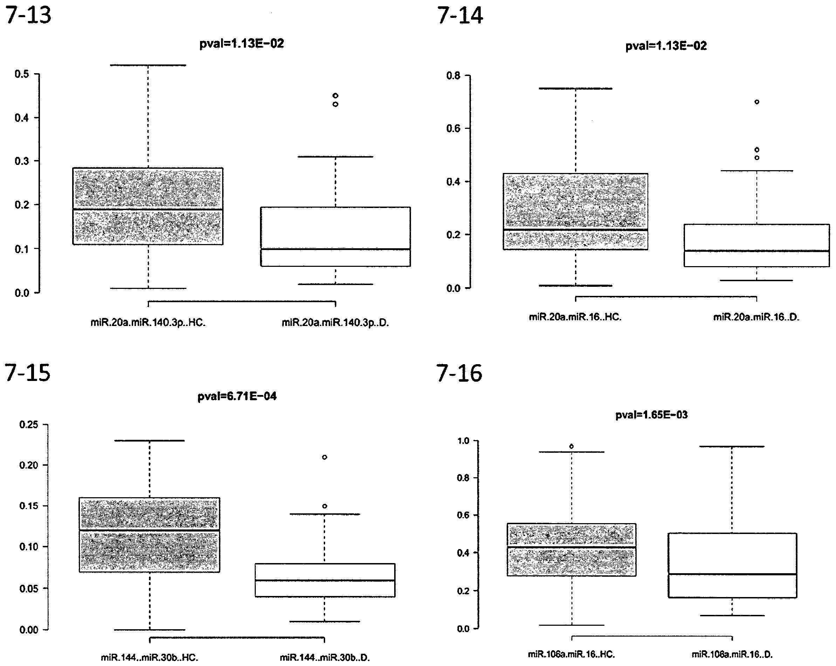

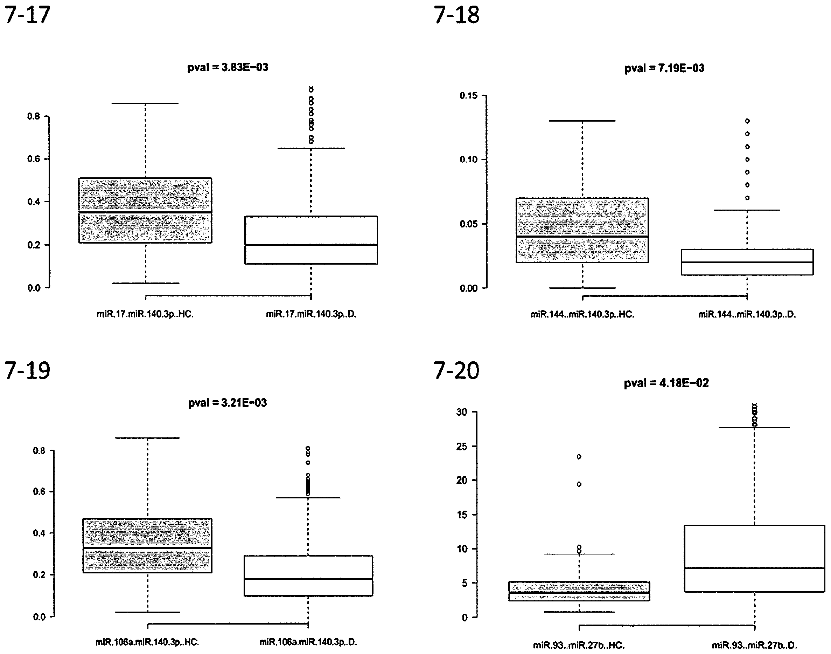

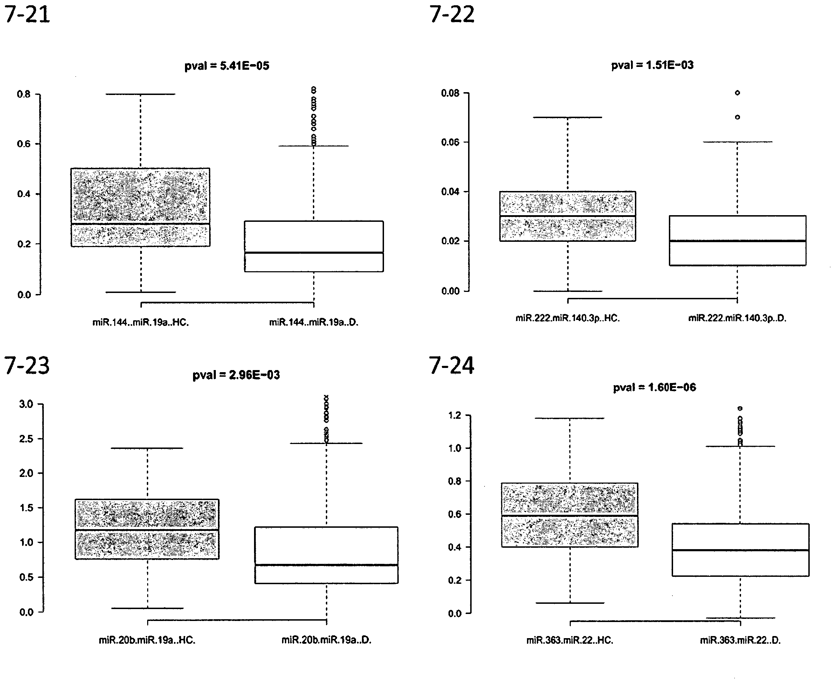

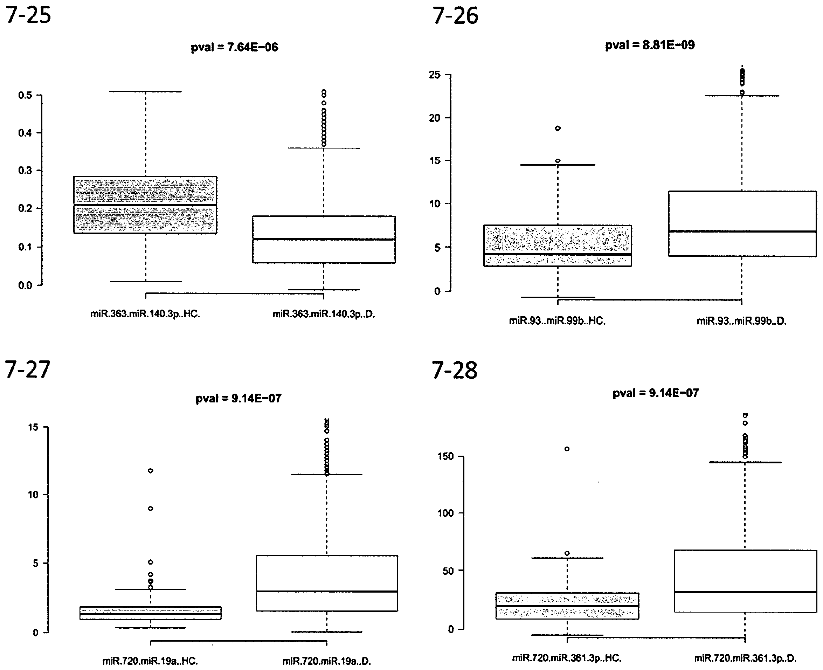

In summary, the following items are described:
1. A method for diagnosing the health status in a subject, comprising the steps of:
   (a) Determining the expression profile of a first predetermined set of one or more miRNAs in a blood sample from a subject, wherein the miRNAs comprised in said first predetermined set of miRNAs are selected from miRNAs listed in Figure 1 and/or Figure 3
   (b) Determining the expression profile of a second predetermined set of one or more miRNAs in said blood sample from said subject, wherein the miRNAs comprised in said second predetermined set of miRNAs are selected from miRNAs listed in Figure 2 and/or Figure 4
   (c) Normalizing the expression profile of said first predetermined set of miRNAs to said second predetermined set of miRNAs
   (d) Comparing the normalized expression profile of step (c) to a reference
   (e) Diagnosing a change of the health status in said subject when the comparison to said reference is altered
   wherein said blood sample is selected from whole blood, blood cells, blood cellular fractions consisting of erythrocytes, leukocytes and thrombocytes, blood cellular fractions comprising erythrocytes, leukocytes and thrombocytes or blood cellular fractions comprising erythrocytes, leukocytes or thrombocytes.
2. The method according to item 1, wherein the normalizing in step (c) generates relative expression levels for each of the miRNAs comprised in said first predetermined set of miRNAs.
3. The method according to item 2, wherein said reference is a normalized reference expression profile that is obtained comprising the steps of:
   (i.) Determining the expression profile of a first predetermined set of one or more miRNAs in a blood sample from one or more reference subjects, wherein the miRNAs comprised in said first predetermined set of miRNAs are selected from miRNAs listed in Figure 1 and/or Figure 3
   (ii.) Determining the expression profile of a second predetermined set of one or more miRNAs in said blood sample from one or more reference subjects, wherein the miRNAs comprised in said second predetermined set of miRNAs are selected from miRNAs listed in Figure 2 and/or Figure 4
   (iii.) Normalizing the expression profile of said first predetermined set of miRNAs to said second predetermined set of miRNAs said one or more reference subjects
4. The method according to item 3, wherein the normalizing in step (iii) generates relative reference expression levels for each of the miRNAs comprised in said first predetermined set of miRNAs.
5. The method according to any of the items 1 to 4, wherein the comparison in step (d) is by comparing the relative expression levels from said subject to the relative reference expression levels for each of the miRNAs comprised in said first predetermined set of miRNAs.
6. The method according to any of the items 2, 4 or 5, wherein the relative expression levels are obtained by calculating a ratio from the (disease-regulated) miRNAs listed in Figure 1 and the (disease-preserved) miRNAs listed in Figure 2.
7. The method according to item 6, wherein the relative expression levels are selected from the group of ratios listed in Figure 7.
8. The method according to any of the items 1 to 7, wherein in step (e) a change of the health status is diagnosed when the comparison of the relative expression levels from said subject to the relative reference expression levels exceeds a threshold for one or more of said predetermined miRNAs comprised in said set.
9. The method according to any of the items 1 to 8, wherein said threshold is set at 3 standard deviations, preferably is set at 5 standard deviations, more preferably is set at 7 standard deviations.
10. A method for diagnosing the health status in a subject, comprising the steps of:
   (a) Determining the expression profile of a first predetermined set of miRNAs in a blood sample from a subject
   (b) Determining the expression profile of a second predetermined set of miRNAs in said blood sample from said subject
   (c) Comparing said expression profile of step (a) and said expression profile of step (b) to reference expression profiles
   (d) Diagnosing a change of the health status in said subject when the comparison to said first reference expression profile is altered and when the comparison to said second reference expression profile is not altered,
   wherein
   (i.) said miRNAs comprised in said first set of predetermined miRNAs and the miRNAs comprised in said first reference expression profile are selected from miRNAs listed in Figure 1 and/or Figure 3, and
   (ii.) said miRNAs comprised in said second set of predetermined miRNAs are selected from miRNAs listed in Figure 2 and/or Figure 4, and
   (iii.) wherein said blood sample is selected from whole blood, blood cells, blood cellular fractions consisting of erythrocytes, leukocytes and thrombocytes, blood cellular fractions comprising erythrocytes, leukocytes and thrombocytes or blood cellular fractions comprising erythrocytes, leukocytes or thrombocytes
11. The method according to any of the items 3 to 10, wherein said reference expression profiles are derived from an earlier time point from the same subject.
12. The method according to any of the items 1 to 11, wherein the change of the health status is an improvement or a worsening of the health status.
13. Use of the method according to any of the items 1 to 12for:
   (i.) comparing the health status between one or more subjects,
   (ii.) monitoring the health status in a subject,
   (iii.) monitoring the state of the immune system in a subject,
   (iv.) monitoring the response to therapeutic treatment, preferably drug treatment, in a subject.
14. The method, use or kit according to any of the items 1 to 13, wherein further miRNAs of said first set of (disease-regulated) miRNAs are selected from the miRNAs listed in Figure 3 and , wherein further miRNAs of said second set of (disease-preserved) miRNAs are selected from the miRNAs listed in Figure 3 and wherein the change of the health status relates to the absence or presence of cancer in said subject.

### BRIEF DESCRIPTION OF THE DRAWINGS

Figure 1: Overview of the disease-regulated miRNAs (SEQ ID NO: 1 to 26) determined in blood cell samples derived from whole blood samples collected in PAXgene Blood RNA tubes, comprising red blood cells, white blood cells and platelets. Herein, the blood samples were drawn into in PAXgene Blood RNA tubes, the total RNA of the blood cells - comprising the miRNA-fraction of blood cells - was isolated by use of the miRNeasy kit (http://www.qiagen.com) and analyzed on dna-microarrays (febit biomed) representing miRBase version 12 (Experimental details : SEQ ID NO: Sequence identification number; miRNA : miRNA annotation according to miRBase version 12; median g1(Healthy Control) = median expression level of healthy control patients; median g2 (Disease) = median expression level of the diseased subjects (; qmedian= ratio of median g1 (Healthy Control) and median g2 (Diseased); ttest_adjp= Benjamini-Hochberg-adjusted p-value calculated according to ttest; AUC= area under the curve statistics.
Figure 2: Overview of the disease-preserved miRNAs (SEQ ID NO: 27 to 59) determined in blood cell samples derived from whole blood samples collected in PAXgene Blood RNA tubes, comprising red blood cells, white blood cells and platelets. Herein, the blood samples were drawn into in PAXgene Blood RNA tubes, the total RNA of the blood cells - comprising the miRNA-fraction of blood cells - was isolated by use of the miRNeasy kit (http://www.qiagen.com) and analyzed on dna-microarrays (febit biomed) representing miRBase version 12 (Experimental details : SEQ ID NO: Sequence identification number; miRNA : miRNA annotation according to miRBase version 12; median g1(Healthy Control) = median expression level of healthy control patients; median g2 (Disease) = median expression level of the diseased subjects (; qmedian= ratio of median g1 (Healthy Control) and median g2 (Diseased); ttest_adjp= Benjamini-Hochberg-adjusted p-value calculated according to ttest; AUC= area under the curve statistics..
Figure 3: Overview of the cancer-regulated miRNAs determined in blood cell samples derived from whole blood samples collected in PAXgene Blood RNA tubes, comprising red blood cells, white blood cells and platelets. Herein, the blood samples were drawn into in PAXgene Blood RNA tubes, the total RNA of the blood cells - comprising the miRNA-fraction of blood cells - was isolated by use of the miRNeasy kit (http://www.qiagen.com) and analyzed on dna-microarrays (febit biomed) representing miRBase version 12 (Experimental details : SEQ ID NO: Sequence identification number; miRNA : miRNA annotation according to miRBase version 12; median g1(Healthy Control) = median expression level of healthy control patients; median g2 (Cancer) = median expression level of the diseased (cancer) subjects (; qmedian= ratio of median g1 (Healthy Control) and median g2 (Cancer); ttest_adjp= Benjamini-Hochberg-adjusted p-value calculated according to ttest; AUC= area under the curve statistics.
Figure 4: Overview of the cancer-preserved miRNAs (determined in blood cell samples derived from whole blood samples collected in PAXgene Blood RNA tubes, comprising red blood cells, white blood cells and platelets. Herein, the blood samples were drawn into in PAXgene Blood RNA tubes, the total RNA of the blood cells - comprising the miRNA-fraction of blood cells - was isolated by use of the miRNeasy kit (http://www.qiagen.com) and analyzed on dna-microarrays (febit biomed) representing miRBase version 12 (Experimental details : SEQ ID NO: Sequence identification number; miRNA : miRNA annotation according to miRBase version 12; median g1(Healthy Control) = median expression level of healthy control patients; median g2 (Cancer) = median expression level of the diseased (cancer) subjects (; qmedian= ratio of median g1 (Healthy Control) and median g2 (Cancer); ttest_adjp= Benjamini-Hochberg-adjusted p-value calculated according to ttest; AUC= area under the curve statistics..

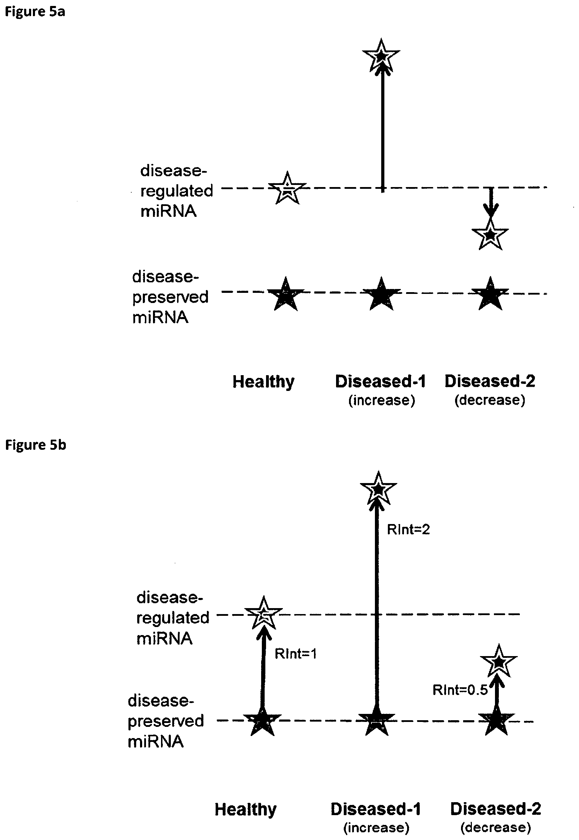

Figure 5: Schematic depiction of the method according to (a) the second aspect of the invention and (b) the first aspect of the invention.

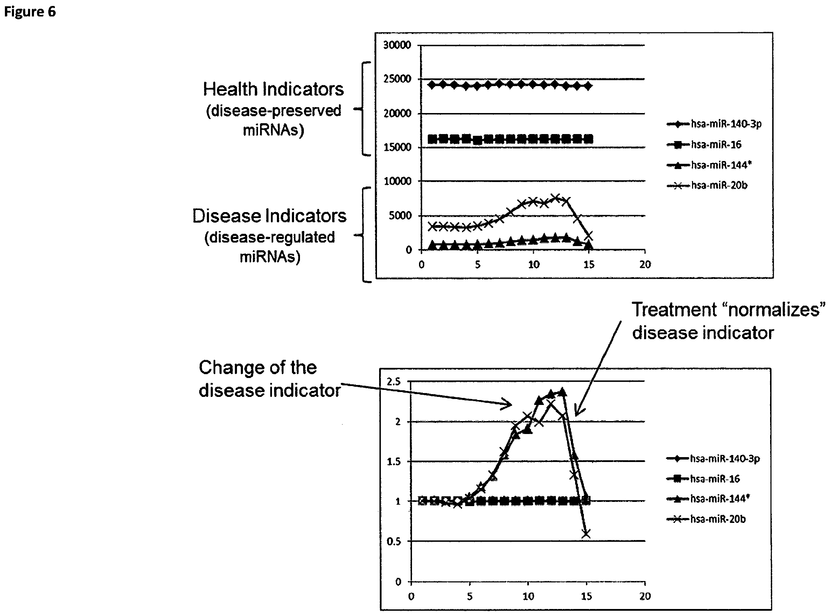

Figure 6: Graphical depiction for use of the method according for monitoring the health status in a subject according to the present invention.
Figure 7 : Boxplots depicting the relative expression levels obtained from normalizing the expression profile of a first predetermined set of one or more miRNAs (disease-regulated miRNAs selected from Figure 1) to a second predetermined set of miRNAs (disease-preserved miRNAs selected from Figure 2) . Herein the left grey boxplots represent the relative expression levels within Healthy Control subjects (n=67) and the right white boxplots represent the relative expression level within Diseased subjects (n=713); with pval = Benjamini-Hochberg-adjusted p-value calculated according to ttest. Herein all samples were collected and processed according to Examples 1-5 employing miRNAs derived from blood cellular fractions comprising erythrocytes, leukocytes and thrombocytes.
   With : Fig. 7-1 : hsa-miR-144^{∗}/hsa-miR-16 ; Fig. 7-2 : hsa-miR-144^{∗}/hsa-miR-92a ; Fig. 7-3 : hsa-miR-126/hsa-miR-16 ; Fig. 7-4 : hsa-miR-20b/hsa-miR-16 ; Fig. 7-5 : hsa-miR-20b/hsa-miR-140-3p ; Fig. 7-6 : hsa-miR-20b/hsa-miR-92a ; Fig. 7-7 : hsa-miR-363/hsa-miR-16 ; Fig. 7-8 : hsa-miR-20b/hsa-miR-22 ; Fig. 7-9 : hsa-miR-363/hsa-miR-192 ; Fig. 7-10 : hsa-miR-20b/hsa-miR-192 ; Fig. 7-11 : hsa-miR-720/hsa-miR-874 ; Fig. 7-12 : hsa-miR-17/hsa-miR-16 ; Fig. 7-13 : hsa-miR-20a/hsa-miR-140-3p ; Fig. 7-14 : hsa-miR-20a/hsa-miR-16 ; Fig. 7-15 : hsa-miR-144^{∗}/hsa-miR-30b ; Fig. 7-16 : hsa-miR-106a/hsa-miR-16 ; Fig. 7-17 : hsa-miR-17/hsa-miR-140-3p ; Fig. 7-18 : hsa-miR-144^{∗}/hsa-miR-140-3p ; Fig. 7-19 : hsa-miR-106a/hsa-miR-140-3p ; Fig. 7-20 : hsa-miR-93^{∗}/hsa-miR-27b ; Fig. 7-21 : hsa-miR-144^{∗}/hsa-miR-19a; Fig. 7-22 : hsa-miR-222/hsa-miR-140-3p ; Fig. 7-23 : hsa-miR-20b/hsa-miR-19a ; Fig. 7-24 : hsa-miR-363/hsa-miR-22 ; Fig. 7-25 : hsa-miR-363/hsa-miR-140-3p ; Fig. 7-26 : hsa-miR-93^{∗}/hsa-miR-99b ; Fig. 7-27 : hsa-miR-720/hsa-miR-19a ; Fig. 7-28 : hsa-miR-720/hsa-miR-361-3p.
   (Within the graphs the annotation of the miRNA-identifiers is truncated due to visualization constraints; herein the relative expression values are as listed above)
Figure 8: Overview of relative expression levels obtained from normalizing the expression profile of a first predetermined set of one or more miRNAs (disease-regulated miRNAs selected from Figure 1) to a second predetermined set of miRNAs (disease-preserved miRNAs selected from Figure 2). (Experimental details : miRNA = miRNA annotation according to miRBase version 12; median ratio r/p (Healthy Control) = median relative expression level in healthy control group with median calculated from ratios of expression level of disease-regulated miRNA (r) and disease-preserved miRNA (p) within healthy control group; median ratio r/p (Disease) = median relative expression level in disease group with median calculated from ratios of expression level of disease-regulated miRNA (r) and disease-preserved miRNA (p) within disease group; fold change = median fold change of median relative expression levels between Healthy Control and Diseasegroup; Wilcoxon Mann Whitney Test adjusted p-Value = adjusted p-value calculated according to WMW-test; t-Test adjusted p-Value = Benjamini-Hochberg-adjusted p-value calculated according to ttest; AUC= area under the curve statistics.

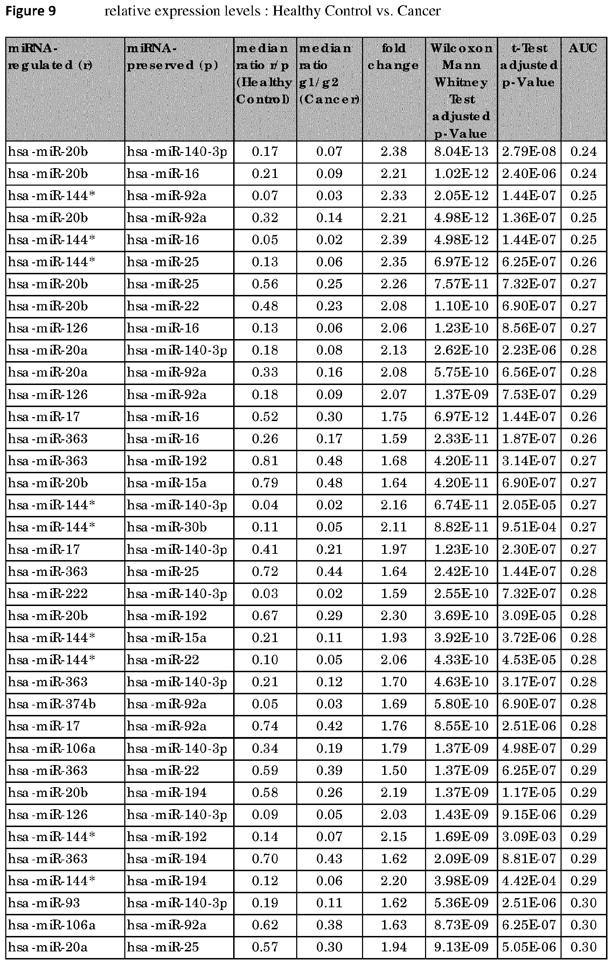

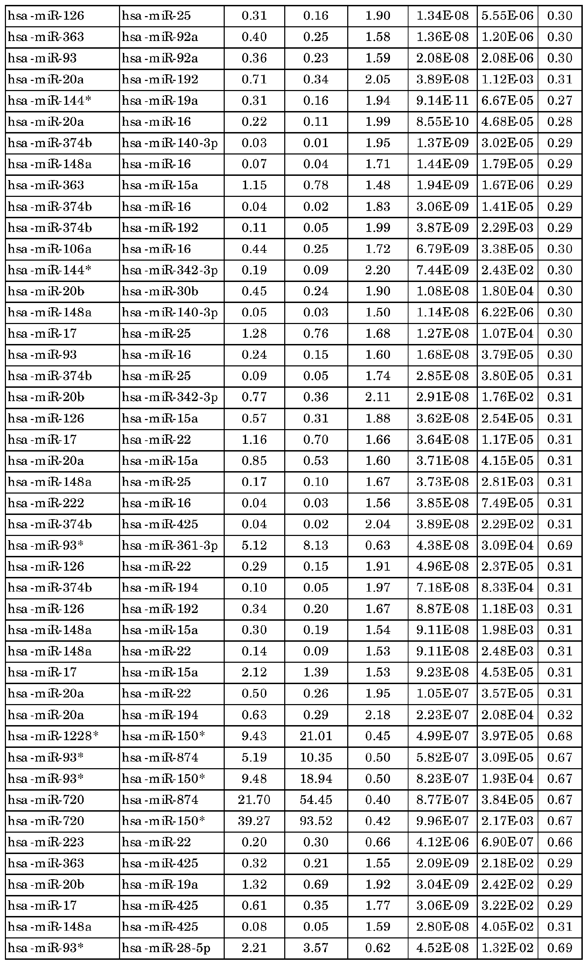

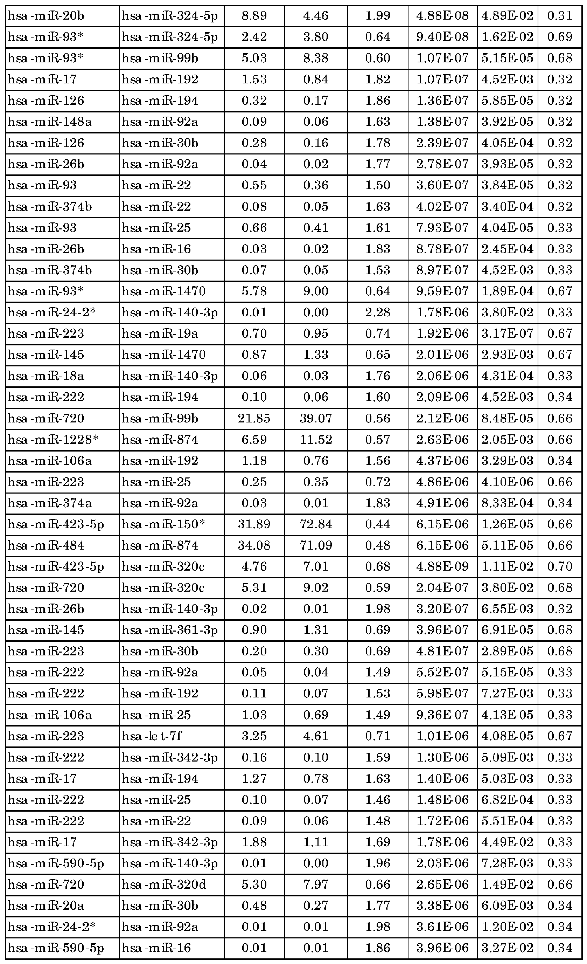

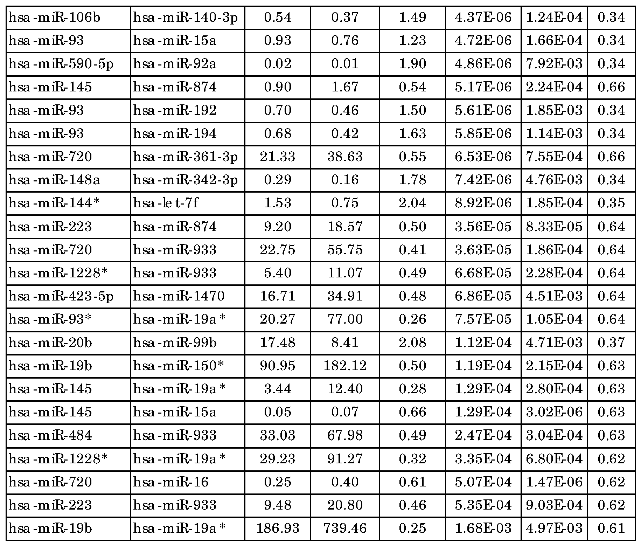

Figure 9: Overview of relative expression levels obtained from normalizing the expression profile of a first predetermined set of one or more miRNAs (cancer-regulated miRNAs selected from Figure 3) to a second predetermined set of miRNAs (cancer-preserved miRNAs selected from Figure 4 ). (Experimental details : miRNA = miRNA annotation according to miRBase version 12; median ratio r/p (Healthy Control) = median relative expression level in healthy control group with median calculated from ratios of expression level of cancer-regulated miRNA (r) and cancer-preserved miRNA (p) within healthy control group; median ratio r/p (Cancer) = median relative expression level in cancer group with median calculated from ratios of expression level of cancer-regulated miRNA (r) and cancer-preserved miRNA (p) within cancer group; fold change = median fold change of median relative expression levels between Healthy Control and Cancergroup; Wilcoxon Mann Whitney Test adjusted p-Value = adjusted p-value calculated according to WMW-test; t-Test adjusted p-Value = Benjamini-Hochberg-adjusted p-value calculated according to ttest; AUC= area under the curve statistics.

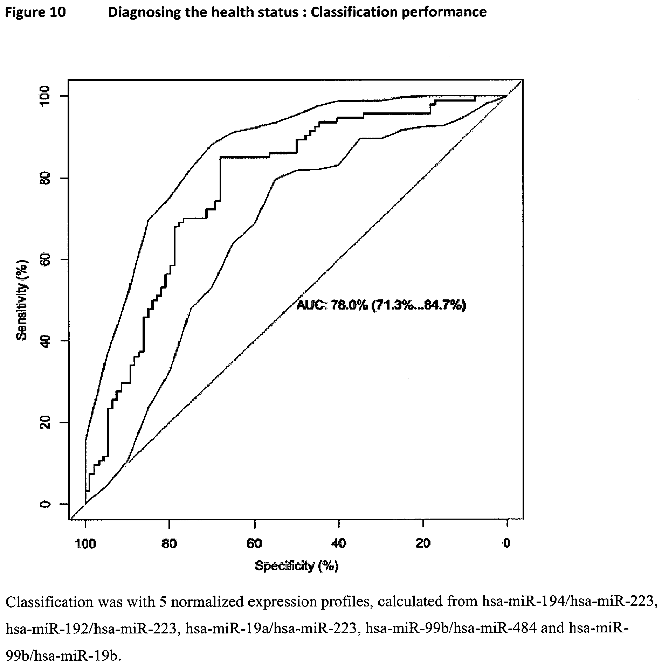

Figure 10: Classification performance of a predetermined set of 5 disease-regulated and 5 disease-preserved miRNAs for diagnosing the health status in a subject. The set consisting of 5 normalized expression profiles classifies the health status (health control vs. Disease) with an AUC of 0.78. Classification was with 5 normalized expression profiles, calculated from hsa-miR-194/hsa-miR-223, hsa-miR-192/hsa-miR-223, hsa-miR-19a/hsa-miR-223, hsa-miR-99b/hsa-miR-484 and hsa-miR-99b/hsa-miR-19b.

### EXAMPLES

The Examples are designed in order to further illustrate the present invention and serve a better understanding. They are not to be construed as limiting the scope of the invention in any way.

### EXAMPLE 1: Deriving blood cell samples from whole blood samples

Blood of healthy control and diseased subjects PAXgene Blood RNA Tubes (PreAnalytix). For each blood donor 2.5 ml in PAXgene Blood RNA Tubes. The blood cells preparations were derived/obtained from processing the whole blood samples by centrifugation. Herein, the blood cells from the whole blood collected in said blood collection tubes were spun down by 10 min, 5000xg centrifugation. The blood cell pellet (the cellular blood fraction comprising red blood cells, white blood cells and platelets) was harvested for further processing, while the supernatant (including the extra-cellular blood fraction) was discarded. Total RNA, including the small RNA (miRNA-fraction) was extracted from the harvested blood cells using the miRNeasy Mini Kit (Qiagen GmbH, Hilden, Germany); for details see Example 2.

### EXAMPLE 2: Isolation of total RNA incl. microRNA

The isolation of total RNA, including the small RNA (miRNA-fraction) was performed by use of the miRNeasy Mini Kit (Qiagen GmbH, Hilden, Germany). Herein, the blood cell pellet (obtained as outlined in Example 1) was thoroughly resuspended in 700 µl QIAzol lysis reagent by pipetting up and down and immediately the suspension was transferred to a new 1,5 ml Eppendorf tube. Then 140 µl chloroform were added, vortexed thoroughly and incubated for 2-3 min at room temperature, followed by centrifugation at 12,000 g for 15 min at 4°C. Afterwards, the upper, aqueous phase was transferred to a new 2 ml tube with great care, without touching the other two phases. Then 1.5 volumes of 100% ethanol were added to the transferred aqueous phase and thoroughly mixing was done by pipetting. 700 µl of sample were then transferred into a column and centrifuged at 13,000 rpm for 15 sec at RT, discarding the flow-through. Afterwards 700 µl of Buffer RWT were added to each column, centrifuged again at 13,000 rpm for 15 sec at RT, discarding the flow-through. Then 500 µl Buffer RPE was added to the column and centrifuged at 13,000 rpm for 15 sec at RT, discarding the flow-through. Afterwards another 500 µl Buffer RPE was added to the column and centrifuged at 13,000 rpm for 2 min at RT, discarding the flow-through. Then the column was placed into a new 2 ml collection tube and centrifuged at 13,000 rpm for 1 min at RT to dry it. The column was transferred into a new 1.5 ml collection tube. For elution of the total RNA incl. microRNA 40 µl RNase-free water was pipetted onto the column and incubated for 1 min, centrifuged at 13.000 rpm at RT for 1 min. Then the eluate was put back onto the same column, incubated for 1 min at RT and centrifuged again for 1 min. The eluted total RNA incl. microRNA was quantified using the NanoDrop 1000 and stored at -20°C before use in expression profiling experiments. For quality control of the total RNA, 1 µl of total RNA was applied on Agilent's Bioanalyzer, selecting either Agilent's nano- or pico- RNA Chip depending on RNA concentration determined by NanoDrop measurement.

### EXAMPLE 3: Microarray-based determination of expression profiles

The total RNA-samples including the miRNA-fraction (obtained by protocol Example 2) were analyzed employing microarray hybridization on the Geniom Realtime Analyzer (febit biomed GmbH, Heidelberg, Germany) using the Geniom Biochip miRNA homo sapiens. Each microfluidic microarray contains complementary dna-probes of 866 miRNAs and miRNA^{∗} (each represented by 7 replicates) as annotated in the Sanger miRBase 12.0. Sample labeling with biotine has been carried out by enzymatic on-chip labeling of miRNAs employing febit's MPEA-assay. Following hybridization for 16 hours at 42°C the biochip was washed automatically and a program for signal enhancement was processed with the Geniom Realtime Analyzer. The resulting detection pictures were evaluated using the Geniom Wizard Software. For each array, the median signal intensity was extracted from the raw data file such that for each miRNA seven intensity values have been calculated corresponding to each replicate copy of miRBase on the array. Following background correction, the seven replicate intensity values of each miRNA were summarized by their median value. To normalize the data across different arrays, quantile normalization was applied and all further analyses were carried out using the normalized and background subtracted intensity values. From median g1 and median g2 the Fold Change of the expression (=qmedian) was calculated as the ratio g1/g2.

### EXAMPLE 4: Statistical Analysis

After having verified the normal distribution of the measured data, a parametric t-test (unpaired, two-tailed) was carried out for each miRNA separately, to detect miRNAs that show a different behavior in different groups of blood donors. The resulting raw p-values were adjusted for multiple testing by Benjamini-Hochberg adjustment (=ttest_adj). Furthermore, we applied the limma-test for each miRNA separately and corrected according to Benjamini-Hochberg (=limma_adj). Additionally, we applied receiver operating characteristics and calculated the "Area under the Curve"-value (=AUC). The ttest-, limma-test- and AUC-values allow to judge on the statistical significance for each miRNA to be differential expressed between group 1 (g1 subjects) and group 2 (g2 subjects).

### EXAMPLE 5: Determination of disease-regulated and disease-preserved miRNAs

The goal of this study was to identify miRNAs, which show a significantly altered expression in different diseases and healthy controls, and miRNAs, which by contrast show very low expression variability in diseases and in controls. The two groups of miRNAs are in the following referred to as "disease-regulated" or "disease-preserved" miRNAs. In total, miRNA profiles have been obtained from 1,049 blood samples all of which were collected in PAXgene Blood RNA tubes using the same protocol. In each case the profiles were generated from the expression values of 848 miRNAs recorded by microarray analysis. To identify "disease-regulated" or "disease-preserved" miRNAs, we used t-test and AUC values. Using 20 different thresholds for p-values or AUC values we obtained different sets of "disease-regulated" (Figure 1) or "disease-preserved" miRNAs (Figure 2).

## Claims

1. A method for assessing the health status in a subject, comprising the steps of:
(a) isolating total intracellular RNA from erythrocytes, leukocytes, and thrombocytes which have been obtained from a whole blood sample which has been taken from a subject,
(b) determining in said total intracellular RNA an expression profile of one or more first miRNAs, wherein the one or more first miRNAs have a nucleotide sequence selected from the group consisting of SEQ ID NO: 1 to SEQ ID NO: 16 and SEQ ID NO: 20 to SEQ ID NO: 26,
(c) determining in said total intracellular RNA an expression profile of one or more second miRNAs, wherein the one or more second miRNAs have a nucleotide sequence selected from the group consisting of SEQ ID NO: 27, SEQ ID NO: 29, SEQ ID NO: 35, SEQ ID NO: 38, SEQ ID NO: 39, SEQ ID NO: 43 to SEQ ID NO: 47, SEQ ID NO: 49, SEQ ID NO: 50, SEQ ID NO: 52, SEQ ID NO: 55 to SEQ ID NO: 57, and SEQ ID NO: 59,
(d) normalizing the expression profile of said one or more first miRNAs to the expression profile of said one or more second miRNAs, thereby obtaining one or more relative expression levels of: SEQ ID NO: 1/SEQ ID NO: 45, SEQ ID NO: 1/SEQ ID NO: 59, SEQ ID NO: 1/SEQ ID NO: 56, SEQ ID NO: 2/SEQ ID NO: 27, SEQ ID NO: 2/SEQ ID NO: 35, SEQ ID NO: 3/SEQ ID NO: 27, SEQ ID NO: 4/SEQ ID NO: 55, SEQ ID NO: 4/SEQ ID NO: 47, SEQ ID NO: 4/SEQ ID NO: 29^{∗}, SEQ ID NO: 4/SEQ ID NO: 56, SEQ ID NO: 4/SEQ ID NO: 59, SEQ ID NO: 4/SEQ ID NO: 43, SEQ ID NO: 5/SEQ ID NO: 59, SEQ ID NO: 5/SEQ ID NO: 29, SEQ ID NO: 5/SEQ ID NO: 55, SEQ ID NO: 5/SEQ ID NO: 45, SEQ ID NO: 5/SEQ ID NO: 44, SEQ ID NO: 5/SEQ ID NO: 57, SEQ ID NO: 5/SEQ ID NO: 46, SEQ ID NO: 5/SEQ ID NO: 50, SEQ ID NO: 5/SEQ ID NO: 49, SEQ ID NO: 5/SEQ ID NO: 56, SEQ ID NO: 5/SEQ ID NO: 47, SEQ ID NO: 6/SEQ ID NO: 35, SEQ ID NO: 6/SEQ ID NO: 27, SEQ ID NO: 6/SEQ ID NO: 38, SEQ ID NO: 6/SEQ ID NO: 29, SEQ ID NO: 6/SEQ ID NO: 43, SEQ ID NO: 6/SEQ ID NO: 49, SEQ ID NO: 6/SEQ ID NO: 50, SEQ ID NO: 6/SEQ ID NO: 52, SEQ ID NO: 6/SEQ ID NO: 39, SEQ ID NO: 7/SEQ ID NO: 27, SEQ ID NO: 7/SEQ ID NO: 35, SEQ ID NO: 7/SEQ ID NO: 29, SEQ ID NO: 7/SEQ ID NO: 43, SEQ ID NO: 7/SEQ ID NO: 50, SEQ ID NO: 7/SEQ ID NO: 39, SEQ ID NO: 7/SEQ ID NO: 38, SEQ ID NO: 7/SEQ ID NO: 47, SEQ ID NO: 8/SEQ ID NO: 35, SEQ ID NO: 8/SEQ ID NO: 27, SEQ ID NO: 8/SEQ ID NO: 38, SEQ ID NO: 8/SEQ ID NO: 39, SEQ ID NO: 8/SEQ ID NO: 43, SEQ ID NO: 9/SEQ ID NO: 35, SEQ ID NO: 9/SEQ ID NO: 27, SEQ ID NO: 9/SEQ ID NO: 38, SEQ ID NO: 9/SEQ ID NO: 43, SEQ ID NO: 9/SEQ ID NO: 29, SEQ ID NO: 9/SEQ ID NO: 39, SEQ ID NO: 10/SEQ ID NO: 35, SEQ ID NO: 10/SEQ ID NO: 27, SEQ ID NO: 10/SEQ ID NO: 38, SEQ ID NO: 10/SEQ ID NO: 49, SEQ ID NO: 10/SEQ ID NO: 39, SEQ ID NO: 10/SEQ ID NO: 43, SEQ ID NO: 11/SEQ ID NO: 27, SEQ ID NO: 11/SEQ ID NO: 35, SEQ ID NO: 11/SEQ ID NO: 39, SEQ ID NO: 11/SEQ ID NO: 38, SEQ ID NO: 12/SEQ ID NO: 35, SEQ ID NO: 13/SEQ ID NO: 35, SEQ ID NO: 13/SEQ ID NO: 27, SEQ ID NO: 13/SEQ ID NO: 39, SEQ ID NO: 14/SEQ ID NO: 35, SEQ ID NO: 15/SEQ ID NO: 35, SEQ ID NO: 15/SEQ ID NO: 38, SEQ ID NO: 15/SEQ ID NO: 27, SEQ ID NO: 15/SEQ ID NO: 39, SEQ ID NO: 16/SEQ ID NO: 35, SEQ ID NO: 20/SEQ ID NO: 29, SEQ ID NO: 20/SEQ ID NO: 59, SEQ ID NO: 20/SEQ ID NO: 57, SEQ ID NO: 20/SEQ ID NO: 38, SEQ ID NO: 20/SEQ ID NO: 39, SEQ ID NO: 20/SEQ ID NO: 47, SEQ ID NO: 21/SEQ ID NO: 50, SEQ ID NO: 21/SEQ ID NO: 59, SEQ ID NO: 21/SEQ ID NO: 29^{∗}, SEQ ID NO: 21/SEQ ID NO: 57, SEQ ID NO: 21/SEQ ID NO: 55, SEQ ID NO: 21/SEQ ID NO: 49, SEQ ID NO: 21/SEQ ID NO: 56, SEQ ID NO: 22/SEQ ID NO: 59, SEQ ID NO: 22/SEQ ID NO: 47, SEQ ID NO: 22/SEQ ID NO: 56, SEQ ID NO: 22/SEQ ID NO: 57, SEQ ID NO: 22/SEQ ID NO: 29^{∗}, SEQ ID NO: 23/SEQ ID NO: 59, SEQ ID NO: 23/SEQ ID NO: 56, SEQ ID NO: 23/SEQ ID NO: 57, SEQ ID NO: 24/SEQ ID NO: 27, SEQ ID NO: 24/SEQ ID NO: 35, SEQ ID NO: 24/SEQ ID NO: 38, SEQ ID NO: 24/SEQ ID NO: 39, SEQ ID NO: 24/SEQ ID NO: 43, SEQ ID NO: 25/SEQ ID NO: 35, SEQ ID NO: 25/SEQ ID NO: 39, SEQ ID NO: 25/SEQ ID NO: 43, SEQ ID NO: 25/SEQ ID NO: 38, SEQ ID NO: 25/SEQ ID NO: 27, SEQ ID NO: 26/SEQ ID NO: 35, or SEQ ID NO: 26/SEQ ID NO: 27,
(e) comparing the one or more relative expression levels of step (d) to one or more relative reference expression levels, and
(f) assessing a change of the health status in said subject when the comparison to said one or more relative reference expression levels is altered.

2. The method according to claim 1, wherein said one or more relative reference expression levels are obtained comprising the steps of:
(i) isolating total intracellular RNA from erythrocytes, leukocytes, and thrombocytes which have been obtained from a whole blood sample which has been taken from one or more reference subjects,
(ii) determining in said total intracellular RNA a reference expression profile of one or more first miRNAs, wherein the one or more first miRNAs have a nucleotide sequence selected from the group consisting of SEQ ID NO: 1 to SEQ ID NO: 16 and SEQ ID NO: 20 to SEQ ID NO: 26,
(iii) determining in said total intracellular RNA a reference expression profile of one or more second miRNAs, wherein the one or more second miRNAs have a nucleotide sequence selected from the group consisting of SEQ ID NO: 27, SEQ ID NO: 29, SEQ ID NO: 35, SEQ ID NO: 38, SEQ ID NO: 39, SEQ ID NO: 43 to SEQ ID NO: 47, SEQ ID NO: 49, SEQ ID NO: 50, SEQ ID NO: 52, SEQ ID NO: 55 to SEQ ID NO: 57, and SEQ ID NO: 59, and
(iv) normalizing the reference expression profile of said one or more first miRNAs to the reference expression profile of said one or more second miRNAs of said one or more reference subjects thereby obtaining one or more relative reference expression levels of: SEQ ID NO: 1/SEQ ID NO: 45, SEQ ID NO: 1/SEQ ID NO: 59, SEQ ID NO: 1/SEQ ID NO: 56, SEQ ID NO: 2/SEQ ID NO: 27, SEQ ID NO: 2/SEQ ID NO: 35, SEQ ID NO: 3/SEQ ID NO: 27, SEQ ID NO: 4/SEQ ID NO: 55, SEQ ID NO: 4/SEQ ID NO: 47, SEQ ID NO: 4/SEQ ID NO: 29^{∗}, SEQ ID NO: 4/SEQ ID NO: 56, SEQ ID NO: 4/SEQ ID NO: 59, SEQ ID NO: 4/SEQ ID NO: 43, SEQ ID NO: 5/SEQ ID NO: 59, SEQ ID NO: 5/SEQ ID NO: 29, SEQ ID NO: 5/SEQ ID NO: 55, SEQ ID NO: 5/SEQ ID NO: 45, SEQ ID NO: 5/SEQ ID NO: 44, SEQ ID NO: 5/SEQ ID NO: 57, SEQ ID NO: 5/SEQ ID NO: 46, SEQ ID NO: 5/SEQ ID NO: 50, SEQ ID NO: 5/SEQ ID NO: 49, SEQ ID NO: 5/SEQ ID NO: 56, SEQ ID NO: 5/SEQ ID NO: 47, SEQ ID NO: 6/SEQ ID NO: 35, SEQ ID NO: 6/SEQ ID NO: 27, SEQ ID NO: 6/SEQ ID NO: 38, SEQ ID NO: 6/SEQ ID NO: 29, SEQ ID NO: 6/SEQ ID NO: 43, SEQ ID NO: 6/SEQ ID NO: 49, SEQ ID NO: 6/SEQ ID NO: 50, SEQ ID NO: 6/SEQ ID NO: 52, SEQ ID NO: 6/SEQ ID NO: 39, SEQ ID NO: 7/SEQ ID NO: 27, SEQ ID NO: 7/SEQ ID NO: 35, SEQ ID NO: 7/SEQ ID NO: 29, SEQ ID NO: 7/SEQ ID NO: 43, SEQ ID NO: 7/SEQ ID NO: 50, SEQ ID NO: 7/SEQ ID NO: 39, SEQ ID NO: 7/SEQ ID NO: 38, SEQ ID NO: 7/SEQ ID NO: 47, SEQ ID NO: 8/SEQ ID NO: 35, SEQ ID NO: 8/SEQ ID NO: 27, SEQ ID NO: 8/SEQ ID NO: 38, SEQ ID NO: 8/SEQ ID NO: 39, SEQ ID NO: 8/SEQ ID NO: 43, SEQ ID NO: 9/SEQ ID NO: 35, SEQ ID NO: 9/SEQ ID NO: 27, SEQ ID NO: 9/SEQ ID NO: 38, SEQ ID NO: 9/SEQ ID NO: 43, SEQ ID NO: 9/SEQ ID NO: 29, SEQ ID NO: 9/SEQ ID NO: 39, SEQ ID NO: 10/SEQ ID NO: 35, SEQ ID NO: 10/SEQ ID NO: 27, SEQ ID NO: 10/SEQ ID NO: 38, SEQ ID NO: 10/SEQ ID NO: 49, SEQ ID NO: 10/SEQ ID NO: 39, SEQ ID NO: 10/SEQ ID NO: 43, SEQ ID NO: 11/SEQ ID NO: 27, SEQ ID NO: 11/SEQ ID NO: 35, SEQ ID NO: 11/SEQ ID NO: 39, SEQ ID NO: 11/SEQ ID NO: 38, SEQ ID NO: 12/SEQ ID NO: 35, SEQ ID NO: 13/SEQ ID NO: 35, SEQ ID NO: 13/SEQ ID NO: 27, SEQ ID NO: 13/SEQ ID NO: 39, SEQ ID NO: 14/SEQ ID NO: 35, SEQ ID NO: 15/SEQ ID NO: 35, SEQ ID NO: 15/SEQ ID NO: 38, SEQ ID NO: 15/SEQ ID NO: 27, SEQ ID NO: 15/SEQ ID NO: 39, SEQ ID NO: 16/SEQ ID NO: 35, SEQ ID NO: 20/SEQ ID NO: 29, SEQ ID NO: 20/SEQ ID NO: 59, SEQ ID NO: 20/SEQ ID NO: 57, SEQ ID NO: 20/SEQ ID NO: 38, SEQ ID NO: 20/SEQ ID NO: 39, SEQ ID NO: 20/SEQ ID NO: 47, SEQ ID NO: 21/SEQ ID NO: 50, SEQ ID NO: 21/SEQ ID NO: 59, SEQ ID NO: 21/SEQ ID NO: 29^{∗}, SEQ ID NO: 21/SEQ ID NO: 57, SEQ ID NO: 21/SEQ ID NO: 55, SEQ ID NO: 21/SEQ ID NO: 49, SEQ ID NO: 21/SEQ ID NO: 56, SEQ ID NO: 22/SEQ ID NO: 59, SEQ ID NO: 22/SEQ ID NO: 47, SEQ ID NO: 22/SEQ ID NO: 56, SEQ ID NO: 22/SEQ ID NO: 57, SEQ ID NO: 22/SEQ ID NO: 29^{∗}, SEQ ID NO: 23/SEQ ID NO: 59, SEQ ID NO: 23/SEQ ID NO: 56, SEQ ID NO: 23/SEQ ID NO: 57, SEQ ID NO: 24/SEQ ID NO: 27, SEQ ID NO: 24/SEQ ID NO: 35, SEQ ID NO: 24/SEQ ID NO: 38, SEQ ID NO: 24/SEQ ID NO: 39, SEQ ID NO: 24/SEQ ID NO: 43, SEQ ID NO: 25/SEQ ID NO: 35, SEQ ID NO: 25/SEQ ID NO: 39, SEQ ID NO: 25/SEQ ID NO: 43, SEQ ID NO: 25/SEQ ID NO: 38, SEQ ID NO: 25/SEQ ID NO: 27, SEQ ID NO: 26/SEQ ID NO: 35, or SEQ ID NO: 26/SEQ ID NO: 27.

3. The method according to claim 1, wherein the relative expression levels are obtained by calculating a ratio from the one or more first miRNAs having a nucleotide sequence selected from the group consisting of SEQ ID NO: 1 to SEQ ID NO: 16 and SEQ ID NO: 20 to SEQ ID NO: 26 and the one or more second miRNAs having a nucleotide sequence selected from the group consisting of SEQ ID NO: 27, SEQ ID NO: 29, SEQ ID NO: 35, SEQ ID NO: 38, SEQ ID NO: 39, SEQ ID NO: 43 to SEQ ID NO: 47, SEQ ID NO: 49, SEQ ID NO: 50, SEQ ID NO: 52, SEQ ID NO: 55 to SEQ ID NO: 57, and SEQ ID NO: 59, wherein the relative expression levels are preferably selected from the group of ratios listed in Figure 8.

4. The method according to any of the claims 1 to 3, wherein in step (f) a change of the health status is assessed when the comparison of the relative expression levels from said subject to the relative reference expression levels exceeds a threshold.

5. The method according to claim 4, wherein said threshold is set at 3 standard deviations, preferably is set at 5 standard deviations, more preferably is set at 7 standard deviations.

6. A method for assessing the health status in a subject, comprising the steps of:
(a) isolating total intracellular RNA from erythrocytes, leukocytes, and thrombocytes which have been obtained from a whole blood sample which has been taken from a subject,
(b) determining in said total intracellular RNA an expression profile of at least one first miRNA,
(c) determining in said total intracellular RNA an expression profile of at least one second miRNA,
(d) comparing the expression profile of the at least one first miRNA of step (b) to a reference expression profile of said at least one first miRNA and the expression profile of the at least one second miRNA of step (c) to a reference expression profile of said at least one second miRNA, and
(e) assessing a change of the health status in said subject when the comparison of the expression profile of the at least one first miRNA to the reference expression profile of said at least one first miRNA is altered and when the comparison of the expression profile of the at least one second miRNA to the reference expression profile of said at least one second miRNA is not altered,
wherein
the at least one first miRNA has a nucleotide sequence according to SEQ ID NO: 1 and the at least one second miRNA has a nucleotide sequence according to SEQ ID NO: 27,
the at least one first miRNA has a nucleotide sequence according to SEQ ID NO: 1 and the at least one second miRNA has a nucleotide sequence according to SEQ ID NO: 29,
the at least one first miRNA has a nucleotide sequence according to SEQ ID NO: 1 and the at least one second miRNA has a nucleotide sequence according to SEQ ID NO: 35,
the at least one first miRNA has a nucleotide sequence according to SEQ ID NO: 2 and the at least one second miRNA has a nucleotide sequence according to SEQ ID NO: 27,
the at least one first miRNA has a nucleotide sequence according to SEQ ID NO: 2 and the at least one second miRNA has a nucleotide sequence according to SEQ ID NO: 29,
the at least one first miRNA has a nucleotide sequence according to SEQ ID NO: 3 and the at least one second miRNA has a nucleotide sequence according to SEQ ID NO: 27,
the at least one first miRNA has a nucleotide sequence according to SEQ ID NO: 3 and the at least one second miRNA has a nucleotide sequence according to SEQ ID NO: 29,
the at least one first miRNA has a nucleotide sequence according to SEQ ID NO: 4 and the at least one second miRNA has a nucleotide sequence according to SEQ ID NO: 27,
the at least one first miRNA has a nucleotide sequence according to SEQ ID NO: 4 and the at least one second miRNA has a nucleotide sequence according to SEQ ID NO: 29,
the at least one first miRNA has a nucleotide sequence according to SEQ ID NO: 5 and the at least one second miRNA has a nucleotide sequence according to SEQ ID NO: 27,
the at least one first miRNA has a nucleotide sequence according to SEQ ID NO: 5 and the at least one second miRNA has a nucleotide sequence according to SEQ ID NO: 29,
the at least one first miRNA has a nucleotide sequence according to SEQ ID NO: 5 and the at least one second miRNA has a nucleotide sequence according to SEQ ID NO: 55,
the at least one first miRNA has a nucleotide sequence according to SEQ ID NO: 5 and the at least one second miRNA has a nucleotide sequence according to SEQ ID NO: 59,
the at least one first miRNA has a nucleotide sequence according to SEQ ID NO: 6 and the at least one second miRNA has a nucleotide sequence according to SEQ ID NO: 27,
the at least one first miRNA has a nucleotide sequence according to SEQ ID NO: 6 and the at least one second miRNA has a nucleotide sequence according to SEQ ID NO: 29,
the at least one first miRNA has a nucleotide sequence according to SEQ ID NO: 6 and the at least one second miRNA has a nucleotide sequence according to SEQ ID NO: 35,
the at least one first miRNA has a nucleotide sequence according to SEQ ID NO: 6 and the at least one second miRNA has a nucleotide sequence according to SEQ ID NO: 38,
the at least one first miRNA has a nucleotide sequence according to SEQ ID NO: 7 and the at least one second miRNA has a nucleotide sequence according to SEQ ID NO: 27,
the at least one first miRNA has a nucleotide sequence according to SEQ ID NO: 7 and the at least one second miRNA has a nucleotide sequence according to SEQ ID NO: 29,
the at least one first miRNA has a nucleotide sequence according to SEQ ID NO: 7 and the at least one second miRNA has a nucleotide sequence according to SEQ ID NO: 35,
the at least one first miRNA has a nucleotide sequence according to SEQ ID NO: 8 and the at least one second miRNA has a nucleotide sequence according to SEQ ID NO: 27,
the at least one first miRNA has a nucleotide sequence according to SEQ ID NO: 8 and the at least one second miRNA has a nucleotide sequence according to SEQ ID NO: 29,
the at least one first miRNA has a nucleotide sequence according to SEQ ID NO: 8 and the at least one second miRNA has a nucleotide sequence according to SEQ ID NO: 35,
the at least one first miRNA has a nucleotide sequence according to SEQ ID NO: 9 and the at least one second miRNA has a nucleotide sequence according to SEQ ID NO: 27,
the at least one first miRNA has a nucleotide sequence according to SEQ ID NO: 10 and the at least one second miRNA has a nucleotide sequence according to SEQ ID NO: 35,
the at least one first miRNA has a nucleotide sequence according to SEQ ID NO: 13 and the at least one second miRNA has a nucleotide sequence according to SEQ ID NO: 35,
the at least one first miRNA has a nucleotide sequence according to SEQ ID NO: 15 and the at least one second miRNA has a nucleotide sequence according to SEQ ID NO: 35,
the at least one first miRNA has a nucleotide sequence according to SEQ ID NO: 24 and the at least one second miRNA has a nucleotide sequence according to SEQ ID NO: 35, or
the at least one first miRNA has a nucleotide sequence according to SEQ ID NO: 24 and the at least one second miRNA has a nucleotide sequence according to SEQ ID NO: 38.

7. The method according to any of the claims 2 to 5, wherein the one or more relative reference expression levels are obtained from an earlier time point from the same subject.

8. The method according to any of the claims 1 to 7, wherein the change of the health status is an improvement or a worsening of the health status.

9. Use of the method according to any of the claims 1 to 8 for:
(i.) comparing the health status between one or more subjects,
(ii.) monitoring the health status in a subject,
(iii.) monitoring the state of the immune system in a subject, and/or
(iv.) monitoring the response to therapeutic treatment, preferably drug treatment, in a subject.

10. A method for monitoring the health status in a subject, comprising the steps of:
(a) assessing the health status in a subject at a first point in time by carrying out the method according to any one of claims 1 to 8,
(b) assessing the health status in the subject at one or more later points in time by carrying out the method according to any one of claims 1 to 8, and
(c) comparing the health status assessed in step (a) with the health status assessed in step (b), thereby monitoring the health status in the subject,
wherein the health status in the subject which is monitored preferably encompasses the state of the immune system of said subject.

11. The method of claim 10, wherein the health status in the subject improves or worsens over time.

12. The method of claims 10 or 11, wherein the subject has received a therapeutic treatment between the first point in time and the one or more later points in time.

13. The method of claim 12, wherein the therapeutic treatment comprises the administration of a drug, surgery, chemotherapy, radiation therapy, and/or immunotherapy.

## Patentansprüche

1. Verfahren zur Beurteilung des Gesundheitszustands eines Subjekts, umfassend die Schritte:
(a) Isolieren der gesamten intrazellulären RNA aus Erythrozyten, Leukozyten und Thrombozyten, die aus einer Vollblutprobe erhalten wurden, die einem Subjekt entnommen wurde,
(b) Bestimmen eines Expressionsprofils einer oder mehrerer erster miRNAs in der gesamten intrazellulären RNA, wobei die eine oder mehreren ersten miRNAs eine Nukleotidsequenz aufweisen, die aus der Gruppe bestehend aus SEQ ID NO: 1 bis SEQ ID NO: 16 und SEQ ID NO: 20 bis SEQ ID NO: 26 ausgewählt ist,
(c) Bestimmen eines Expressionsprofils einer oder mehrerer zweiter miRNAs in der gesamten intrazellulären RNA, wobei die eine oder mehreren zweiten miRNAs eine Nukleotidsequenz aufweisen, die aus der Gruppe bestehend aus SEQ ID NO: 27, SEQ ID NO: 29, SEQ ID NO: 35, SEQ ID NO: 38, SEQ ID NO: 39, SEQ ID NO: 43 bis SEQ ID NO: 47, SEQ ID NO: 49, SEQ ID NO: 50, SEQ ID NO: 52, SEQ ID NO: 55 bis SEQ ID NO: 57, und SEQ ID NO: 59 ausgewählt ist,
(d) Normalisieren des Expressionsprofils der einen oder mehreren ersten miRNAs auf das Expressionsprofil der einen oder mehreren zweiten miRNAs, wobei ein oder mehrere relative Expressionslevel von: SEQ ID NO: 1/SEQ ID NO: 45, SEQ ID NO: 1/SEQ ID NO: 59, SEQ ID NO: 1/SEQ ID NO: 56, SEQ ID NO: 2/SEQ ID NO: 27, SEQ ID NO: 2/SEQ ID NO: 35, SEQ ID NO: 3/SEQ ID NO: 27, SEQ ID NO: 4/SEQ ID NO: 55, SEQ ID NO: 4/SEQ ID NO: 47, SEQ ID NO: 4/SEQ ID NO: 29^{∗}, SEQ ID NO: 4/SEQ ID NO: 56, SEQ ID NO: 4/SEQ ID NO: 59, SEQ ID NO: 4/SEQ ID NO: 43, SEQ ID NO: 5/SEQ ID NO: 59, SEQ ID NO: 5/SEQ ID NO: 29, SEQ ID NO: 5/SEQ ID NO: 55, SEQ ID NO: 5/SEQ ID NO: 45, SEQ ID NO: 5/SEQ ID NO: 44, SEQ ID NO: 5/SEQ ID NO: 57, SEQ ID NO: 5/SEQ ID NO: 46, SEQ ID NO: 5/SEQ ID NO: 50, SEQ ID NO: 5/SEQ ID NO: 49, SEQ ID NO: 5/SEQ ID NO: 56, SEQ ID NO: 5/SEQ ID NO: 47, SEQ ID NO: 6/SEQ ID NO: 35, SEQ ID NO: 6/SEQ ID NO: 27, SEQ ID NO: 6/SEQ ID NO: 38, SEQ ID NO: 6/SEQ ID NO: 29, SEQ ID NO: 6/SEQ ID NO: 43, SEQ ID NO: 6/SEQ ID NO: 49, SEQ ID NO: 6/SEQ ID NO: 50, SEQ ID NO: 6/SEQ ID NO: 52, SEQ ID NO: 6/SEQ ID NO: 39, SEQ ID NO: 7/SEQ ID NO: 27, SEQ ID NO: 7/SEQ ID NO: 35, SEQ ID NO: 7/SEQ ID NO: 29, SEQ ID NO: 7/SEQ ID NO: 43, SEQ ID NO: 7/SEQ ID NO: 50, SEQ ID NO: 7/SEQ ID NO: 39, SEQ ID NO: 7/SEQ ID NO: 38, SEQ ID NO: 7/SEQ ID NO: 47, SEQ ID NO: 8/SEQ ID NO: 35, SEQ ID NO: 8/SEQ ID NO: 27, SEQ ID NO: 8/SEQ ID NO: 38, SEQ ID NO: 8/SEQ ID NO: 39, SEQ ID NO: 8/SEQ ID NO: 43, SEQ ID NO: 9/SEQ ID NO: 35, SEQ ID NO: 9/SEQ ID NO: 27, SEQ ID NO: 9/SEQ ID NO: 38, SEQ ID NO: 9/SEQ ID NO: 43, SEQ ID NO: 9/SEQ ID NO: 29, SEQ ID NO: 9/SEQ ID NO: 39, SEQ ID NO: 10/SEQ ID NO: 35, SEQ ID NO: 10/SEQ ID NO: 27, SEQ ID NO: 10/SEQ ID NO: 38, SEQ ID NO: 10/SEQ ID NO: 49, SEQ ID NO: 10/SEQ ID NO: 39, SEQ ID NO: 10/SEQ ID NO: 43, SEQ ID NO: 11/SEQ ID NO: 27, SEQ ID NO: 11/SEQ ID NO: 35, SEQ ID NO: 11/SEQ ID NO: 39, SEQ ID NO: 11/SEQ ID NO: 38, SEQ ID NO: 12/SEQ ID NO: 35, SEQ ID NO: 13/SEQ ID NO: 35, SEQ ID NO: 13/SEQ ID NO: 27, SEQ ID NO: 13/SEQ ID NO: 39, SEQ ID NO: 14/SEQ ID NO: 35, SEQ ID NO: 15/SEQ ID NO: 35, SEQ ID NO: 15/SEQ ID NO: 38, SEQ ID NO: 15/SEQ ID NO: 27, SEQ ID NO: 15/SEQ ID NO: 39, SEQ ID NO: 16/SEQ ID NO: 35, SEQ ID NO: 20/SEQ ID NO: 29, SEQ ID NO: 20/SEQ ID NO: 59, SEQ ID NO: 20/SEQ ID NO: 57, SEQ ID NO: 20/SEQ ID NO: 38, SEQ ID NO: 20/SEQ ID NO: 39, SEQ ID NO: 20/SEQ ID NO: 47, SEQ ID NO: 21/SEQ ID NO: 50, SEQ ID NO: 21/SEQ ID NO: 59, SEQ ID NO: 21/SEQ ID NO: 29^{∗}, SEQ ID NO: 21/SEQ ID NO: 57, SEQ ID NO: 21/SEQ ID NO: 55, SEQ ID NO: 21/SEQ ID NO: 49, SEQ ID NO: 21/SEQ ID NO: 56, SEQ ID NO: 22/SEQ ID NO: 59, SEQ ID NO: 22/SEQ ID NO: 47, SEQ ID NO: 22/SEQ ID NO: 56, SEQ ID NO: 22/SEQ ID NO: 57, SEQ ID NO: 22/SEQ ID NO: 29^{∗}, SEQ ID NO: 23/SEQ ID NO: 59, SEQ ID NO: 23/SEQ ID NO: 56, SEQ ID NO: 23/SEQ ID NO: 57, SEQ ID NO: 24/SEQ ID NO: 27, SEQ ID NO: 24/SEQ ID NO: 35, SEQ ID NO: 24/SEQ ID NO: 38, SEQ ID NO: 24/SEQ ID NO: 39, SEQ ID NO: 24/SEQ ID NO: 43, SEQ ID NO: 25/SEQ ID NO: 35, SEQ ID NO: 25/SEQ ID NO: 39, SEQ ID NO: 25/SEQ ID NO: 43, SEQ ID NO: 25/SEQ ID NO: 38, SEQ ID NO: 25/SEQ ID NO: 27, SEQ ID NO: 26/SEQ ID NO: 35, or SEQ ID NO: 26/SEQ ID NO: 27 erhalten werden,
(e) Vergleichen des einen oder der mehreren relativen Expressionslevel von Schritt (d) mit einem oder mehreren relativen Referenzexpressionsleveln, und
(f) Beurteilen einer Änderung des Gesundheitszustands des Subjekts, wenn sich der Vergleich mit dem einen oder den mehreren relativen Referenzexpressionsleveln ändert.

2. Verfahren nach Anspruch 1, wobei das eine oder die mehreren relativen Referenzexpressionslevel erhalten werden, umfassend die Schritte:
(i) Isolieren der gesamten intrazellulären RNA aus Erythrozyten, Leukozyten und Thrombozyten, die aus einer Vollblutprobe erhalten wurden, die einem oder mehreren Referenzsubjekten entnommen wurde,
(ii) Bestimmen eines Referenzexpressionsprofils einer oder mehrerer erster miRNAs in der gesamten intrazellulären RNA, wobei die eine oder mehreren ersten miRNAs eine Nukleotidsequenz aufweisen, die aus der Gruppe bestehend aus SEQ ID NO: 1 bis SEQ ID NO: 16 und SEQ ID NO: 20 bis SEQ ID NO: 26 ausgewählt ist,
(iii) Bestimmen eines Referenzexpressionsprofils einer oder mehrerer zweiter miRNAs in der gesamten intrazellulären RNA, wobei die eine oder mehreren zweiten miRNAs eine Nukleotidsequenz aufweisen, die aus der Gruppe bestehend aus SEQ ID NO: 27, SEQ ID NO: 29, SEQ ID NO: 35, SEQ ID NO: 38, SEQ ID NO: 39, SEQ ID NO: 43 bis SEQ ID NO: 47, SEQ ID NO: 49, SEQ ID NO: 50, SEQ ID NO: 52, SEQ ID NO: 55 bis SEQ ID NO: 57, und SEQ ID NO: 59 ausgewählt ist, und
(iv) Normalisieren des Referenzexpressionsprofils der einen oder mehreren ersten miRNAs auf das Referenzexpressionsprofil der einen oder mehreren zweiten miRNAs des einen oder der mehreren Referenzsubjekte, wobei ein oder mehrere relative Referenzexpressionslevel von: SEQ ID NO: 1/SEQ ID NO: 45, SEQ ID NO: 1/SEQ ID NO: 59, SEQ ID NO: 1/SEQ ID NO: 56, SEQ ID NO: 2/SEQ ID NO: 27, SEQ ID NO: 2/SEQ ID NO: 35, SEQ ID NO: 3/SEQ ID NO: 27, SEQ ID NO: 4/SEQ ID NO: 55, SEQ ID NO: 4/SEQ ID NO: 47, SEQ ID NO: 4/SEQ ID NO: 29^{∗}, SEQ ID NO: 4/SEQ ID NO: 56, SEQ ID NO: 4/SEQ ID NO: 59, SEQ ID NO: 4/SEQ ID NO: 43, SEQ ID NO: 5/SEQ ID NO: 59, SEQ ID NO: 5/SEQ ID NO: 29, SEQ ID NO: 5/SEQ ID NO: 55, SEQ ID NO: 5/SEQ ID NO: 45, SEQ ID NO: 5/SEQ ID NO: 44, SEQ ID NO: 5/SEQ ID NO: 57, SEQ ID NO: 5/SEQ ID NO: 46, SEQ ID NO: 5/SEQ ID NO: 50, SEQ ID NO: 5/SEQ ID NO: 49, SEQ ID NO: 5/SEQ ID NO: 56, SEQ ID NO: 5/SEQ ID NO: 47, SEQ ID NO: 6/SEQ ID NO: 35, SEQ ID NO: 6/SEQ ID NO: 27, SEQ ID NO: 6/SEQ ID NO: 38, SEQ ID NO: 6/SEQ ID NO: 29, SEQ ID NO: 6/SEQ ID NO: 43, SEQ ID NO: 6/SEQ ID NO: 49, SEQ ID NO: 6/SEQ ID NO: 50, SEQ ID NO: 6/SEQ ID NO: 52, SEQ ID NO: 6/SEQ ID NO: 39, SEQ ID NO: 7/SEQ ID NO: 27, SEQ ID NO: 7/SEQ ID NO: 35, SEQ ID NO: 7/SEQ ID NO: 29, SEQ ID NO: 7/SEQ ID NO: 43, SEQ ID NO: 7/SEQ ID NO: 50, SEQ ID NO: 7/SEQ ID NO: 39, SEQ ID NO: 7/SEQ ID NO: 38, SEQ ID NO: 7/SEQ ID NO: 47, SEQ ID NO: 8/SEQ ID NO: 35, SEQ ID NO: 8/SEQ ID NO: 27, SEQ ID NO: 8/SEQ ID NO: 38, SEQ ID NO: 8/SEQ ID NO: 39, SEQ ID NO: 8/SEQ ID NO: 43, SEQ ID NO: 9/SEQ ID NO: 35, SEQ ID NO: 9/SEQ ID NO: 27, SEQ ID NO: 9/SEQ ID NO: 38, SEQ ID NO: 9/SEQ ID NO: 43, SEQ ID NO: 9/SEQ ID NO: 29, SEQ ID NO: 9/SEQ ID NO: 39, SEQ ID NO: 10/SEQ ID NO: 35, SEQ ID NO: 10/SEQ ID NO: 27, SEQ ID NO: 10/SEQ ID NO: 38, SEQ ID NO: 10/SEQ ID NO: 49, SEQ ID NO: 10/SEQ ID NO: 39, SEQ ID NO: 10/SEQ ID NO: 43, SEQ ID NO: 11/SEQ ID NO: 27, SEQ ID NO: 11/SEQ ID NO: 35, SEQ ID NO: 11/SEQ ID NO: 39, SEQ ID NO: 11/SEQ ID NO: 38, SEQ ID NO: 12/SEQ ID NO: 35, SEQ ID NO: 13/SEQ ID NO: 35, SEQ ID NO: 13/SEQ ID NO: 27, SEQ ID NO: 13/SEQ ID NO: 39, SEQ ID NO: 14/SEQ ID NO: 35, SEQ ID NO: 15/SEQ ID NO: 35, SEQ ID NO: 15/SEQ ID NO: 38, SEQ ID NO: 15/SEQ ID NO: 27, SEQ ID NO: 15/SEQ ID NO: 39, SEQ ID NO: 16/SEQ ID NO: 35, SEQ ID NO: 20/SEQ ID NO: 29, SEQ ID NO: 20/SEQ ID NO: 59, SEQ ID NO: 20/SEQ ID NO: 57, SEQ ID NO: 20/SEQ ID NO: 38, SEQ ID NO: 20/SEQ ID NO: 39, SEQ ID NO: 20/SEQ ID NO: 47, SEQ ID NO: 21/SEQ ID NO: 50, SEQ ID NO: 21/SEQ ID NO: 59, SEQ ID NO: 21/SEQ ID NO: 29^{∗}, SEQ ID NO: 21/SEQ ID NO: 57, SEQ ID NO: 21/SEQ ID NO: 55, SEQ ID NO: 21/SEQ ID NO: 49, SEQ ID NO: 21/SEQ ID NO: 56, SEQ ID NO: 22/SEQ ID NO: 59, SEQ ID NO: 22/SEQ ID NO: 47, SEQ ID NO: 22/SEQ ID NO: 56, SEQ ID NO: 22/SEQ ID NO: 57, SEQ ID NO: 22/SEQ ID NO: 29^{∗}, SEQ ID NO: 23/SEQ ID NO: 59, SEQ ID NO: 23/SEQ ID NO: 56, SEQ ID NO: 23/SEQ ID NO: 57, SEQ ID NO: 24/SEQ ID NO: 27, SEQ ID NO: 24/SEQ ID NO: 35, SEQ ID NO: 24/SEQ ID NO: 38, SEQ ID NO: 24/SEQ ID NO: 39, SEQ ID NO: 24/SEQ ID NO: 43, SEQ ID NO: 25/SEQ ID NO: 35, SEQ ID NO: 25/SEQ ID NO: 39, SEQ ID NO: 25/SEQ ID NO: 43, SEQ ID NO: 25/SEQ ID NO: 38, SEQ ID NO: 25/SEQ ID NO: 27, SEQ ID NO: 26/SEQ ID NO: 35, or SEQ ID NO: 26/SEQ ID NO: 27 erhalten werden.

3. Verfahren nach Anspruch 1, wobei die relativen Expressionslevel durch Berechnen eines Verhältnisses aus der einen oder mehreren ersten miRNAs mit einer Nukleotidsequenz, ausgewählt aus der Gruppe bestehend aus SEQ ID NO: 1 bis SEQ ID NO: 16 und SEQ ID NO: 20 bis SEQ ID NO: 26, und der einen oder mehreren zweiten miRNAs mit einer Nukleotidsequenz, ausgewählt aus der Gruppe bestehend aus SEQ ID NO: 27, SEQ ID NO: 29, SEQ ID NO: 35, SEQ ID NO: 38, SEQ ID NO: 39, SEQ ID NO. 43 bis SEQ ID NO: 47, SEQ ID NO: 49, SEQ ID NO: 50, SEQ ID NO: 52, SEQ ID NO: 55 bis SEQ ID NO: 57 und SEQ ID NO: 59, erhalten werden, wobei die relativen Expressionslevel vorzugsweise aus der Gruppe der in Figur 8 aufgeführten Verhältnisse ausgewählt sind.

4. Verfahren nach einem der Ansprüche 1 bis 3, wobei in Schritt (f) eine Änderung des Gesundheitszustands beurteilt wird, wenn der Vergleich der relativen Expressionslevel des Subjekts mit den relativen Referenzexpressionsleveln einen Schwellenwert überschreitet.

5. Verfahren nach Anspruch 4, wobei der Schwellenwert auf 3 Standardabweichungen, vorzugsweise auf 5 Standardabweichungen und noch bevorzugter auf 7 Standardabweichungen festgelegt wird.

6. Verfahren zur Beurteilung des Gesundheitszustands eines Subjekts, umfassend die Schritte:
(a) Isolieren der gesamten intrazellulären RNA aus Erythrozyten, Leukozyten und Thrombozyten, die aus einer Vollblutprobe erhalten wurden, die einem Subjekt entnommen wurde,
(b) Bestimmen eines Expressionsprofils von mindestens einer ersten miRNA in der gesamten intrazellulären RNA,
(c) Bestimmen eines Expressionsprofils von mindestens einer zweiten miRNA in der gesamten intrazellulären RNA,
(d) Vergleichen des Expressionsprofils der mindestens einen ersten miRNA aus Schritt (b) mit einem Referenzexpressionsprofil der mindestens einen ersten miRNA und des Expressionsprofils der mindestens einen zweiten miRNA aus Schritt (c) mit einem Referenzexpressionsprofil der mindestens einen zweiten miRNA, und
(e) Beurteilen einer Änderung des Gesundheitszustands des Subjekt, wenn der Vergleich des Expressionsprofils der mindestens einen ersten miRNA mit dem Referenzexpressionsprofil der mindestens einen ersten miRNA verändert ist und wenn der Vergleich des Expressionsprofils der mindestens einen zweiten miRNA mit dem Referenzexpressionsprofil der mindestens einen zweiten miRNA nicht verändert ist, wobei
die mindestens eine erste miRNA eine Nukleotidsequenz gemäß SEQ ID NO: 1 aufweist und die mindestens eine zweite miRNA eine Nukleotidsequenz gemäß SEQ ID NO: 27 aufweist,
die mindestens eine erste miRNA eine Nukleotidsequenz gemäß SEQ ID NO: 1 aufweist und die mindestens eine zweite miRNA eine Nukleotidsequenz gemäß SEQ ID NO: 29 aufweist,
die mindestens eine erste miRNA eine Nukleotidsequenz gemäß SEQ ID NO: 1 aufweist und die mindestens eine zweite miRNA eine Nukleotidsequenz gemäß SEQ ID NO: 35 aufweist,
die mindestens eine erste miRNA eine Nukleotidsequenz gemäß SEQ ID NO: 2 aufweist und die mindestens eine zweite miRNA eine Nukleotidsequenz gemäß SEQ ID NO: 27 aufweist,
die mindestens eine erste miRNA eine Nukleotidsequenz gemäß SEQ ID NO: 2 aufweist und die mindestens eine zweite miRNA eine Nukleotidsequenz gemäß SEQ ID NO: 29 aufweist,
die mindestens eine erste miRNA eine Nukleotidsequenz gemäß SEQ ID NO: 3 aufweist und die mindestens eine zweite miRNA eine Nukleotidsequenz gemäß SEQ ID NO: 27 aufweist,
die mindestens eine erste miRNA eine Nukleotidsequenz gemäß SEQ ID NO: 3 aufweist und die mindestens eine zweite miRNA eine Nukleotidsequenz gemäß SEQ ID NO: 29 aufweist,
die mindestens eine erste miRNA eine Nukleotidsequenz gemäß SEQ ID NO: 4 aufweist und die mindestens eine zweite miRNA eine Nukleotidsequenz gemäß SEQ ID NO: 27 aufweist,
die mindestens eine erste miRNA eine Nukleotidsequenz gemäß SEQ ID NO: 4 aufweist und die mindestens eine zweite miRNA eine Nukleotidsequenz gemäß SEQ ID NO: 29 aufweist,
die mindestens eine erste miRNA eine Nukleotidsequenz gemäß SEQ ID NO: 5 aufweist und die mindestens eine zweite miRNA eine Nukleotidsequenz gemäß SEQ ID NO: 27 aufweist,
die mindestens eine erste miRNA eine Nukleotidsequenz gemäß SEQ ID NO: 5 aufweist und die mindestens eine zweite miRNA eine Nukleotidsequenz gemäß SEQ ID NO: 29 aufweist,
die mindestens eine erste miRNA eine Nukleotidsequenz gemäß SEQ ID NO: 5 aufweist und die mindestens eine zweite miRNA eine Nukleotidsequenz gemäß SEQ ID NO: 55 aufweist,
die mindestens eine erste miRNA eine Nukleotidsequenz gemäß SEQ ID NO: 5 aufweist und die mindestens eine zweite miRNA eine Nukleotidsequenz gemäß SEQ ID NO: 59 aufweist,
die mindestens eine erste miRNA eine Nukleotidsequenz gemäß SEQ ID NO: 6 aufweist und die mindestens eine zweite miRNA eine Nukleotidsequenz gemäß SEQ ID NO: 27 aufweist,
die mindestens eine erste miRNA eine Nukleotidsequenz gemäß SEQ ID NO: 6 aufweist und die mindestens eine zweite miRNA eine Nukleotidsequenz gemäß SEQ ID NO: 29 aufweist,
die mindestens eine erste miRNA eine Nukleotidsequenz gemäß SEQ ID NO: 6 aufweist und die mindestens eine zweite miRNA eine Nukleotidsequenz gemäß SEQ ID NO: 35 aufweist,
die mindestens eine erste miRNA eine Nukleotidsequenz gemäß SEQ ID NO: 6 aufweist und die mindestens eine zweite miRNA eine Nukleotidsequenz gemäß SEQ ID NO: 38 aufweist,
die mindestens eine erste miRNA eine Nukleotidsequenz gemäß SEQ ID NO: 7 aufweist und die mindestens eine zweite miRNA eine Nukleotidsequenz gemäß SEQ ID NO: 27 aufweist,
die mindestens eine erste miRNA eine Nukleotidsequenz gemäß SEQ ID NO: 7 aufweist und die mindestens eine zweite miRNA eine Nukleotidsequenz gemäß SEQ ID NO: 29 aufweist,
die mindestens eine erste miRNA eine Nukleotidsequenz gemäß SEQ ID NO: 7 aufweist und die mindestens eine zweite miRNA eine Nukleotidsequenz gemäß SEQ ID NO: 35 aufweist,
die mindestens eine erste miRNA eine Nukleotidsequenz gemäß SEQ ID NO: 8 aufweist und die mindestens eine zweite miRNA eine Nukleotidsequenz gemäß SEQ ID NO: 27 aufweist,
die mindestens eine erste miRNA eine Nukleotidsequenz gemäß SEQ ID NO: 8 aufweist und die mindestens eine zweite miRNA eine Nukleotidsequenz gemäß SEQ ID NO: 29 aufweist,
die mindestens eine erste miRNA eine Nukleotidsequenz gemäß SEQ ID NO: 8 aufweist und die mindestens eine zweite miRNA eine Nukleotidsequenz gemäß SEQ ID NO: 35 aufweist,
die mindestens eine erste miRNA eine Nukleotidsequenz gemäß SEQ ID NO: 9 aufweist und die mindestens eine zweite miRNA eine Nukleotidsequenz gemäß SEQ ID NO: 27 aufweist,
die mindestens eine erste miRNA eine Nukleotidsequenz gemäß SEQ ID NO: 10 aufweist und die mindestens eine zweite miRNA eine Nukleotidsequenz gemäß SEQ ID NO: 35 aufweist,
die mindestens eine erste miRNA eine Nukleotidsequenz gemäß SEQ ID NO: 13 aufweist und die mindestens eine zweite miRNA eine Nukleotidsequenz gemäß SEQ ID NO: 35 aufweist,
die mindestens eine erste miRNA eine Nukleotidsequenz gemäß SEQ ID NO: 15 aufweist und die mindestens eine zweite miRNA eine Nukleotidsequenz gemäß SEQ ID NO: 35 aufweist,
die mindestens eine erste miRNA eine Nukleotidsequenz gemäß SEQ ID NO: 24 aufweist und die mindestens eine zweite miRNA eine Nukleotidsequenz gemäß SEQ ID NO: 35 aufweist, oder
die mindestens eine erste miRNA hat eine Nukleotidsequenz gemäß SEQ ID NO: 24 und die mindestens eine zweite miRNA hat eine Nukleotidsequenz gemäß SEQ ID NO: 38.

7. Verfahren nach einem der Ansprüche 2 bis 5, wobei das eine oder die mehreren relativen Referenzexpressionslevel zu einem früheren Zeitpunkt von demselben Subjekt erhalten werden.

8. Verfahren nach einem der Ansprüche 1 bis 7, wobei die Änderung des Gesundheitszustands eine Verbesserung oder eine Verschlechterung des Gesundheitszustands ist.

9. Verwendung des Verfahrens nach einem der Ansprüche 1 bis 8 zum:
(i.) Vergleichen des Gesundheitszustands zwischen einem oder mehreren Subjekten,
(ii.) Überwachen des Gesundheitszustands eines Subjekts,
(iii.) Überwachen des Zustands des Immunsystems eines Subjekts, und/oder
(iv.) Überwachung des Ansprechens auf eine therapeutische Behandlung, vorzugsweise eine medikamentöse Behandlung, bei einem Subjekt.

10. Verfahren zur Überwachung des Gesundheitszustands eines Subjekts, umfassend die Schritte:
(a) Beurteilen des Gesundheitszustands eines Subjekts zu einem ersten Zeitpunkt durch Ausführen des Verfahrens nach einem der Ansprüche 1 bis 8,
(b) Beurteilen des Gesundheitszustands eines Subjekts zu einem oder mehreren späteren Zeitpunkten durch Ausführen des Verfahrens nach einem der Ansprüche 1 bis 8, und
(c) Vergleichen des in Schritt (a) ermittelten Gesundheitszustands mit dem in Schritt (b) ermittelten Gesundheitszustand, wobei der Gesundheitszustand des Subjekts überwacht wird,
wobei der überwachte Gesundheitszustand des Subjekts vorzugsweise den Zustand des Immunsystems des Subjekts umfasst.

11. Verfahren nach Anspruch 10, wobei sich der Gesundheitszustand des Subjekts im Laufe der Zeit verbessert oder verschlechtert.

12. Verfahren nach Ansprüchen 10 oder 11, wobei das Subjekt zwischen dem ersten Zeitpunkt und dem einen oder mehreren späteren Zeitpunkten eine therapeutische Behandlung erhalten hat.

13. Verfahren nach Anspruch 12, wobei die therapeutische Behandlung die Verabreichung eines Medikaments, eine Operation, einer Chemotherapie, einer Strahlentherapie und/oder einer Immuntherapie umfasst.

## Revendications

1. Méthode permettant d'évaluer l'état de santé chez un sujet, comprenant les étapes de :
(a) isolement de l'ARN intracellulaire total à partir d'érythrocytes, de leucocytes et de thrombocytes qui ont été obtenus à partir d'un échantillon de sang entier qui a été prélevé sur un sujet,
(b) détermination dans ledit ARN intracellulaire total d'un profil d'expression d'un ou plusieurs premiers miARN, lesdits un ou plusieurs premiers miARN présentant une séquence nucléotidique choisie dans le groupe constitué par SEQ ID NO : 1 à SEQ ID NO : 16 et SEQ ID NO : 20 à SEQ ID NO : 26,
(c) détermination dans ledit ARN intracellulaire total d'un profil d'expression d'un ou plusieurs seconds miARN, lesdits un ou plusieurs seconds miARN présentant une séquence nucléotidique choisie dans le groupe constitué par SEQ ID NO : 27, SEQ ID NO : 29, SEQ ID NO : 35, SEQ ID NO : 38, SEQ ID NO : 39, SEQ ID NO : 43 à SEQ ID NO : 47, SEQ ID NO : 49, SEQ ID NO : 50, SEQ ID NO : 52, SEQ ID NO : 55 à SEQ ID NO : 57, et SEQ ID NO : 59,
(d) normalisation du profil d'expression desdits un ou plusieurs premiers miARN par rapport au profil d'expression desdits un ou plusieurs seconds miARN, ce qui permet d'obtenir un ou plusieurs niveaux d'expression relatifs de : SEQ ID NO : 1/SEQ ID NO : 45, SEQ ID NO : 1/SEQ ID NO : 59, SEQ ID NO : 1/SEQ ID NO : 56, SEQ ID NO : 2/SEQ ID NO: 27, SEQ ID NO : 2/SEQ ID NO : 35, SEQ ID NO : 3/SEQ ID NO : 27, SEQ ID NO : 4/SEQ ID NO : 55, SEQ ID NO : 4/SEQ ID NO : 47, SEQ ID NO : 4/SEQ ID NO : 29^{∗}, SEQ ID NO : 4/SEQ ID NO : 56, SEQ ID NO : 4/SEQ ID NO : 59, SEQ ID NO : 4/SEQ ID NO : 43, SEQ ID NO : 5/SEQ ID NO : 59, SEQ ID NO : 5/SEQ ID NO : 29, SEQ ID NO : 5/SEQ ID NO : 55, SEQ ID NO : 5/SEQ ID NO : 45, SEQ ID NO : 5/SEQ ID NO : 44, SEQ ID NO : 5/SEQ ID NO : 57, SEQ ID NO : 5/SEQ ID NO : 46, SEQ ID NO : 5/SEQ ID NO : 50, SEQ ID NO : 5/SEQ ID NO : 49, SEQ ID NO : 5/SEQ ID NO : 56, SEQ ID NO : 5/SEQ ID NO : 47, SEQ ID NO : 6/SEQ ID NO : 35, SEQ ID NO : 6/SEQ ID NO : 27, SEQ ID NO : 6/SEQ ID NO : 38, SEQ ID NO : 6/SEQ ID NO : 29, SEQ ID NO : 6/SEQ ID NO : 43, SEQ ID NO : 6/SEQ ID NO : 49, SEQ ID NO : 6/SEQ ID NO : 50, SEQ ID NO : 6/SEQ ID NO : 52, SEQ ID NO : 6/SEQ ID NO : 39, SEQ ID NO : 7/SEQ ID NO : 27, SEQ ID NO : 7/SEQ ID NO : 35, SEQ ID NO : 7/SEQ ID NO : 29, SEQ ID NO : 7/SEQ ID NO : 43, SEQ ID NO : 7/SEQ ID NO : 50, SEQ ID NO : 7/SEQ ID NO : 39, SEQ ID NO : 7/SEQ ID NO : 38, SEQ ID NO : 7/SEQ ID NO : 47, SEQ ID NO : 8/SEQ ID NO : 35, SEQ ID NO : 8/SEQ ID NO : 27, SEQ ID NO : 8/SEQ ID NO : 38, SEQ ID NO : 8/SEQ ID NO : 39, SEQ ID NO : 8/SEQ ID NO : 43, SEQ ID NO : 9/SEQ ID NO : 35, SEQ ID NO : 9/SEQ ID NO : 27, SEQ ID NO : 9/SEQ ID NO : 38, SEQ ID NO : 9/SEQ ID NO : 43, SEQ ID NO : 9/SEQ ID NO : 29, SEQ ID NO : 9/SEQ ID NO : 39, SEQ ID NO : 10/SEQ ID NO : 35, SEQ ID NO : 10/SEQ ID NO : 27, SEQ ID NO : 10/SEQ ID NO : 38, SEQ ID NO : 10/SEQ ID NO : 49, SEQ ID NO : 10/SEQ ID NO : 39, SEQ ID NO : 10/SEQ ID NO : 43, SEQ ID NO : 11/SEQ ID NO : 27, SEQ ID NO : 11/SEQ ID NO : 35, SEQ ID NO : 11/SEQ ID NO : 39, SEQ ID NO : 11/SEQ ID NO : 38, SEQ ID NO : 12/SEQ ID NO : 35, SEQ ID NO : 13/SEQ ID NO : 35, SEQ ID NO : 13/SEQ ID NO : 27, SEQ ID NO : 13/SEQ ID NO : 39, SEQ ID NO : 14/SEQ ID NO : 35, SEQ ID NO : 15/SEQ ID NO : 35, SEQ ID NO : 15/SEQ ID NO : 38, SEQ ID NO : 15/SEQ ID NO : 27, SEQ ID NO : 15/SEQ ID NO : 39, SEQ ID NO : 16/SEQ ID NO : 35, SEQ ID NO : 20/SEQ ID NO : 29, SEQ ID NO : 20/SEQ ID NO: 59, SEQ ID NO : 20/SEQ ID NO : 57, SEQ ID NO : 20/SEQ ID NO : 38, SEQ ID NO : 20/SEQ ID NO : 39, SEQ ID NO : 20/SEQ ID NO : 47, SEQ ID NO : 21/SEQ ID NO : 50, SEQ ID NO : 21/SEQ ID NO : 59, SEQ ID NO : 21/SEQ ID NO : 29^{∗}, SEQ ID NO : 21/SEQ ID NO : 57, SEQ ID NO : 21/SEQ ID NO : 55, SEQ ID NO : 21/SEQ ID NO : 49, SEQ ID NO : 21/SEQ ID NO : 56, SEQ ID NO : 22/SEQ ID NO : 59, SEQ ID NO : 22/SEQ ID NO : 47, SEQ ID NO : 22/SEQ ID NO : 56, SEQ ID NO : 22/SEQ ID NO : 57, SEQ ID NO : 22/SEQ ID NO : 29^{∗}, SEQ ID NO : 23/SEQ ID NO : 59, SEQ ID NO : 23/SEQ ID NO : 56, SEQ ID NO : 23/SEQ ID NO : 57, SEQ ID NO : 24/SEQ ID NO : 27, SEQ ID NO : 24/SEQ ID NO : 35, SEQ ID NO : 24/SEQ ID NO : 38, SEQ ID NO : 24/SEQ ID NO : 39, SEQ ID NO : 24/SEQ ID NO : 43, SEQ ID NO : 25/SEQ ID NO : 35, SEQ ID NO : 25/SEQ ID NO : 39, SEQ ID NO : 25/SEQ ID NO : 43, SEQ ID NO : 25/SEQ ID NO : 38, SEQ ID NO : 25/SEQ ID NO : 27, SEQ ID NO : 26/SEQ ID NO : 35, ou SEQ ID NO : 26/SEQ ID NO : 27,
(e) comparaison desdits un ou plusieurs niveaux d'expression relatifs de l'étape (d) à un ou plusieurs niveaux d'expression de référence relatifs, et
(f) évaluation d'un changement d'état de santé chez ledit sujet lorsque la comparaison auxdits un ou plusieurs niveaux d'expression de référence relatifs est altérée.

2. Méthode selon la revendication 1, lesdits un ou plusieurs niveaux d'expression de référence relatifs étant obtenus en comprenant les étapes de :
(i) isolement de l'ARN intracellulaire total à partir d'érythrocytes, de leucocytes et de thrombocytes qui ont été obtenus à partir d'un échantillon de sang entier qui a été prélevé sur un ou plusieurs sujets de référence,
(ii) détermination dans ledit ARN intracellulaire total d'un profil d'expression de référence d'un ou plusieurs premiers miARN, lesdits un ou plusieurs premiers miARN présentant une séquence nucléotidique choisie dans le groupe constitué par SEQ ID NO : 1 à SEQ ID NO : 16 et SEQ ID NO : 20 à SEQ ID NO : 26,
(iii) détermination dans ledit ARN intracellulaire total d'un profil d'expression de référence d'un ou plusieurs seconds miARN, lesdits un ou plusieurs seconds miARN présentant une séquence nucléotidique choisie dans le groupe constitué par SEQ ID NO : 27, SEQ ID NO : 29, SEQ ID NO : 35, SEQ ID NO : 38, SEQ ID NO : 39, SEQ ID NO : 43 à SEQ ID NO : 47, SEQ ID NO : 49, SEQ ID NO : 50, SEQ ID NO : 52, SEQ ID NO : 55 à SEQ ID NO : 57, et SEQ ID NO : 59, et
(iv) normalisation du profil d'expression de référence desdits un ou plusieurs premiers miARN par rapport au profil d'expression de référence desdits un ou plusieurs seconds miARN desdits un ou plusieurs sujets de référence, ce qui permet d'obtenir un ou plusieurs niveaux d'expression de référence relatifs de : SEQ ID NO : 1/SEQ ID NO : 45, SEQ ID NO : 1/SEQ ID NO : 59, SEQ ID NO : 1/SEQ ID NO : 56, SEQ ID NO : 2/SEQ ID NO: 27, SEQ ID NO : 2/SEQ ID NO : 35, SEQ ID NO : 3/SEQ ID NO : 27, SEQ ID NO : 4/SEQ ID NO : 55, SEQ ID NO : 4/SEQ ID NO : 47, SEQ ID NO : 4/SEQ ID NO : 29^{∗}, SEQ ID NO : 4/SEQ ID NO : 56, SEQ ID NO : 4/SEQ ID NO : 59, SEQ ID NO : 4/SEQ ID NO : 43, SEQ ID NO : 5/SEQ ID NO : 59, SEQ ID NO : 5/SEQ ID NO : 29, SEQ ID NO : 5/SEQ ID NO : 55, SEQ ID NO : 5/SEQ ID NO : 45, SEQ ID NO : 5/SEQ ID NO : 44, SEQ ID NO : 5/SEQ ID NO : 57, SEQ ID NO : 5/SEQ ID NO : 46, SEQ ID NO : 5/SEQ ID NO : 50, SEQ ID NO : 5/SEQ ID NO : 49, SEQ ID NO : 5/SEQ ID NO : 56, SEQ ID NO : 5/SEQ ID NO : 47, SEQ ID NO : 6/SEQ ID NO : 35, SEQ ID NO : 6/SEQ ID NO : 27, SEQ ID NO : 6/SEQ ID NO : 38, SEQ ID NO : 6/SEQ ID NO : 29, SEQ ID NO : 6/SEQ ID NO : 43, SEQ ID NO : 6/SEQ ID NO : 49, SEQ ID NO : 6/SEQ ID NO : 50, SEQ ID NO : 6/SEQ ID NO : 52, SEQ ID NO : 6/SEQ ID NO : 39, SEQ ID NO : 7/SEQ ID NO : 27, SEQ ID NO : 7/SEQ ID NO : 35, SEQ ID NO : 7/SEQ ID NO : 29, SEQ ID NO : 7/SEQ ID NO : 43, SEQ ID NO : 7/SEQ ID NO : 50, SEQ ID NO : 7/SEQ ID NO : 39, SEQ ID NO : 7/SEQ ID NO : 38, SEQ ID NO : 7/SEQ ID NO : 47, SEQ ID NO : 8/SEQ ID NO : 35, SEQ ID NO : 8/SEQ ID NO : 27, SEQ ID NO : 8/SEQ ID NO : 38, SEQ ID NO : 8/SEQ ID NO : 39, SEQ ID NO : 8/SEQ ID NO : 43, SEQ ID NO : 9/SEQ ID NO : 35, SEQ ID NO : 9/SEQ ID NO : 27, SEQ ID NO : 9/SEQ ID NO : 38, SEQ ID NO : 9/SEQ ID NO : 43, SEQ ID NO : 9/SEQ ID NO : 29, SEQ ID NO : 9/SEQ ID NO : 39, SEQ ID NO : 10/SEQ ID NO : 35, SEQ ID NO : 10/SEQ ID NO : 27, SEQ ID NO : 10/SEQ ID NO : 38, SEQ ID NO : 10/SEQ ID NO : 49, SEQ ID NO : 10/SEQ ID NO : 39, SEQ ID NO : 10/SEQ ID NO : 43, SEQ ID NO : 11/SEQ ID NO : 27, SEQ ID NO : 11/SEQ ID NO : 35, SEQ ID NO : 11/SEQ ID NO : 39, SEQ ID NO : 11/SEQ ID NO : 38, SEQ ID NO : 12/SEQ ID NO : 35, SEQ ID NO : 13/SEQ ID NO : 35, SEQ ID NO : 13/SEQ ID NO : 27, SEQ ID NO : 13/SEQ ID NO : 39, SEQ ID NO : 14/SEQ ID NO : 35, SEQ ID NO : 15/SEQ ID NO : 35, SEQ ID NO : 15/SEQ ID NO : 38, SEQ ID NO : 15/SEQ ID NO : 27, SEQ ID NO : 15/SEQ ID NO : 39, SEQ ID NO : 16/SEQ ID NO : 35, SEQ ID NO : 20/SEQ ID NO : 29, SEQ ID NO : 20/SEQ ID NO: 59, SEQ ID NO : 20/SEQ ID NO : 57, SEQ ID NO : 20/SEQ ID NO : 38, SEQ ID NO : 20/SEQ ID NO : 39, SEQ ID NO : 20/SEQ ID NO : 47, SEQ ID NO : 21/SEQ ID NO : 50, SEQ ID NO : 21/SEQ ID NO : 59, SEQ ID NO : 21/SEQ ID NO : 29^{∗}, SEQ ID NO : 21/SEQ ID NO : 57, SEQ ID NO : 21/SEQ ID NO : 55, SEQ ID NO : 21/SEQ ID NO : 49, SEQ ID NO : 21/SEQ ID NO : 56, SEQ ID NO : 22/SEQ ID NO : 59, SEQ ID NO : 22/SEQ ID NO : 47, SEQ ID NO : 22/SEQ ID NO : 56, SEQ ID NO : 22/SEQ ID NO : 57, SEQ ID NO : 22/SEQ ID NO : 29^{∗}, SEQ ID NO : 23/SEQ ID NO : 59, SEQ ID NO : 23/SEQ ID NO : 56, SEQ ID NO : 23/SEQ ID NO : 57, SEQ ID NO : 24/SEQ ID NO : 27, SEQ ID NO : 24/SEQ ID NO : 35, SEQ ID NO : 24/SEQ ID NO : 38, SEQ ID NO : 24/SEQ ID NO : 39, SEQ ID NO : 24/SEQ ID NO : 43, SEQ ID NO : 25/SEQ ID NO : 35, SEQ ID NO : 25/SEQ ID NO : 39, SEQ ID NO : 25/SEQ ID NO : 43, SEQ ID NO : 25/SEQ ID NO : 38, SEQ ID NO : 25/SEQ ID NO : 27, SEQ ID NO : 26/SEQ ID NO : 35, ou SEQ ID NO : 26/SEQ ID NO : 27.

3. Méthode selon la revendication 1, lesdits niveaux d'expression relatifs étant obtenus en calculant un rapport à partir desdits un ou plusieurs premiers miARN présentant une séquence nucléotidique choisie dans le groupe constitué par SEQ ID NO : 1 à SEQ ID NO : 16 et SEQ ID NO : 20 à SEQ ID NO : 26 et desdits un ou plusieurs seconds miARN présentant une séquence nucléotidique choisie dans le groupe constitué par SEQ ID NO : 27, SEQ ID NO : 29, SEQ ID NO : 35, SEQ ID NO : 38, SEQ ID NO : 39, SEQ ID NO : 43 à SEQ ID NO : 47, SEQ ID NO : 49, SEQ ID NO : 50, SEQ ID NO : 52, SEQ ID NO : 55 à SEQ ID NO : 57, et SEQ ID NO : 59, lesdits niveaux d'expression relatifs étant de préférence choisis dans le groupe de rapports énumérés à la figure 8.

4. Méthode selon l'une quelconque des revendications 1 à 3, dans l'étape (f) un changement de l'état de santé étant évalué lorsque la comparaison des niveaux d'expression relatifs dudit sujet aux niveaux d'expression de référence relatifs dépasse un seuil.

5. Méthode selon la revendication 4, ledit seuil étant fixé à 3 écarts types, de préférence étant fixé à 5 écarts types, plus préférablement étant fixé à 7 écarts types.

6. Méthode d'évaluation de l'état de santé chez un sujet, comprenant les étapes de :
(a) isolement de l'ARN intracellulaire total à partir d'érythrocytes, de leucocytes et de thrombocytes qui ont été obtenus à partir d'un échantillon de sang entier qui a été prélevé sur un sujet,
(b) détermination dans ledit ARN intracellulaire total d'un profil d'expression d'au moins un premier miARN,
(c) détermination dans ledit ARN intracellulaire total d'un profil d'expression d'au moins un second miARN,
(d) comparaison du profil d'expression dudit au moins un premier miARN de l'étape (b) à un profil d'expression de référence dudit au moins premier miARN et du profil d'expression dudit au moins un second miARN de l'étape (c) à un profil d'expression de référence dudit au moins un second miARN, et
(e) évaluation d'un changement de l'état de santé chez ledit sujet lorsque la comparaison du profil d'expression dudit au moins un premier miARN au profil d'expression de référence dudit au moins un premier miARN est altérée et lorsque la comparaison du profil d'expression dudit au moins un second miARN au profil d'expression de référence dudit au moins un second miARN n'est pas altérée,
ledit au moins un premier miARN présentant une séquence nucléotidique selon SEQ ID NO : 1 et ledit au moins un second miARN présentant une séquence nucléotidique selon SEQ ID NO : 27,
ledit au moins un premier miARN présentant une séquence nucléotidique selon SEQ ID NO : 1 et ledit au moins un second miARN présentant une séquence nucléotidique selon SEQ ID NO : 29,
ledit au moins un premier miARN présentant une séquence nucléotidique selon SEQ ID NO : 1 et ledit au moins un second miARN présentant une séquence nucléotidique selon SEQ ID NO : 35,
ledit au moins un premier miARN présentant une séquence nucléotidique selon SEQ ID NO : 2 et ledit au moins un second miARN présentant une séquence nucléotidique selon SEQ ID NO : 27,
ledit au moins un premier miARN présentant une séquence nucléotidique selon SEQ ID NO : 2 et ledit au moins un second miARN présentant une séquence nucléotidique selon SEQ ID NO : 29,
ledit au moins un premier miARN présentant une séquence nucléotidique selon SEQ ID NO : 3 et ledit au moins un second miARN présentant une séquence nucléotidique selon SEQ ID NO : 27,
ledit au moins un premier miARN présentant une séquence nucléotidique selon SEQ ID NO : 3 et ledit au moins un second miARN présentant une séquence nucléotidique selon SEQ ID NO : 29,
ledit au moins un premier miARN présentant une séquence nucléotidique selon SEQ ID NO : 4 et ledit au moins un second miARN présentant une séquence nucléotidique selon SEQ ID NO : 27,
ledit au moins un premier miARN présentant une séquence nucléotidique selon SEQ ID NO : 4 et ledit au moins un second miARN présentant une séquence nucléotidique selon SEQ ID NO : 29,
ledit au moins un premier miARN présentant une séquence nucléotidique selon SEQ ID NO : 5 et ledit au moins un second miARN présentant une séquence nucléotidique selon SEQ ID NO : 27,
ledit au moins un premier miARN présentant une séquence nucléotidique selon SEQ ID NO : 5 et ledit au moins un second miARN présentant une séquence nucléotidique selon SEQ ID NO : 29,
ledit au moins un premier miARN présentant une séquence nucléotidique selon SEQ ID NO : 5 et ledit au moins un second miARN présentant une séquence nucléotidique selon SEQ ID NO : 55,
ledit au moins un premier miARN présentant une séquence nucléotidique selon SEQ ID NO : 5 et ledit au moins un second miARN présentant une séquence nucléotidique selon SEQ ID NO : 59,
ledit au moins un premier miARN présentant une séquence nucléotidique selon SEQ ID NO : 6 et ledit au moins un second miARN présentant une séquence nucléotidique selon SEQ ID NO : 27,
ledit au moins un premier miARN présentant une séquence nucléotidique selon SEQ ID NO : 6 et ledit au moins un second miARN présentant une séquence nucléotidique selon SEQ ID NO : 29,
ledit au moins un premier miARN présentant une séquence nucléotidique selon SEQ ID NO : 6 et ledit au moins un second miARN présentant une séquence nucléotidique selon SEQ ID NO : 35,
ledit au moins un premier miARN présentant une séquence nucléotidique selon SEQ ID NO : 6 et ledit au moins un second miARN présentant une séquence nucléotidique selon SEQ ID NO : 38,
ledit au moins un premier miARN présentant une séquence nucléotidique selon SEQ ID NO : 7 et ledit au moins un second miARN présentant une séquence nucléotidique selon SEQ ID NO : 27,
ledit au moins un premier miARN présentant une séquence nucléotidique selon SEQ ID NO : 7 et ledit au moins un second miARN présentant une séquence nucléotidique selon SEQ ID NO : 29,
ledit au moins un premier miARN présentant une séquence nucléotidique selon SEQ ID NO : 7 et ledit au moins un second miARN présentant une séquence nucléotidique selon SEQ ID NO : 35,
ledit au moins un premier miARN présentant une séquence nucléotidique selon SEQ ID NO : 8 et ledit au moins un second miARN présentant une séquence nucléotidique selon SEQ ID NO : 27,
ledit au moins un premier miARN présentant une séquence nucléotidique selon SEQ ID NO : 8 et ledit au moins un second miARN présentant une séquence nucléotidique selon SEQ ID NO : 29,
ledit au moins un premier miARN présentant une séquence nucléotidique selon SEQ ID NO : 8 et ledit au moins un second miARN présentant une séquence nucléotidique selon SEQ ID NO : 35,
ledit au moins un premier miARN présentant une séquence nucléotidique selon SEQ ID NO : 9 et ledit au moins un second miARN présentant une séquence nucléotidique selon SEQ ID NO : 27,
ledit au moins un premier miARN présentant une séquence nucléotidique selon SEQ ID NO : 10 et ledit au moins un second miARN présentant une séquence nucléotidique selon SEQ ID NO : 35,
ledit au moins un premier miARN présentant une séquence nucléotidique selon SEQ ID NO : 13 et ledit au moins un second miARN présentant une séquence nucléotidique selon SEQ ID NO : 35,
ledit au moins un premier miARN présentant une séquence nucléotidique selon SEQ ID NO : 15 et ledit au moins un second miARN présentant une séquence nucléotidique selon SEQ ID NO : 35,
ledit au moins un premier miARN présentant une séquence nucléotidique selon SEQ ID NO : 24 et ledit au moins un second miARN présentant une séquence nucléotidique selon SEQ ID NO : 35, ou
ledit au moins un premier miARN présentant une séquence nucléotidique selon SEQ ID NO : 24 et ledit au moins un second miARN présentant une séquence nucléotidique selon SEQ ID NO : 38.

7. Méthode selon l'une quelconque des revendications 2 à 5, lesdits un ou plusieurs niveaux d'expression de référence relatifs étant obtenus à partir d'un moment antérieur auprès du même sujet.

8. Méthode selon l'une quelconque des revendications 1 à 7, ledit changement d'état de santé étant une amélioration ou une détérioration de l'état de santé.

9. Utilisation de la méthode selon l'une quelconque des revendications 1 à 8 pour :
(i.) comparaison de l'état de santé entre un ou plusieurs sujets,
(ii.) surveillance de l'état de santé chez un sujet,
(iii.) surveillance de l'état du système immunitaire chez un sujet, et/ou
(iv.) surveillance de la réponse à un traitement thérapeutique, de préférence un traitement médicamenteux, chez un sujet.

10. Méthode permettant la surveillance de l'état de santé chez un sujet, comprenant les étapes de :
(a) évaluation de l'état de santé chez un sujet à un premier moment dans le temps en exécutant la méthode selon l'une quelconque des revendications 1 à 8,
(b) évaluation de l'état de santé chez le sujet à un ou plusieurs moments postérieurs dans le temps en exécutant la méthode selon l'une quelconque des revendications 1 à 8, et
(c) comparaison de l'état de santé évalué dans l'étape (a) à l'état de santé évalué dans l'étape (b), ce qui permet de surveiller l'état de santé chez le sujet,
ledit état de santé chez le sujet qui est surveillé de préférence incluant l'état du système immunitaire dudit sujet.

11. Méthode selon la revendication 10, ledit état de santé chez le sujet s'améliorant ou se détériorant dans le temps.

12. Méthode selon la revendication 10 ou 11, ledit sujet ayant reçu un traitement thérapeutique entre le premier moment dans le temps et lesdits un ou plusieurs moments postérieurs dans le temps.

13. Méthode selon la revendication 12, ledit traitement thérapeutique comprenant l'administration d'un médicament, une intervention chirurgicale, une chimiothérapie, une radiothérapie et/ou une immunothérapie.


### SEQUENCE LISTING

<110> Comprehensive Biomarker Center GmbH
<120> SIGNATURE OF HEALTH
<130> 505-47 PCT
<160> 77
<170> PatentIn version 3.5
<210> 1
   <211> 23
   <212> RNA
   <213> Homo sapiens
<400> 1
   ugaggggcag agagcgagac uuu 23
<210> 2
   <211> 21
   <212> RNA
   <213> Homo sapiens
<400> 2
   uucaaguaau ucaggauagg u 21
<210> 3
   <211> 22
   <212> RNA
   <213> Homo sapiens
<400> 3
   uuauaauaca accugauaag ug 22
<210> 4
   <211> 23
   <212> RNA
   <213> Homo sapiens
<400> 4
   guccaguuuu cccaggaauc ccu 23
<210> 5
   <211> 17
   <212> RNA
   <213> Homo sapiens
<400> 5
   ucucgcuggg gccucca 17
<210> 6
   <211> 23
   <212> RNA
   <213> Homo sapiens
<400> 6
   caaagugcuc auagugcagg uag 23
<210> 7
   <211> 22
   <212> RNA
   <213> Homo sapiens
<400> 7
   ggauaucauc auauacugua ag 22
<210> 8
   <211> 23
   <212> RNA
   <213> Homo sapiens
<400> 8
   caaagugcuu acagugcagg uag 23
<210> 9
   <211> 22
   <212> RNA
   <213> Homo sapiens
<400> 9
   ucguaccgug aguaauaaug cg 22
<210> 10
   <211> 23
   <212> RNA
   <213> Homo sapiens
<400> 10
   uaaagugcuu auagugcagg uag 23
<210> 11
   <211> 22
   <212> RNA
   <213> Homo sapiens
<400> 11
   auauaauaca accugcuaag ug 22
<210> 12
   <211> 23
   <212> RNA
   <213> Homo sapiens
<400> 12
   uaaggugcau cuagugcaga uag 23
<210> 13
   <211> 23
   <212> RNA
   <213> Homo sapiens
<400> 13
   aaaagugcuu acagugcagg uag 23
<210> 14
   <211> 21
   <212> RNA
   <213> Homo sapiens
<400> 14
   uaaagugcug acagugcaga u 21
<210> 15
   <211> 21
   <212> RNA
   <213> Homo sapiens
<400> 15
   agcuacaucu ggcuacuggg u 21
<210> 16
   <211> 22
   <212> RNA
   <213> Homo sapiens
<400> 16
   ugccuacuga gcugaaacac ag 22
<210> 17
   <211> 22
   <212> RNA
   <213> Homo sapiens
<400> 17
   uucaaguaau ccaggauagg cu 22
<210> 18
   <211> 23
   <212> RNA
   <213> Homo sapiens
<400> 18
   caaagugcug uucgugcagg uag 23
<210> 19
   <211> 23
   <212> RNA
   <213> Homo sapiens
<400> 19
   ugugcaaauc caugcaaaac uga 23
<210> 20
   <211> 22
   <212> RNA
   <213> Homo sapiens
<400> 20
   ugucaguuug ucaaauaccc ca 22
<210> 21
   <211> 22
   <212> RNA
   <213> Homo sapiens
<400> 21
   acugcugagc uagcacuucc cg 22
<210> 22
   <211> 21
   <212> RNA
   <213> Homo sapiens
<400> 22
   gugggcgggg gcaggugugu g 21
<210> 23
   <211> 22
   <212> RNA
   <213> Homo sapiens
<400> 23
   ucaggcucag uccccucccg au 22
<210> 24
   <211> 22
   <212> RNA
   <213> Homo sapiens
<400> 24
   aauugcacgg uauccaucug ua 22
<210> 25
   <211> 22
   <212> RNA
   <213> Homo sapiens
<400> 25
   ucagugcacu acagaacuuu gu 22
<210> 26
   <211> 22
   <212> RNA
   <213> Homo sapiens
<400> 26
   gagcuuauuc auaaaagugc ag 22
<210> 27
   <211> 22
   <212> RNA
   <213> Homo sapiens
<400> 27
   uauugcacuu gucccggccu gu 22
<210> 28
   <211> 22
   <212> RNA
   <213> Homo sapiens
<400> 28
   uagcagcacg uaaauauugg cg 22
<210> 29
   <211> 23
   <212> RNA
   <213> Homo sapiens
<400> 29
   ugugcaaauc uaugcaaaac uga 23
<210> 30
   <211> 22
   <212> RNA
   <213> Homo sapiens
<400> 30
   caggccauau ugugcugccu ca 22
<210> 31
   <211> 22
   <212> RNA
   <213> Homo sapiens
<400> 31
   cugguacagg ccugggggac ag 22
<210> 32
   <211> 21
   <212> RNA
   <213> Homo sapiens
<400> 32
   caacaccagu cgaugggcug u 21
<210> 33
   <211> 22
   <212> RNA
   <213> Homo sapiens
<400> 33
   aguuuugcau aguugcacua ca 22
<210> 34
   <211> 21
   <212> RNA
   <213> Homo sapiens
<400> 34
   uucacagugg cuaaguucug c 21
<210> 35
   <211> 21
   <212> RNA
   <213> Homo sapiens
<400> 35
   uaccacaggg uagaaccacg g 21
<210> 36
   <211> 23
   <212> RNA
   <213> Homo sapiens
<400> 36
   aaugacacga ucacucccgu uga 23
<210> 37
   <211> 22
   <212> RNA
   <213> Homo sapiens
<400> 37
   uguaaacauc cuacacucag cu 22
<210> 38
   <211> 22
   <212> RNA
   <213> Homo sapiens
<400> 38
   aagcugccag uugaagaacu gu 22
<210> 39
   <211> 22
   <212> RNA
   <213> Homo sapiens
<400> 39
   cauugcacuu gucucggucu ga 22
<210> 40
   <211> 22
   <212> RNA
   <213> Homo sapiens
<400> 40
   uguaacagca acuccaugug ga 22
<210> 41
   <211> 21
   <212> RNA
   <213> Homo sapiens
<400> 41
   cugaccuaug aauugacagc c 21
<210> 42
   <211> 23
   <212> RNA
   <213> Homo sapiens
<400> 42
   ucucacacag aaaucgcacc cgu 23
<210> 43
   <211> 22
   <212> RNA
   <213> Homo sapiens
<400> 43
   uagcagcaca uaaugguuug ug 22
<210> 44
   <211> 19
   <212> RNA
   <213> Homo sapiens
<400> 44
   aaaagcuggg uugagagga 19
<210> 45
   <211> 20
   <212> RNA
   <213> Homo sapiens
<400> 45
   aaaagcuggg uugagagggu 20
<210> 46
   <211> 20
   <212> RNA
   <213> Homo sapiens
<400> 46
   acugccccag gugcugcugg 20
<210> 47
   <211> 22
   <212> RNA
   <213> Homo sapiens
<400> 47
   ugagguagua gauuguauag uu 22
<210> 48
   <211> 22
   <212> RNA
   <213> Homo sapiens
<400> 48
   ucggccugac cacccacccc ac 22
<210> 49
   <211> 22
   <212> RNA
   <213> Homo sapiens
<400> 49
   aaggagcuca cagucuauug ag 22
<210> 50
   <211> 23
   <212> RNA
   <213> Homo sapiens
<400> 50
   cgcauccccu agggcauugg ugu 23
<210> 51
   <211> 22
   <212> RNA
   <213> Homo sapiens
<400> 51
   ucugggcaac aaagugagac cu 22
<210> 52
   <211> 22
   <212> RNA
   <213> Homo sapiens
<400> 52
   cagcagcaau ucauguuuug aa 22
<210> 53
   <211> 21
   <212> RNA
   <213> Homo sapiens
<400> 53
   ugagaugaag cacuguagcu c 21
<210> 54
   <211> 22
   <212> RNA
   <213> Homo sapiens
<400> 54
   cacccguaga accgaccuug cg 22
<210> 55
   <211> 23
   <212> RNA
   <213> Homo sapiens
<400> 55
   ucccccaggu gugauucuga uuu 23
<210> 56
   <211> 21
   <212> RNA
   <213> Homo sapiens
<400> 56
   gcccuccgcc cgugcacccc g 21
<210> 57
   <211> 22
   <212> RNA
   <213> Homo sapiens
<400> 57
   ugugcgcagg gagaccucuc cc 22
<210> 58
   <211> 20
   <212> RNA
   <213> Homo sapiens
<400> 58
   cugcaaaggg aagcccuuuc 20
<210> 59
   <211> 22
   <212> RNA
   <213> Homo sapiens
<400> 59
   cugcccuggc ccgagggacc ga 22
<210> 60
   <211> 23
   <212> RNA
   <213> Homo sapiens
<400> 60
   agcagcauug uacagggcua uca 23
<210> 61
   <211> 21
   <212> RNA
   <213> Homo sapiens
<400> 61
   uggcagggag gcugggaggg g 21
<210> 62
   <211> 18
   <212> RNA
   <213> Homo sapiens
<400> 62
   cgggcguggu gguggggg 18
<210> 63
   <211> 23
   <212> RNA
   <213> Homo sapiens
<400> 63
   agcagcauug uacagggcua uga 23
<210> 64
   <211> 20
   <212> RNA
   <213> Homo sapiens
<400> 64
   uacaguauag augauguacu 20
<210> 65
   <211> 22
   <212> RNA
   <213> Homo sapiens
<400> 65
   uucaccaccu ucuccaccca gc 22
<210> 66
   <211> 18
   <212> RNA
   <213> Homo sapiens
<400> 66
   aucccaccuc ugccacca 18
<210> 67
   <211> 23
   <212> RNA
   <213> Homo sapiens
<400> 67
   agcucggucu gaggccccuc agu 23
<210> 68
   <211> 23
   <212> RNA
   <213> Homo sapiens
<400> 68
   uagugcaaua uugcuuauag ggu 23
<210> 69
   <211> 22
   <212> RNA
   <213> Homo sapiens
<400> 69
   ucagcaaaca uuuauugugu gc 22
<210> 70
   <211> 22
   <212> RNA
   <213> Homo sapiens
<400> 70
   caaaccacac ugugguguua ga 22
<210> 71
   <211> 21
   <212> RNA
   <213> Homo sapiens
<400> 71
   gugcauugua guugcauugc a 21
<210> 72
   <211> 22
   <212> RNA
   <213> Homo sapiens
<400> 72
   cuauacgacc ugcugccuuu cu 22
<210> 73
   <211> 22
   <212> RNA
   <213> Homo sapiens
<400> 73
   aaaagcuggg uugagagggc ga 22
<210> 74
   <211> 22
   <212> RNA
   <213> Homo sapiens
<400> 74
   aaugcaccug ggcaaggauu ca 22
<210> 75
   <211> 22
   <212> RNA
   <213> Homo sapiens
<400> 75
   caacaaauca cagucugcca ua 22
<210> 76
   <211> 23
   <212> RNA
   <213> Homo sapiens
<400> 76
   cucucaccac ugcccuccca cag 23
<210> 77
   <211> 21
   <212> RNA
   <213> Homo sapiens
<400> 77
   cccggagcca ggaugcagcu c 21